(19)

Europäisches
Patentamt
European
Patent Office
Office européen
des brevets

(11) **EP 3 715 375 A1**

(12) **EUROPEAN PATENT APPLICATION**

(43) Date of publication:
**30.09.2020  Bulletin 2020/40**

(51) Int Cl.:
*C07K 17/06* (2006.01)      *A61K 39/00* (2006.01)
*A61K 47/54* (2017.01)      *C07K 14/05* (2006.01)

(21) Application number: **19164785.8**

(22) Date of filing: **23.03.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Ablevia biotech GmbH
1030 Vienna (AT)**

(72) Inventor: **The designation of the inventor has not
yet been filed**

(74) Representative: **Sonn & Partner Patentanwälte
Riemergasse 14
1010 Wien (AT)**

(54) **COMPOUND FOR THE PREVENTION OR TREATMENT OF PRE-ECLAMPSIA**

(57)    The present invention provides a compound for the sequestration of anti-Epstein-Barr virus nuclear antigen 1 (EBNA-1) antibodies and/or anti human melatonin-related receptor (GPR50) antibodies present in a human individual, the compound comprising a biopolymer scaffold and at least two peptides with a sequence length of 7-13 amino acids, wherein each of the peptides independently comprises a 7-13 amino-acid sequence fragment of the EBNA1 sequence identified by UniProt accession code Q1HVF7 or P03211 or of the GPR50 sequence identified by UniProt accession code Q13585, wherein the peptides are covalently bound to the biopolymer scaffold, wherein the biopolymer scaffold is selected from the group consisting of human globulins, preferably from the group consisting of human immunoglobulins and human haptoglobin, and human albumin. This compound is useful in the prevention or treatment of pre-eclampsia.

EP 3 715 375 A1

**Description**

The field of present invention relates to compounds for the sequestration of undesirable antibodies in an individual, such as antibodies related to autoimmune diseases.

In general, antibodies are essential components of the humoral immune system, offering protection from infections by foreign organisms including bacteria, viruses, fungi or parasites. However, under certain circumstances - including autoimmune diseases, organ transplantation, blood transfusion or upon administration of biomolecular drugs or gene delivery vectors - antibodies can target the patient's own body (or the foreign tissue or cells or the biomolecular drug or vector just administered), thereby turning into harmful or disease-causing entities. Certain antibodies can also interfere with probes for diagnostic imaging. In the following, such antibodies are generally referred to as "undesired antibodies" or "undesirable antibodies".

With few exceptions, selective removal of undesired antibodies has not reached clinical practice. It is presently restricted to very few indications: One of the known techniques for selective antibody removal (although not widely established) is selective immunoapheresis. In contrast to Immunoapheresis (which removes Immunoglobulin), selective immunoapheresis involves the filtration of plasma through an extracorporeal, selective antibody-adsorber cartridge that will deplete the undesired antibody based on selective binding to its antigen binding site. Selective immunoapheresis has for instance been used for removing anti-A or anti-B antibodies from the blood prior to AB0-incompatible transplantation or with respect to indications in transfusion medicine (Teschner et al). Selective apheresis was also experimentally applied in other indications, such as neuroimmunological indications (Tetala et al) or myasthenia gravis (Lazaridis et al), but is not yet established in the clinical routine. One reason that selective immunoapheresis is only hesitantly applied is the fact that it is a cost intensive and cumbersome intervention procedure that requires specialized medical care. Moreover it is not known in the prior art how to deplete undesired antibodies rapidly and efficiently.

As mentioned, apheresis is applied extracorporeally. By contrast, also several approaches to deplete undesirable antibodies intracorporeally were proposed in the prior art, mostly in connection with certain autoimmune diseases involving autoantibodies or anti-drug antibodies:

Lorentz et al discloses a technique whereby erythrocytes are charged in situ with a tolerogenic payload driving the deletion of antigen-specific T cells. This is supposed to ultimately lead to reduction of the undesired humoral response against a model antigen. A similar approach is proposed in Pishesha et al. In this approach, erythrocytes are loaded ex vivo with a peptide-antigen construct that is covalently bound to the surface and reinjected into the animal model for general immunotolerance induction.

WO 92/13558 A1 relates to conjugates of stable nonimmunogenic polymers and analogs of immunogens that possess the specific B cell binding ability of the immunogen and which, when introduced into individuals, induce humoral anergy to the immunogen. Accordingly, these conjugates are disclosed to be useful for treating antibody-mediated pathologies that are caused by foreign- or self-immunogens.

Taddeo et al discloses selectively depleting antibody producing plasma cells using anti-CD138 antibody derivatives fused to an ovalbumin model antigen thereby inducing receptor crosslinking and cell suicide in vitro selectively in those cells that express the antibody against the model antigen.

Apitope International NV (Belgium) is presently developing soluble tolerogenic T-cell epitope peptides which may lead to expression of low levels of co-stimulatory molecules from antigen presenting cells inducing tolerance, thereby suppressing antibody response (see e.g. Jansson et al). These products are currently under preclinical and early clinical evaluation, e.g. in multiple sclerosis, Grave's disease, intermediate uveitis, and other autoimmune conditions as well as Factor VIII intolerance.

Similarly, Selecta Biosciences, Inc. (USA) is currently pursuing strategies of tolerance induction by so-called Synthetic Vaccine Particles (SVPs). SVP-Rapamycin is supposed to induce tolerance by preventing undesired antibody production via selectively inducing regulatory T cells (see Mazor et al).

Mingozzi et al discloses decoy adeno-associated virus (AAV) capsids that adsorb antibodies but cannot enter a target cell.

WO 2015/136027 A1 discloses carbohydrate ligands presenting the minimal Human Natural Killer-1 (HNK-1) epitope that bind to anti-MAG (myelin-associated glycoprotein) IgM antibodies, and their use in diagnosis as well as for the treatment of anti-MAG neuropathy. WO 2017/046172 A1 discloses further carbohydrate ligands and moieties, respectively, mimicking glycoepitopes comprised by glycosphingolipids of the nervous system which are bound by anti-glycan antibodies associated with neurological diseases. The document further relates to the use of these carbohydrate ligands/moieties in diagnosis as well as for the treatment of neurological diseases associated with anti-glycan antibodies.

US 2004/0258683 A1 discloses methods for treating systemic lupus erythematosus (SLE) including renal SLE and methods of reducing risk of renal flare in individuals with SLE, and methods of monitoring such treatment. One disclosed method of treating SLE including renal SLE and reducing risk of renal flare in an individual with SLE involves the administration of an effective amount of an agent for reducing the level of anti-double-stranded DNA (dsDNA) antibody, such as a dsDNA epitope as in the form of an epitope-presenting carrier or an epitope-presenting valency platform molecule, to the individual.

US patent no. 5,637,454 relates to assays and treatments of autoimmune diseases. Agents used for treatment might include peptides homologous to the identified antigenic, molecular mimicry sequences. It is disclosed that these peptides could be delivered to a patient in order to decrease the amount of circulating antibody with a particular specificity.

US 2007/0026396 A1 relates to peptides directed against antibodies, which cause cold-intolerance, and the use thereof. It is taught that by using the disclosed peptides, in vivo or ex vivo neutralization of undesired autoantibodies is possible. A comparable approach is disclosed in WO 1992/014150 A1 or in WO 1998/030586 A2.

WO 2018/102668 A1 discloses a fusion protein for selective degradation of disease-causing or otherwise undesired antibodies. The fusion protein (termed "Seldeg") includes a targeting component that specifically binds to a cell surface receptor or other cell surface molecule at near-neutral pH, and an antigen component fused directly or indirectly to the targeting component. Also disclosed is a method of depleting a target antigen-specific antibody from a patient by administering to the patient a Seldeg having an antigen component configured to specifically bind the target antigen-specific antibody.

Erlandsson et al discloses in vivo clearing of idiotypic antibodies with anti-idiotypic antibodies and their derivatives.

Berlin Cures Holding AG (Germany) has proposed an intravenous broad spectrum neutralizer DNA aptamer (see e.g. WO 2016/020377 A1 and WO 2012/000889 A1) for the treatment of dilated cardiomyopathy and other GPCR-autoantibody related diseases that in high dosage is supposed to block autoantibodies by competitive binding to the antigen binding regions of autoantibodies. In general, aptamers did not yet achieve a breakthrough and are still in a preliminary stage of clinical development. The major concerns are still biostability and bioavailability, constraints such as nuclease sensitivity, toxicity, small size and renal clearance. A particular problem with respect to their use as selective antibody antagonists are their propensity to stimulate the innate immune response.

WO 00/33887 A2 discloses methods for reducing circulating levels of antibodies, particularly disease-associated antibodies. The methods entail administering effective amounts of epitope-presenting carriers to an individual. In addition, ex vivo methods for reducing circulating levels of antibodies are disclosed which employ epitope-presenting carriers.

However, the approaches to deplete undesirable antibodies intracorporeally disclosed in the prior art have many shortcomings. In particular, neither of them has been approved for regular clinical use.

It is thus an object of the present invention to provide improved compounds and methods for intracorporeal depletion (or sequestration) of undesired antibodies (such as antibodies related to autoimmune disease) in an individual, in particular for use in treatment or prevention of a disease or condition related to the undesired antibody (e.g. of autoimmune disease).

The present invention provides a compound comprising

- a biopolymer scaffold and at least

- a first peptide n-mer of the general formula:

$$\text{P} \ ( \ - \ \text{S} \ - \ \text{P} \ )_{(n-1)}$$

and

- a second peptide n-mer of the general formula:

$$\text{P} \ ( \ - \ \text{S} \ - \ \text{P} \ )_{(n-1)} \ .$$

Independently for each occurrence, P is a peptide with a sequence length of 2-13 amino acids, preferably 3-11 amino acids, more preferably 4-9 amino acids, especially 5-8 amino acids, and S is a non-peptide spacer. Independently for each of the peptide n-mers, n is an integer of at least 1, preferably of at least 2, more preferably of at least 3, even more preferably of at least 4, especially of at least 5. Each of the peptide n-mers is bound to the biopolymer scaffold, preferably via a linker each.

Preferably, at least one occurrence of P is $P_a$ and/or at least one occurrence of P is $P_b$. $P_a$ is a defined peptide (i.e. a peptide of defined sequence) with a sequence length of 2-13 amino acids, preferably 3-11 amino acids, more preferably 4-9 amino acids, especially 5-8 amino acids. $P_b$ is a defined peptide (i.e. a peptide of defined sequence) with a sequence length of 2-13 amino acids, preferably 3-11 amino acids, more preferably 4-9 amino acids, especially 5-8 amino acids.

The present invention also provides a compound comprising

- a biopolymer scaffold and at least

- a first peptide n-mer which is a peptide dimer of the formula $P_a$ - **S** - $P_a$ or $P_a$ - **S** - $P_b$, wherein $P_a$ is a defined peptide (i.e. a peptide of defined sequence) with a sequence length of 2-13 amino acids, preferably 3-11 amino acids, more preferably 4-9 amino acids, especially 5-8 amino acids, $P_b$ is a defined peptide (i.e. a peptide of defined sequence) with a sequence length of 2-13 amino acids, preferably 3-11 amino acids, more preferably 4-9 amino acids, especially 5-8 amino acids, and S is a non-peptide spacer, wherein the first peptide

n-mer is bound to the biopolymer scaffold, preferably via a linker.

This compound preferably comprises a second peptide n-mer which is a peptide dimer of the formula $P_b$ - **S** - $P_b$ or $P_a$ - **S** - $P_b$, wherein the second peptide n-mer is bound to the biopolymer scaffold, preferably via a linker.

The present invention further provides a compound, preferably for the sequestration (or depletion) of anti human muscle nicotinic acetylcholine receptor (AChR) antibodies, anti human muscle-specific receptor tyrosine kinase antibodies and/or anti human low-density lipoprotein receptor related protein 4 antibodies present in a human individual, the compound comprising a biopolymer scaffold and at least two peptides with a sequence length of 7-13 amino acids, wherein each of the peptides independently comprises a 7-13 amino-acid sequence fragment of the AChR subunit alpha sequence identified by UniProt accession code P02708 or of the muscle-specific receptor tyrosine kinase sequence identified by UniProt accession code 015146 or of the low-density lipoprotein receptor related protein 4 sequence identified by UniProt accession code O75096, wherein the peptides are covalently bound to the biopolymer scaffold, preferably via a linker, wherein the biopolymer scaffold is selected from the group consisting of human globulins and human albumin.

The present invention also provides a compound, preferably for the sequestration (or depletion) of anti-Epstein-Barr virus nuclear antigen 1 (EBNA-1) antibodies, anti human melatonin-related receptor (GPR50) antibodies and/or anti human type-1 angiotensin II receptor (AT1AR) antibodies present in a human individual, the compound comprising a biopolymer scaffold and at least two peptides with a sequence length of 7-13 amino acids, wherein each of the peptides independently comprises a 7-13 amino-acid sequence fragment of the EBNA1 sequence identified by UniProt accession code Q1HVF7 or P03211 or of the GPR50 sequence identified by UniProt accession code Q13585 or of the type-1 angiotensin II receptor (AT1AR) sequence identified by UniProt accession code P30556, wherein the peptides are covalently bound to the biopolymer scaffold, preferably via a linker, wherein the biopolymer scaffold is selected from the group consisting of human globulins, preferably from the group consisting of human immunoglobulins and human haptoglobin, and human albumin.

Furthermore, the present invention provides a pharmaceutical composition comprising any one of the aforementioned compounds and at least one pharmaceutically acceptable excipient. Preferably, this pharmaceutical composition is for use in therapy, in particular of any one of the diseases or conditions mentioned herein.

In another aspect, the present invention provides a method of sequestering (or depleting) one or more antibodies present in an individual, comprising obtaining a pharmaceutical composition as defined herein, the composition being non-immunogenic in the individual, where the one or more antibodies present in the individual are specific for at least one occurrence of P, or for peptide $P_a$ and/or peptide $P_b$; and administering the pharmaceutical composition to the individual.

In yet another aspect, the present invention relates to a pharmaceutical composition, comprising the compound defined herein and further comprising an active agent and optionally at least one pharmaceutically acceptable excipient. The active agent comprises a peptide fragment with a sequence length of 2-13 amino acids, preferably 3-11 amino acids, more preferably 4-9 amino acids, especially 5-8 amino acids. The sequence of at least one occurrence of peptide P, or peptide $P_a$ and/or peptide $P_b$, of the compound is at least 70% identical, preferably at least 75% identical, more preferably at least 80% identical, yet more preferably at least 85% identical, even more preferably at least 90% identical, yet even more preferably at least 95% identical, especially completely identical to the sequence of said peptide fragment. Preferably, this pharmaceutical composition is for use in prevention or inhibition of an immune reaction against the active agent.

In even yet another aspect, the present invention provides a method of inhibiting an immune reaction to a treatment with an active agent in an individual in need of treatment with the active agent, comprising obtaining said pharmaceutical composition comprising the compound and the active agent; wherein the compound of the pharmaceutical composition is non-immunogenic in the individual, and administering the pharmaceutical composition to the individual.

In the course of the present invention, it was surprisingly found that the compound of the present invention is particularly effective in reducing titres of undesired antibodies in an individual. In particular, the compound achieved especially good results with regard to selectivity, duration of titre reduction and/or level of titre reduction in an in vivo model (see experimental examples).

The detailed description given below relates to all of the above aspects of the invention unless explicitly excluded.

**EP 3 715 375 A1**

Intracorporeal depletion of undesired antibodies (as achieved by the present invention) is for example advantageous in autoimmune diseases, organ- and cell transplantation, blood transfusion, or with respect to antibodies that are directed against biotherapeutics, substitution therapeutics or viral gene delivery vectors or even in diagnostic imaging or in emergency intervention following antibody treatment or active vaccination. In particular, with respect to autoimmune diseases, several hundreds of autoimmune conditions are connected to autoantibodies that bind to defined self-epitopes or neoepitopes present in the body. There appears to be practically no restriction with respect to organs or tissues that can be affected by disease-causing autoantibodies: Table 1 below lists some examples to illustrate the diversity of indication fields where it is beneficial to target undesired antibodies selectively.

Table 1 - Exemplary list of applications of the present invention:

| AUTOIMMUNE DISEASES | ANTI-DRUG and NEUTRALIZING ANTIBODIES |
|---|---|
| CNS & Muscle | |
| | ADAs/nABs against coagulation factors |
| MOG seropositive neuromyelitis optica spectrum disorders | ADAs/nABs against enzymes |
| | ADAs/nABs against growth factors and hormones |
| Neuromyelitis Optica | ADAs/nABs against fusion proteins and decoy receptor drugs |
| Autoimmune-Encephalitis | |
| Multiple Sclerosis | |
| Amyotrophic Lateral Sclerosis | |
| SLE dementia | **TRANSPLANTATION and TRANSFUSION MEDICINE** |
| Myasthenia gravis | Alloantibodies |
| | Blood group antibodies |
| Heart & Organs: | AB0-incompatible organ Transplantation |
| Dilatative Cardiomyopathy, pulmonary hypertension | **EMERGENCY INTERVENTION** |
| Chagas' Disease | Active vaccine intervention (therapeutic vaccines) |
| other diseases with $\beta$1-adrenergic receptors | |
| Primary biliary cholangitis | Passive vaccine intervention (therapeutic mABs) |
| Sjögren's Syndrome | Cytokine Release Syndrome (e.g. After CART treatment) |
| Caeliac Disease | |
| Graves Disease | |
| Goodpasture Disease | **VIRAL VECTORS IN GENE THERAPY** |
| Systemic conditions: | **IN VIVO DIAGNOSTIC IMAGING** |
| Systemic Lupus Erythematosus | |
| Sjögren's syndrome | |
| Systemic Sclerosis | |
| Behcet's Disease | |
| Hypertension | |
| type I Diabetes | |
| type II Diabetes | |
| Preeclampsia | |
| Blood: | |
| Immune Thrombocytopenic Purpura (ITP) | |
| Skin: | |
| pemphigus vulgaris | |
| bullous pemphigoid | |
| Epidermolysis bullosa acquista and bullous SLE | |

5

For instance, in transplantation medicine, undesired alloantibodies may occur. Alloantibodies are antibodies directed against foreign tissue antigens which can contribute to accelerated transplant rejection after transplantation (Garces et al, 2017). Upon tissue-, bone marrow- and stem cell transplantation, foreign tissue antigens are recognized by T-cells and B-cells producing antibodies against major and minor histocompatibility antigens. Inverse correlation between transplant survival and alloantibody levels confirms the pathogenic role of alloantibodies. Further, depletion of undesired blood group antibodies before and after AB0-incompatible transplantation of organs have been shown to be beneficial to transplant survival (Rummler et al, 2016).

Anti-drug Antibodies (ADAs), sometimes also referred to as neutralizing antibodies, are a category of undesired antibodies having emerged with the advent of biological drugs carrying epitopes that are recognized as "foreign" thereby inducing an anti-drug antibody response. This immune response can induce neutralizing antibodies (e.g. acting by depletion or blocking of the drug, or by forming immunocomplexes), a phenomenon that correlates with the amount of "foreign" sequences of the drug, inherent immunogenicity of the drug and, importantly, with the propensity to aggregate and to form complexes, once the drug is in the plasma (Moussa et al, 2016). Examples of drugs inducing ADAs include certain antibodies such as anti TNF-alpha antibodies, substitution therapeutics such as Hemophilia Factor VIII or enzymes used in enzyme replacement therapies such as Fabry Disease or such as uricase for the treatment fo refractory gout and other classes of biological therapeutics such as e.g. erythropoietin or interferon.

Similar to the situation with ADAs, pre-existing or induced undesired antibodies against gene therapy vectors are an emerging problem in the field of gene therapy (see e.g. Mingozzi & High, 2017): Gene therapy is rapidly progressing and Adeno Associated Viruses (AAV) but also other viral vector gene delivery vectors show promising preclinical and clinical results. This is particularly important for hematologic diseases such as hemophilia, or in Gaucher disease, porphyria or hemochromatosis or several other genetic diseases with enzyme defects. Much effort has been invested into optimization of AAV vectors, however the problem of pre-existing antibodies or newly induced antibodies against the vector, but also their T-cell immunogenicity is not yet solved. Several strategies to overcome B-cell and T-cell immunity were proposed including serological pre-selection of the target patients eligible for AAV treatment, high vector dosing, capsid decoys that pre-adsorb or deplete pre-existing or newly induced anti AAV antibodies, coadministration of decoy T-cell receptors or less selective approaches such as concomitant immunosuppression. The more pragmatic approaches were AAV serotype switching, plasma exchange therapies or local application of AAV vectors. Essentially, the immunological strategies boiled down to immunosuppression or tolerance induction. However, none of these strategies could solve the problem of pre-existing neutralizing anti AAV antibodies satisfactorily. The main disadvantage of the capsid decoy strategy was that empty capsids were processed by target cells similar to intact AAV particles, thereby facilitating antigen presentation by MHC I and stimulation of T-cells ultimately providing an immunostimulatory effect.

More than 2000 clinical trials for gene therapy (mostly clinical phase I or II trials) were undertaken during the last years. Monogenetic diseases still constitute a significant portion of the typical indications for gene-based therapies. They include a great diversity of indication fields, such as primary immunodeficiencies, inherited neurological disorders, cystic fibrosis, ocular disorders, hemoglobinopathies, hemophilias, alpha-1-antitrypsin deficiencies, lipoprotein lipase deficiency, enzyme defects, and many others. Other formats of gene therapy-based strategies such as Chimeric Antigen Receptor T-Cells (CAR T-Cells) evolve rapidly but still carry the risk for humoral responses against functional components of the system, such as switches, suicide gene products or other non-self and modified protein components, or against the viral gene delivery components or neo-antigens emerging by uncontrolled gene insertions into protein coding sequences. Mechanistically, gene therapy includes gene repair strategies, genome editing technologies and stable or transient gene expression strategies. As mentioned above, a common challenge remains that patients carry pre-existing neutralizing antibodies against viral gene therapy vectors that reduce the efficacy. Importantly, viral gene therapy vectors are often capable of inducing T-cell responses and neutralizing antibodies against viral proteins and their products. In addition, antibody- or T-cell responses can be introduced against the gene product itself or against the introduced DNA editing machinery such as components of the CRISPR/Cas9 containing natural or artificially modified endonucleases (such as prototype Cas9) that can be applied for therapeutic genome editing. Therefore, neutralizing antibodies that affect gene therapy efficacy remain a major challenge in the field of viral gene therapy vector development, in particular when using AAVs, lentiviruses or retroviruses.

Finally, in the context of safety intervention in active immunization trials (i.e. therapeutic vaccination) or passive treatments with antibodies or antibody-like compounds, interventional drugs for rapid selective antibody removal of e.g. therapeutic antibodies or antibody-like biotherapeutic compounds that cause complications under emergency conditions are needed. For these situations, there is especially a lack of rapid and effective selective antibody lowering strategies.

In response to the lack of satisfactory therapeutic strategies that can rapidly and safely remove undesired antibodies such as e.g. myasthenia gravis during a myasthenic crisis, before administration of a substitution therapeutic, before applying a gene targeting vector or in case of an adverse event induced by a therapeutic antibody or any antibody-like biological compound, the present invention provides a particularly suitable solution. The present invention represents a platform for flexible, optionally personalized, biotherapeutics that can be adapted to any type of undesired or harmful polyclonal or monoclonal antibody. In particular, these biotherapeutics can remove undesired antibodies rapidly, making them suitable for urgent interventions.

The biopolymer scaffold used in the present invention may be a mammalian biopolymer such as a human biopolymer, a non-human primate biopolymer, a sheep biopolymer, a pig biopolymer, a dog biopolymer or a rodent biopolymer. In particular the biopolymer scaffold is a protein, especially a (non-modified) plasma protein. Preferably, the biopolymer scaffold is a mammalian protein such as a human protein, a non-human primate protein, a sheep protein, a pig protein, a dog protein or a rodent protein. Typically, the biopolymer scaffold is a non-immunogenic and/or non-toxic protein that preferably circulates in the plasma of healthy (human) individuals and can e.g. be efficiently scavenged or recycled by scavenging receptors, such as e.g. present on liver sinusoidal endothelial cells (reviewed by Sorensen et al 2015).

According to a particular preference, the biopolymer scaffold is a (preferably human) globulin, preferably selected from the group consisting of immunoglobulins, alpha1-globulins, alpha2-globulins and beta-globulins, in particular immunoglobulin G, haptoglobin and transferrin.

The biopolymer scaffold may also be (preferably human) albumin, hemopexin, alpha-1-antitrypsin, C1 esterase inhibitor, lactoferrin or non-immunogenic (i.e. non-immunogenic in the individual to be treated) fragments of all of the aforementioned proteins, including the globulins.

The peptides (or peptide n-mers) are preferably covalently conjugated (or covalently bound) to the biopolymer scaffold via a (non-immunogenic) linker known in the art such as for example amine-to-sulfhydryl linkers and bifunctional NHS-PEG-maleimide linkers or other linkers known in the art. Alternatively, the peptides (or peptide n-mers) can be bound to the epitope carrier scaffold e.g. by formation of a disulfide bond between the protein and the peptide (which is also referred to as "linker" herein), or using non-covalent assembly techniques, spontaneous isopeptide bond formation or unnatural amino acids for bio-orthogonal chemistry via genetic code expansion techniques (reviewed by Howarth et al 2018 and Lim et al 2016).

The compound of the present invention may comprise e.g. at least two, preferably between 3 and 40 copies of one or several different peptides (which may be present in different forms of peptide n-mers as disclosed herein). The compound may comprise one type of epitopic peptide, however the diversity of epitopic peptides bound to one biopolymer scaffold molecule can be a mixture of e.g. up to 8 different epitopic peptides.

Typically, since the peptides present in the inventive compound specifically bind to selected undesired antibodies, their sequence is usually selected and optimized such that they provide specific binding in order to guarantee selectivity of undesired antibody depletion from the blood. For this purpose, the peptide sequence of the peptides typically corresponds to the entire epitope sequence or portions of the undesired antibody epitope. The peptides used in the present invention can be further optimized by exchanging one, two or up to four amino-acid positions, allowing e.g. for modulating the binding affinity to the undesired antibody that needs to be depleted. Such single amino-acid substitution strategies that can provide "mimotopes" with increased binding affinity and are known in the field and were previously developed using phage display strategies or peptide microarrays (see e.g. Application Note, "T PEPperMAP® Full Substitution Scan of HA and M13 Epitopes", by PEPperPRINT GmbH, Heidelberg, Germany). In other words, the peptides used in the present invention do not have to be completely identical to the native epitope sequences of the undesired antibodies.

Typically, the peptides used in the compound of the present invention (e.g. peptide P or $P_a$ or $P_b$ or $P_1$ or $P_2$) are composed of one or more of the 20 amino acids commonly present in mammalian proteins. In addition, the amino acid repertoire used in the peptides may be expanded to post-translationally modified amino acids e.g. affecting antigenicity of proteins such as post translational modifications, in particular oxidative post translational modifications (see e.g. Ryan 2014) or modifications to the peptide backbone (see e.g. Müller 2018), or to non-natural amino acids (see e.g. Meister et al 2018). These modifications may also be used in the peptides e.g. to adapt the binding interaction and specificity between the peptide and the variable region of an undesired antibody. In particular, epitopes (and therefore the peptides used in the compound of the present invention) can also contain citrulline as for example in autoimmune diseases. Furthermore, by introducing modifications into the peptide sequence the propensity of binding to an HLA molecule may be reduced, the stability and the physicochemical characteristics may be improved or the affinity to the undesired antibody may be increased.

In many cases, the undesired antibody that is to be depleted is oligo- or polyclonal (e.g. autoantibodies, ADAs or alloantibodies are typically poly- or oligoclonal), implying that undesired (polyclonal) antibody epitope covers a larger epitopic region of a target molecule. To adapt to this situation, the compound of the present invention may comprise a mixture of two or several epitopic peptides, thereby allowing to adapt to the polyclonality or oligoclonality of an undesired antibody.

Such poly-epitopic compounds of the present invention can effectively deplete undesired antibodies and are more often effective than mono-epitopic compounds in case the epitope of the undesired antibody extends to larger amino acid sequence stretches.

It is advantageous if the peptides used for the inventive compound are designed such that they will be specifically recognized by the variable region of the undesired antibodies to be depleted. The sequences of peptides used in the present invention may e.g. be selected by applying fine epitope mapping techniques (i.e. epitope walks, peptide deletion mapping, amino acid substitution scanning using peptide arrays such as described in Carter et al 2004, and Hansen et al 2013) on the undesired antibodies.

Preferably, the peptide used for the inventive compound (e.g. peptide P or $P_a$ or $P_b$) comprises an epitope or epitope part (e.g. at least two, preferably at least three, more preferably at least four, even more preferably at least five, yet even more preferably at least six, especially at least seven or even at least eight amino acids) of one of the following antigens (involved in autoimmune diseases) identified by their UniProt accession code:

P01023, A8K2U0, P49588, Q5JTZ9, O95477, Q8IZY2, P08183, P33527, O15438, Q96IU4, P00519, P42684, Q9BYF1, P22303, Q99798, P68133, P60709, P63261, P12814, O43707, P61158, Q13705, P37023, O75077, Q9UKQ2, Q76LX8, Q6ZMM2, P35611, P07327, P00325, P35348, P25100, P08588, P07550, P25098, P35626, P30566, P43652, P02771, Q5U5Z8, Q15109, P35573, Q9UL18, Q9UKV8, O00468, P01019, P30556, Q09666, P02765, O43918, Q9Y6K8, Q02952, P14550, P15121, O95154, P02768, P00352, P49189, Q9UM73, P09923, P05187, P03971, P49418, P03950, Q9BY76, Q15327, P15144, P04083, P50995, P07355, Q3ZCQ2, P12429, P09525, P08758, P08133, O76027, Q13367, P27695, Q9BZZ5, P02647, P04114, P02749, P05067, P29972, P55087, Q8N726, P05089, Q9UNA1, P52566, Q99819, Q15052, P07306, P04424, P08243, Q9BXN1, P15336, P13637, P05026, P98194, P20648, P51164, P06576, P48047, P54252, Q8WXX7, P01185, P25311, Q9H6S1, P61769, Q13072, O75531, Q99728, P10415, P41182, P11274, O14503, Q93088, O00499, O15392, P35226, P12643, P18075, Q8N8U9, Q13873, P17213, Q9NP55, Q96DR5, Q8TDL5, P15056, Q7Z569, P38398, P51587, Q58F21, Q8IWQ3, Q8NE79, Q9Y224, Q13901, P02745, P01024, P00915, P00918, P07451, O00555, Q00975, Q9NY47, Q9Y698, Q8TC20, Q05682, P27482, P27797, P27824, P04632, P52907, P42574, Q14790, P31415, P41180, P20810, O15446, P04040, Q9NTU7, Q5M9N0, Q3V6T2, P10147, P13501, P20248, P14635, P24385, Q8ND76, P51681, P49368, P48643, P50990, Q9NZQ7, P28906, P16671, P04234, P15529, P08174, P13987, P01732, P21926, P30305, P12830, P55291, P22223, P55283, P06493, P42771, P51861, Q01850, Q9H211, P13688, P06731, Q9UNI1, P49450, P07199, Q03188, Q02224, P49454, Q9H3R5, Q92674, Q6IPU0, Q7L2Z9, A8MT69, Q5JTW2, P00751, P08603, Q03591, P36980, Q02985, Q9P2M7, O95992, Q14839, P10645, P36222, Q15782, Q9UKJ5, Q9Y259, P11229, P08172, P20309, P08173, P08912, P02708, Q9UGM1, P11230, Q8NCH0, Q99828, O75339, Q14011, Q07065, P12277, Q96MX0, P06732, A8K7I4, O95832, O75508, P30622, Q96KN2, Q12860, Q02246, Q8IWV2, O94779, Q9UQ52, P78357, Q9UHC6, Q7Z7A1, P38432, Q5TAT6, Q9UMD9, P02452, Q01955, P29400, Q14031, P12111, Q02388, Q9Y215, P49747, Q14019, P00450, P16870, Q8TCG5, P17927, Q9NS37, Q9UJA2, P02741, P02511, P53674, O95825, O75390, Q9Y600, P04141, P09919, P0DML2, Q14406, Q6UVK1, Q01459, Q9GZU7, P16410, P35222, P53634, P07339, P08311, Q14247, O60494, Q14999, Q86UP6, P61073, P05108, P05093, P04798, P05177, P08686, P11509, P20813, P33261, P11712, P10635, P05181, P08684, Q8N907, P09172, P43146, P07585, P20711, Q16832, Q9NR30, O00571, Q86XP3, Q9NY93, O75398, P35659, P17661, Q96SL1, O94907, P10515, P09622, P36957, P24855, Q8NFT8, O00429, Q8N608, P27487, P42658, Q14195, Q9BPU6, P21728, P14416, Q08554, Q02487, Q14574, Q02413, Q14126, P32926, Q86SJ6, P15924, Q03001, Q9NRD8, Q05923, O75923, O95905, Q9NTX5, Q16610, O43854, P25101, Q15075, P68104, O00418, O95967, P01133, P00533, P20042, P38919, Q04637, P08246, Q12926, Q14576, P26378, P15502, P19622, P06733, P09104, P22413, O43768, P11171, P16422, P07099, P34913, P01588, P11678, P58107, P04626, Q96RT1, Q8IUD2, Q14264, P10768, P03372, Q9Y603, Q92817, Q9Y3B2, Q01780, Q13868, Q9NQT5, Q9NPD3, Q9NQT4, Q5RKV6, Q15024, Q96B26, Q06265, P15311, P00488, P08709, P00451, P00740, P15090, Q14320, P48023, P49327, Q8TES7, P22087, P35555, Q75N90, P09467, P12319, O75015, O75636, Q7L513, P02675, P11362, P62942, Q9UIM3, P20930, Q14315, O75955, Q14254, O43155, P35916, P02751, Q04609, P01225, Q12841, O95954, P02794, P02792, P09958, P35637, P51114, Q9UM11, P35575, O95166, P60520, Q9UBS5, O75899, Q99259, Q05329, Q13065, P22466, Q14376, P04406, P41250, P01350, P15976, P50440, P02774, P01275, Q8N6F7, P23434, P55107, P50395, P56159, Q9UJY5, P01241, P01286, Q9UBU3, P09681, O14908, P29033, Q9NS71, Q6ZMI3, P23415, P15104, Q6IB77, P49915, Q13823, P01148, P30968, Q92805, Q08379, Q08378, Q13439, A6NI86, A8MQT2, Q14789, P07359, P55259, P40197, Q9HCN6, P14770, Q9NQX3, P06744, Q13098, P24298, P18283, P42261, P42262, P42263, P48058, O43424, P39086, Q13002, Q16478, Q05586, Q12879, Q13224, Q4V328, Q13255, P41594, P28799, P07492, P08263, P21266, P78417, P09211, Q00403, P35269, P25092, P08236, P02724, P07305, P16104, O75367, P84243, P12081, Q96D42, P68871, Q13547, Q92769, O15379, P56524, Q9UQL6, P19113, Q9UBI9, P51858, Q00341, Q9NRV9, O00291, O75146, P54198, P16402, P58876, P62805, P19367, P09429, P26583, P04035, Q01581, P54868, P05114, P05204, Q14541, P09651, P22626, Q99729, Q14103, P52597, P31943, P31942, P61978, P14866, Q8WVV9, Q9NSC5, Q99714, Q7Z5P4, P14060, P08238, P14625, P0DMV8, P0DMV9, P34932, P11021, P11142, P04792, Q12988, P10809, Q92598, P08908, Q13639, Q9Y4L1, P10997, Q05084, Q9UMF0, O75874, Q5TF58, Q16666, Q9BYX4, P01563, P01574, P01579, Q9NWB7, P05019, P08069, P01344, Q9NZI8, Q9Y6M1, O00425, P11717, P18065, P17936, P01876, P01877, P01854, P01857, P01859, P01860, P01861, A6NGN9, Q8N6C5, P22301, Q13651, Q08334, Q14005, Q16552, Q96PD4, Q14116, P01583, P01584, P14778, P60568, Q9GZX6, P08700, P05112, P05231, P40189, Q96LU5, Q9NV31, P29218, O14732, P12268, Q9NQS7, P01308, Q96T92, P06213, P46940, Q14653, Q13568, P35568, P17301, P08514, P23229, P20701, P11215, P05107, P05106, P16144, Q14643, Q9Y6Y0, O60674, P17275, Q15046, P16389, P22459, Q9UK17, Q9NZI2, Q9NS61, P78508, P48050, P51787, O43525, Q8N5I3, Q6PI47, P35968, Q9Y4F3, Q96Q89, P43626, P43628, Q5JT82, Q53G59, Q8IXQ5, Q9UKR3, P03952, P26715, P26717, Q13241, P13645, P02533, P19012, P08779, Q04695, P05783, P08727, P12035, Q8N1N4, P05787, Q9NSB2, O15230, P11047, P13473, Q14739, P31025, P13796, P07195, P01130, Q9Y2U8, P09382, P05162, P17931, Q08380, Q3ZCW2, O95970, Q5TDP6, P22888, P49917, P07098, P02545, P20700, Q03252, P61968, P29536, P08519, Q07954, P98164, O75096, Q8TF66, Q32MZ4, Q8ND56, Q9Y4Z0, P02788, Q17RY6, P20645, Q8NHW3, P20916, P43358, O15479, O60732, Q9H0U3, P46821, P11137, Q16584, O43318, P45984, Q16644, P21941, O00339, P56270, P02144, Q9UIS9, P11226, P02686, Q01726, P32245, Q8IVS2, Q99705, Q969V1, Q8TDD5, Q8NE86, P40925, Q00987, O00255, P50579, P46013, Q16655, P03956, P45452, P08253, P09237, P14780, Q13201, Q13875, Q16653, Q13724, Q14149, Q9UBU8, O00566, Q99547, P40238, P05164, Q00013, Q9NZW5, P25189, P22897, Q9Y605, P82909, P43246, P52701, Q13421, P26038, Q9UJ68, P26927, Q13043, Q04912, Q9NZJ7, Q86UE4, P15941, Q8WXI7, O15146, Q9UIF7, P10242, P01106, Q99417, P12524, Q8N699, P12882, P35580, P35749, Q9UKX3, Q7Z406, Q9Y2K3, Q9UKX2, P11055, Q9Y623, P13533, P12883, A7E2Y1, P13535, P35579, B0I1T2, P54296, Q14CX7, E9PAV3, Q13765, Q8WY41, Q96I59, Q9UBB6, Q9UHB4, Q00604, P28331, P20929, P07196, P07197, Q8NG66, Q8TD19, O60524, O94856, P01138, Q8N4C6, P30414, P59047, Q8N427, Q13253, Q15155, P29475, P51513, Q9UNW9, P55786, O60500, P06748, P01160, P17342, P01303, Q9Y5X4, Q8IXM6, Q9ULB1, Q9HDB5, Q9Y4C0, Q9NXX6, P04629, Q16620, Q16288, Q02818, P80303, Q14980, P49790, Q8TEM1, O15504, Q14990, Q5BJF6, Q9ULJ1, Q6UX06, P78380, P41143, P35372, Q9P0S3, Q92791, Q9UQ80, Q13310, Q9UM07, Q7Z2X7, Q5JRK9, Q96GU1, Q13177, Q99497, P09874, P40424, Q15154, P12004, P29120, Q8WUM4, O95263, O76083, P16234, P09619, O00330, P30101, Q8N165, O00151, Q5T2W1, P16284, P02776, P10720, P35080, P18669, P00558, O95394, P35232, Q99623, Q9BVI0, Q92576, O43175, P11309, O75364, Q9Y446, P04054, Q13018, P16885, Q15149, Q9H7P9, P40967, P29590, Q01453, Q9NR77, P54277, P16233, P54317, Q8ND90, Q9UL42, P00491, Q9H9Y6, O14802, Q99575, P16435, Q15063, Q01851, Q12837, Q15181, P62937, O60437, P35813, P01298, Q9HAZ2, P32119, Q13162, P30041, P13727, Q92954, P17612, P17252, P01236, P04553, P04554, O60678, P04070, Q9UNN8, P54821, Q99811, P07477, P24158, Q9BXM0, O43653, O75475, P20618, P40306, P49721, P28074, P28062, P28065, P61289, Q6PGN9, P26599, Q8WV60, P01270, P06454, Q06124, Q9Y2R2, P08575, Q12913, Q16849, Q92932, Q86Y79, Q9UHX1, P20472, Q9BRP8, P51153, Q9UI14, Q15276, P63244, Q92878, Q06609, P04049, Q15311, Q9UKM9, Q14498, P38159, P10745, Q06330, P53805, O95199, Q9P258, P35243, P46063, P05451, Q8IX06, P57771, P08100, P12271, O60930, O00584, Q9ULK6, Q99942, Q9UBF6, P13489, O75116, Q01973, P15927, Q9Y2J0, Q9UNE2, Q02878, P05388, P05386, P05387, Q9BUL9, P78346, P78345, P62277, P60866, O75676, O43159, Q15404, O00442, Q92541, Q9NQC3, Q9Y265, Q9Y230, P48443, P21817, Q92736, P31151, P04271, P0DJI8, P0DJI9, P10523, P49591, O43290, Q99590, Q8WTV0, Q14108, P13521, P05408, Q14524, Q9BWW7, P34741, Q86SQ7, Q9UDX4, Q13228, P16109, P04279, Q9HC62, P49908, Q9HD40, P01009, P05543, P30740, P29508, P48594, P35237, P05121, P07093, P05155, Q9BYW2, Q7Z333, Q8N474, Q9BWM7, Q99961, O15266, O60902, Q9NYZ4, Q9Y336, Q9H0K1, Q14190, Q13239, Q14493, Q9H0C2, P12235, P05141, Q9H2B4, O43511, P11168, Q8IWU4, O00400, P08195, Q8IWA5, P48751, Q9Y6R1, Q9BRV3, Q92911, P37840, O76070, P08621, P09012, P14678, P09234, P62314, P62316, P62318, P62304, P62306, P62308, P63162, O14512, P00441, P04179, Q9BQB4, O00570, P56693, P35716, O15370, O60248, Q9UN79, O95416, Q9H6I2, P35713, P48431, Q9Y651, P41225, O94993, Q06945, P35711, P35712, Q9BT81, P57073, P48436, P08047, P23497, Q13342, Q9H930, Q15506, Q8N0X2, P00995, P16150, O43791, P10451, Q8TCT8, Q8TCT7, Q8TCT6, Q13813, Q13501, P10124, P61011, O76094, Q05066, P05455, O43805, P61278, Q13586, Q9P246, P31948, P49842, P16949, Q7Z7C7, Q13033, O75558, P61266, Q13190, Q8IWZ8, Q9Y2Z0, Q8IWU6, P63165, P61956, P17600, P08247, P21579, P37837, Q15633, Q13148, P26639, Q9NYW0, P20226, O60806, P24557, P17987, O60522, O14746, P02787, P05549, Q92734, P10646, P02786, P01266, P01137, P21980, Q08188, P49221, P07204, P40225, P10827, P10828, Q9UPZ6, P31483, P29401, Q9Y490, O60602, Q8TDI7, P17152, P42167, P42166, P01375, O00300, P43489, P19237, P48788, P19429, P13805, P45379, P45378, P09430, Q8NDV7, P11387, Q969P6, P11388, Q13472, O95985, P04637, Q9H3D4, O15350, P60174, P09493, P07202, P12270, P56180, O43280, Q92519, Q96RU7, P19474, O15164, Q9UPN9, Q6AZZ1, P10155, P48995, Q13507, Q7Z4N2, Q7Z2W7, Q9HBA0, Q9BZW7, P01222, P16473, Q9H2G4, Q14166, Q8WZ42, P02766, P07437, O00294, Q15672, Q9P2K2, Q86VQ3, Q6A555, P14679, Q9BZF9, Q13404, Q14139, O95155, P11441, Q9UMX0, P17480, P09936, P15374, Q9Y3C8, P19224, P16662, P07911, Q8TCY9, Q9Y6N9, Q13107, P63027, Q15836, P18206, P55072, P21796, P08670, P04275, O75083, Q14191, P98170, Q13426, P13010, P12956, P67809, Q9Y2T7, O43829, Q13105, Q15915, O95409, Q8N9L1, Q9UDV7, Q9Y3S2, Q9UL40, Q14966, Q9H0M5, Q9Y5V0, Q96C28, Q9H5H4.

In a further preference, the peptide used for the inventive compound (e.g. peptide P or $P_a$ or $P_b$) comprises an epitope or epitope part (e.g. at least two, preferably at least three, more preferably at least four, even more preferably at least five, yet even more preferably at least six, especially at least seven or even at least eight amino acids) of one of the following histocompatibility antigens identified by their UniProt accession code:

P04233, O15523, O14602, Q30201, P01891, P01892, P04439, P05534, P10314, P10316, P13746, P16188, P16189, P16190, P18462, P30443, P30447, P30450, P30453, P30455, P30456, P30457, P30459, P30512, Q09160, P01889, P03989, P10319, P18463, P18464, P18465, P30460, P30461, P30462, P30464, P30466, P30475, P30479, P30480, P30481, P30483, P30484, P30485, P30486, P30487, P30488, P30490, P30491, P30492, P30493, P30495, P30498, P30685, Q04826, Q29718, Q29836, Q29940, Q31610, Q31612, Q95365, P04222, P10321, P30499, P30501, P30504, P30505, P30508, P30510, Q07000, Q29865, Q29960, Q29963, Q95604, Q9TNN7, P28067, P28068, P06340, P13765, P20036, P04440, P01909, P01906, P01920, P05538, P01903, P01911, P01912, P04229, P13760, P13761, P20039, Q29974, Q30134, Q30167, Q5Y7A7, Q95IE3, Q9GIY3, Q9TQE0, P79483, P13762, Q30154, P13747, P30511, P17693, Q9BY66, Q29983, Q29980, P22090, Q03519, O14607, P08048.

In another preference, the peptide used for the inventive compound (e.g. peptide P or $P_a$ or $P_b$) comprises an epitope or epitope part (e.g. at least two, preferably at least three, more preferably at least four, even more preferably at least five, yet even more preferably at least six, especially at least seven or even at least eight amino acids) of one of the following antigens of gene delivery vectors identified by their UniProt accession code:
A9RAI0, B5SUY7, 041855, 056137, 056139, P03135, P04133, P04882, P08362, P10269, P12538, P69353, Q5Y9B2, Q5Y9B4, Q65311, Q6JC40, Q6VGT5, Q8JQF8, Q8JQG0, Q98654, Q9WBP8, Q9YIJ1.

In yet another preference, the peptide used for the inventive compound (e.g. peptide P or $P_a$ or $P_b$) comprises an epitope or epitope part (e.g. at least two, preferably at least three, more preferably at least four, even more preferably at least five, yet even more preferably at least six, especially at least seven or even at least eight amino acids) of one of the following antigens of gene delivery vectors identified by their UniProt accession code:
A9RAI0, B5SUY7, O41855, O56137, O56139, P03135, P04133, P04882, P08362, P10269, P12538, P69353, Q5Y9B2, Q5Y9B4, Q65311, Q6JC40, Q6VGT5, Q8JQF8, Q8JQG0, Q98654, Q9WBP8, Q9YIJ1.

In even yet another preference, the peptide used for the inventive compound (e.g. peptide P or $P_a$ or $P_b$) comprises an epitope or epitope part (e.g. at least two, preferably at least three, more preferably at least four, even more preferably at least five, yet even more preferably at least six, especially at least seven or even at least eight amino acids) of one of the following antigens of drugs/active agents identified in Table 2:

| Drug/Active agent | Sequence available in | Is an international non-proprietary name (INN)? | Exemplary prescription product |
|---|---|---|---|
| Abciximab | DrugBank | YES | |
| Adalimumab | DrugBank | YES | |
| Aflibercept | DrugBank | YES | |
| Agalsidase beta | DrugBank | YES | |
| Albiglutide | DrugBank | YES | |
| Albutrepenonacog alfa | DrugBank | YES | |
| Aldesleukin | DrugBank | YES | |
| Alglucosidase alfa | DrugBank | YES | |
| Anakinra | DrugBank | YES | |
| Asfotase Alfa | DrugBank | YES | |
| Atacicept | DrugBank | YES | |
| Atezolizumab | DrugBank | YES | |
| Becaplermin | DrugBank | YES | |
| Belatacept | DrugBank | YES | |
| Bevacizumab | DrugBank | YES | |
| Cerliponase alfa | DrugBank | YES | |
| Cetuximab | DrugBank | YES | |
| Choriogonadotropin alfa | DrugBank | YES | |
| Denileukin diftitox | DrugBank | YES | |
| Dulaglutide | DrugBank | YES | |
| Elapegademase | DrugBank | YES | |
| Elosulfase alfa | DrugBank | YES | |
| Emicizumab | DrugBank | YES | |
| Eptacog alfa | DrugBank | YES | |
| Erenumab | DrugBank | YES | |
| Etanercept | DrugBank | YES | |
| Filgrastim | DrugBank | YES | |
| Galsulfase | DrugBank | YES | |
| Ibritumomab tiuxetan | DrugBank | YES | |
| Idarucizumab | DrugBank | YES | |

(continued)

| Drug/Active agent | Sequence available in | Is an international non-proprietary name (INN)? | Exemplary prescription product |
|---|---|---|---|
| Idursulfase | DrugBank | YES | |
| interferon alfa-2b | DrugBank | YES | |
| interferon alfacon-1 | DrugBank | YES | |
| Ipilimumab | DrugBank | YES | |
| Ixekizumab | DrugBank | YES | |
| Laronidase | DrugBank | YES | |
| Lutropin alfa | DrugBank | YES | |
| Mecasermin | DrugBank | YES | |
| Metreleptin | DrugBank | YES | |
| Ofatumumab | DrugBank | YES | |
| Omalizumab | DrugBank | YES | |
| Oportuzumab monatox | DrugBank | YES | |
| Oprelvekin | DrugBank | YES | |
| Palifermin | DrugBank | YES | |
| Pegademase | DrugBank | YES | |
| Pegloticase | DrugBank | YES | |
| Rasburicase | DrugBank | YES | |
| Rilonacept | DrugBank | YES | |
| Rituximab | DrugBank | YES | |
| Romiplostim | DrugBank | YES | |
| Sebelipase alfa | DrugBank | YES | |
| Tagraxofusp | DrugBank | YES | |
| Tasonermin | DrugBank | YES | |
| Thyrotropin alfa | DrugBank | YES | |
| Trastuzumab | DrugBank | YES | |
| Turoctocog alfa | DrugBank | YES | |
| Alpha-1-proteinase inhibitor | DrugBank | | Aralast NP |
| Antihemophilic factor human | DrugBank | | Hemofil M |
| Drotrecogin alfa | DrugBank | | Xigris |
| Erythropoietin | DrugBank | | Abseamed |
| Follitropin | DrugBank | | Bemfola |
| Interferon alpha-1 | DrugBank | | MultiferonT |
| Interferon alpha-10 | DrugBank | | MultiferonT |
| Interferon alpha-14 | DrugBank | | MultiferonT |
| Interferon alpha-2 | DrugBank | | MultiferonT |
| Interferon alpha-21 | DrugBank | | MultiferonT |
| Interferon alpha-8 | DrugBank | | MultiferonT |
| Interferon beta-1a | DrugBank | | Avonex |
| interferon gamma-1b | DrugBank | | Actimmune |
| Somatotropin | DrugBank | | Gentropin |
| Corifollitropin alfa | KEGG | YES | |
| Efmoroctocog alfa | KEGG | YES | |
| Eftrenonacog alfa | KEGG | YES | |
| Evolocumab | KEGG | YES | |
| Natalizumab | KEGG | YES | |
| Taliglucerase alfa | KEGG | YES | |
| Teprotumumab | KEGG | YES | |
| Velmanase alpha | UniProt O00754 | YES | |

(continued)

| Drug/Active agent | Sequence available in | Is an international non-proprietary name (INN)? | Exemplary prescription product |
|---|---|---|---|
| Factor XIII (alpha and beta chain) | UniProt P00488 & P05160 | | Corifact |
| E coli Asparaginase | UniProt P00805 | | Elspar |
| Antithrombin III | UniProt P01008 | YES | |
| Beta-nerve growth factor (NGF) | UniProt P01138 | | |
| Parathyroid hormone | UniProt P01270 | YES | |
| insulin | UniProt P01308 | YES | |
| Alglucerase | UniProt P04062 | YES | |
| Von Willebrand Factor Human | UniProt P04275 | | Humate-P |
| Plasma protease C1 inhibitor | UniProt P05155 | | Berinert |
| Fibroblast growth factor 2 (FGF2) | UniProt P09038 | | |
| Granulocyte colony-stimulating factor (G-CSF) | UniProt P09919 | | |
| Sphingomyelin phosphodiesterase | UniProt P17405 | | |
| Brain-derived neurotrophic factor (BDNF) | UniProt P23560 | | |
| Glial cell line-derived neurotrophic factor (GDNF) | UniProt P39905 | | |

DrugBank (https://www.drugbank.ca/); KEGG: Kyoto Encyclopedia of Genes and Genomes (https://www.genome.jp/kegg/).

The respective DrugBank and KEGG database accession numbers are listed below in Table 3 (DrugBank and KEGG database versions as of 20 March 2019):

> ENZYMES

| | |
|---|---|
| DB00058 | Alpha-1-proteinase inhibitor |
| DB00088 | Ceredase / Alglucerase |
| DB00053 | Imiglucerase / Cerezyme |
| D09675(KEGG) | Taliglucerase alfa |
| DB00061 | Pegademase Deaminase bovine |
| DB00103 | Agalsidase beta Fabry |
| DB01272 | Alglucosidase alfa Pompe |
| DB00090 | Laronidase $\alpha$-L-Iduronidase Hurler, MPS I |
| DB01271 | Idursulfase Iduronate-2-Sulfatase M Hunter, MPS II |
| DB09051 | Elosulfase alfa N-Acetylgalactosamine-6 Sulfatase Morquio Snydr A, MPS IVA |
| DB01279 | Galsulfase N-Acetylgalactosamine-4 Sulfatase Maroteaux-Lamy, MPS VI |
| DB11563 | Sebelipase alfa Lysosomal Acid Lipase Wolman, LAL Deficiency |
| DB13173 | Cerliponase alfa Batten disease |
| DB11563 | Sebelipase alfa Lysosomal acid lipase deficiency (LAL-D) |
| DB09105 | Asfotase Alfa Perinatal/infantile- and juvenile-onset hypophosphatasia (HPP) |
| DB14712 | Elapegademase Severe combined immunodeficiency disease (SCID) |
| D10820(KEGG) | Olipudase alpha Niemann Pick |
| D11024 | Velmanase alpha (alpha-Mannosidosis) |
| DB00049 | Rasburicase (Elitec) urate-oxidase |
| DB09208 | Pegloticase (procine-like uricase) |

>COAGULATION FACTORS

| | |
|---|---|
| DB09109 | Turoctocog alfa (modified FVIII) |
| DB00055 | Drotrecogin alfa activated human protein C |
| DB13923 | Emicizumab mimics FVIII |
| DB00036 | Coagulation factor VIIa |
| DB00025 | Antihemophilic factor |
| DB13133 | Von Willebrand Factor Human (Vovendi) |
| D10831(KEGG) | Susoctocog alfa |
| DB00025 | hu rec FVIII |
| DB13152 | Coagulation Factor IX Human |

>CYTOKINES:

| | |
|---|---|
| DB00038 | Oprelvekin (rec IL11) |
| DB00041 | Aldesleukin (IL2) |
| DB06372 | Rilonacept (IL1-inhibitor) |
| DB00026 | Anakinra (IL1Ra) |
| DB00004 | Denileukin diftitox |
| DB00060 | Interferon beta 1a |
| DB05258 | Interferon alpha |
| DB00105 | Interferon alpha 2b |
| DB00069 | Interferon alphacon 1 |
| DB00033 | Interferon gamma-1b |
| DB00034 | Interferon Alfa-2a, Recombinant |

>HORMONES:

| | |
|---|---|
| DB00024 | Thyrotropin alfa |
| DB00097 | Choriogonadotropin alfa |
| DB00066 | Follitropin |
| DB00044 | Lutropin alfa |
| DB00052 | Somatotropin |
| DB09043 | Albiglutide glucagon-like peptide-1 agonist (GLP-1) |
| DB09046 | Metreleptin (Leptin homologue) |
| D08895 (KEGG) | Corifollitropin alfa |

>GROWTH FACTORS:

| | |
|---|---|
| DB00099 | Filgrastim (G-CSF) |
| DB01277 | Mecasermin |
| DB00039 | Palifermin |
| DB00102 | Becaplermin (PDGF) |
| DB00039 | Palifermin (KGF) |
| DB11626 | Tasonermin (TGF alpha) |

>FUSION PROTEINS:

| | |
|---|---|
| DB08885 | Aflibercept (VEGF-R/ Fc fusion) |
| DB06372 | Rilonacept (IL-1 R / Fc fusion) |
| DB05332 | Romiplostim (dimer Fc-pep fusion [peptibody], binding thrombopoietin rec) |
| DB14731 | Tagraxofusp (IL-3 conjugated truncated diphtheria toxin) |

(continued)

| DB01281 | Abatacept (Fc hinge fusion to CTLA-4) |
| D10830 (KEGG) | Efmoroctocog alfa (FVIII-Fc fusion) |
| D10522 (KEGG) | Eftrenonacog alfa (human factor IX (FIX)-Fc fusion) |
| DB06372 | Rilonacept (IL-1R & IL-1R access prot Fc fusion) |
| DB14731 | Tagraxofusp (IL-3 conjugated truncated diphtheria toxin) |
| DB06681 | Belatacept (CTLA-4 / Fc fusion) |
| DB06399 | Atacicept (extracellular ligand binding portion of TACI) |
| DB09043 | GLP-1 receptor agonist-albumin fusion |
| DB13884 | Albutrepenonacog alfa Recombinant factor IX albumin fusion (Idelvion) |
| DB08885 | Aflibercept VEGFR Fc-fusion (Zaltrap) |
| DB09045 | Dulaglutide Glucagon like pep 1 receptor agonist Fc-fusion (Trulicity) |
| DB06681 | Belatacept CTLA-4 Fc-fusion (Nulojix) |
| DB08885 | Aflibercept VEGFR Fc-fusion (Eylea) |
| DB00005 | Etanercept Fc fusion [TNFR Fc-fusion] |
| D10830 (KEGG) | Efmoroctocog alfa Rec F VIII Fc-fusion Elocta |
| D10522 (KEGG) | Eftrenonacog alfa Rec FIX Fc fusion (Alprolix) |
| DB09105 | Asfotase Alfa Fc fusion/enzyme asfotase-alfa [Strensiq] |

### >THERAPEUTIC mABs:

| D06886 (KEGG) | Natalizumab |
| DB00073 | Rituximab |
| DB00051 | Adalimumab/ Humira |
| DB06186 | Ipilimumab |
| DB00072 | Herceptin/Trastuzumab |
| DB00112 | Avastin/Bevacizumab |
| DB00005 | Etanercept (Enbrel) |
| D10557 (KEGG) | Evolocumab |
| DB11569 | Ixekizumab (hulgG4 anti interleukin-17A) |
| DB00043 | Omalizumab |
| D09680 (KEGG) | Teprotumumab |

### >ANTIBODY-FRAGMENTS & DERIVATIVES:

| DB09264 | Idarucizumab (Fab) |
| DB00002 | Cetuximab (epidermal growth factor receptor binding FAB) |
| DB05319 | Oportuzumab monatox scFv-Toxin fusion |
| DB00078 | Ibritumomab (murine IgG1 kappa) tiuxetan |
| DB00054 | Abciximab (chimaeric human/mouse mAB) |
| DB00073 | Rituximab (hu IgG1kappa) |
| DB06650 | Ofatumumab (hu IgG) |
| DB14039 | Erenumab (antag. calcitonin gene-rel pep. rec [CGRPR] migraine) |
| DB13923 | Emicizumab (mimics the function of the coagulation Factor VIII) |
| DB11595 | Atezolizumab (Fc-engineered, hum. mAB recognizing PD-L1) |
| DB09264 | Idarucizumab - Fab (inactivates anticoagulant dabigatran) |

Drugs/active agents on which the present invention can be applied (i.e. drugs/active agents leading to undesirable antibodies which can be depleted by the compound of present invention) are also disclosed e.g. in Spiess et al 2015 and Runcie et al 2018. They may also be a scFv, Fab2, Fab3, Bis-scFv, bivalent minibody, diabody, triabody or tetrabody. Further, such drugs/active agents may be an affibody molecule (Protein Data Bank: 1LP1), affimer (Protein Data Bank: 1NB5), affitin molecule (Protein Data Bank: 4CJ2), anticalin molecule (Protein Data Bank: 4GH7), atrimer molecule (Protein Data Bank: 1TN3), fynomer (Protein Data Bank: 1M27), armadillo repeat protein (Protein Data Bank: 4DB9), Kunitz domain inhibitor (Protein Data Bank: 1ZR0), knottin molecule (Protein Data Bank: 2IT7), designed ankyrin repeat protein (Protein Data Bank: 2Q4J); Protein Databank (PDB) version as of 20 March 2019. Further suitable drugs/active agents are disclosed e.g. in WO 2017/220569 A1, WO 2017/087589 A2, US82100547 and EP 1697421 A2 (in particular SEQ ID NO: 1 thereof). As above, the peptide used for the inventive compound (e.g. peptide P or $P_a$ or $P_b$) may comprise an epitope or epitope part (e.g. at least two, preferably at least three, more preferably at least four, even more preferably at least five, yet even more preferably at least six, especially at least seven or even at least eight amino acids) of the amino acid sequences of any one of the drugs/active agents disclosed in the aforementioned sources.

It is also highly preferred that the peptides used for the inventive compound do not bind to any HLA Class I or HLA Class II molecule (i.e. of the individual to be treated, e.g. human), in order to prevent presentation and stimulation via a T-cell receptor in vivo and thereby induce an immune reaction. It is generally not desired to involve any suppressive (or stimulatory) T-cell reaction in contrast to antigen-specific immunologic tolerization approaches. Therefore, to avoid T-cell epitope activity as much as possible, the peptides of the compound of the present invention (e.g. peptide P or $P_a$ or $P_b$ or $P_1$ or $P_2$) preferably fulfil one or more of the following characteristics:

- To reduce the probability for a peptide used in the compound of the present invention to bind to an HLA Class II or Class I molecule, the peptide (e.g. peptide P or $P_a$ or $P_b$ or $P_1$ or $P_2$) has a preferred length of 4-8 amino acids, although somewhat shorter or longer lengths are still acceptable.

- To further reduce the probability that such a peptide binds to an HLA Class II or Class I molecule, it is preferred to test the candidate peptide sequence by HLA binding prediction algorithms such as NetMHCII-2.3 (reviewed by Jensen et al 2018). Preferably, a peptide (e.g. peptide P or $P_a$ or $P_b$ or $P_1$ or $P_2$) used in the compound of the present invention has (predicted) HLA binding (IC50) of at least 500 nM. More preferably, HLA binding (IC50) is more than 1000 nM, especially more than 2000 nM (cf. e.g. Peters et al 2006). In order to decrease the likelihood of HLA Class I binding, NetMHCpan 4.0 may also be applied for prediction (Jurtz et al 2017).

- To further reduce the probability that such a peptide binds to an HLA Class I molecule, the NetMHCpan Rank percentile threshhold can be set to a background level of 10% according to Koşaloğlu-Yalçın et al 2018 (PMID: 30377561). Preferably, a peptide (e.g. peptide P or $P_a$ or $P_b$ or $P_1$ or $P_2$) used in the compound of the present invention therefore has a %Rank value of more than 3, preferably more than 5, more preferably more than 10 according to the NetMHCpan algorithm.

- To further reduce the probability that such a peptide binds to an HLA Class II molecule, it is beneficial to perform in vitro HLA-binding assays commonly used in the art such as for example for example refolding assays, iTopia, peptide rescuing assays or array-based peptide binding assays. Alternatively, or in addition thereto, LC-MS based analytics can be used, as e.g. reviewed by Gfeller et al 2016.

For stronger reduction of the titre of the undesired antibodies, it is preferred that the peptides used in the present invention are circularized. Accordingly, in a preferred embodiment, at least one occurrence of P is a circularized peptide. Preferably at least 10% of all occurrences of P are circularized peptides, more preferably at least 25% of all occurrences of P are circularized peptides, yet more preferably at least 50% of all occurrences of P are circularized peptides, even more preferably at least 75% of all occurrences of P are circularized peptides, yet even more preferably at least 90% of all occurrences of P are circularized peptides or even at least 95% of all occurrences of P are circularized peptides, especially all of the occurrences of P are circularized peptides. Several common techniques are available for circularization of peptides, see e.g. Ong et al 2017.

Further, for stronger reduction of the titre of the undesired antibodies relative to the amount of scaffold used, in a preferred embodiment of the compound of the present invention, independently for each of the peptide n-mers, n is at least 2, more preferably at least 3, especially at least 4. Usually, in order to avoid complexities in the manufacturing process, independently for each of the peptide n-mers, n is less than 10, preferably less than 9, more preferably less than 8, even more preferably less than 7, yet even more preferably less than 6, especially less than 5. To benefit from higher avidity through divalent binding of the undesired antibody, it is highly preferred that, for each of the peptide n-mers, n is 2.

For multivalent binding of the undesired antibodies, it is advantageous that the peptide dimers or n-mers are spaced by a hydrophilic, structurally flexible, immunologically inert, non-toxic and clinically approved spacer such as (hetero-)bifunctional and -trifunctional Polyethylene glycol (PEG) spacers (e.g. NHS-PEG-Maleimide) - a wide range of PEG chains is available and PEG is approved by the FDA. Alternatives to PEG linkers such as immunologically inert and non-toxic synthetic polymers or glycans are also suitable. Accordingly, in the context of the present invention, the spacer (e.g. spacer S) is preferably selected from PEG molecules or glycans. For instance, the spacer such as PEG can be introduced during peptide synthesis. Such spacers (e.g. PEG spacers) may have a molecular weight of e.g. 10000 Dalton.

Preferably, each of the peptide n-mers is covalently bound to the biopolymer scaffold, preferably via a linker each.

As used herein, the linker may e.g. be selected from disulphide bridges and PEG molecules.

According to a further preferred embodiment of the inventive compound, independently for each occurrence, P is $P_a$ or $P_b$.

Furthermore, it is preferred when in the first peptide n-mer, each occurrence of P is $P_a$ and, in the second peptide n-mer, each occurrence of P is $P_b$. Alternatively, or in addition thereto, $P_a$ and/or $P_b$ is circularized.

Divalent binding is particularly suitable to reduce antibody titres. According, in a preferred embodiment,

the first peptide n-mer is $\mathbf{P_a}$ - $\mathbf{S}$ - $\mathbf{P_a}$ and the second peptide n-mer is $\mathbf{P_a}$ - $\mathbf{S}$ - $\mathbf{P_a}$;

the first peptide n-mer is $\mathbf{P_a}$ - $\mathbf{S}$ - $\mathbf{P_a}$ and the second peptide n-mer is $\mathbf{P_b}$ - $\mathbf{S}$ - $\mathbf{P_b}$;

the first peptide n-mer is $\mathbf{P_b}$ - $\mathbf{S}$ - $\mathbf{P_b}$ and the second peptide n-mer is $\mathbf{P_b}$ - $\mathbf{S}$ - $\mathbf{P_b}$;

the first peptide n-mer is $\mathbf{P_a}$ - $\mathbf{S}$ - $\mathbf{P_b}$ and the second peptide n-mer is $\mathbf{P_a}$ - $\mathbf{S}$ - $\mathbf{P_b}$;

the first peptide n-mer is $\mathbf{P_a}$ - $\mathbf{S}$ - $\mathbf{P_b}$ and the second peptide n-mer is $\mathbf{P_a}$ - $\mathbf{S}$ - $\mathbf{P_a}$; or

the first peptide n-mer is $\mathbf{P_a}$ - $\mathbf{S}$ - $\mathbf{P_b}$ and the second peptide n-mer is $\mathbf{P_b}$ - $\mathbf{S}$ - $\mathbf{P_b}$.

For increasing effectivity, in particular in autoimmune disease (which is usually based on polyclonal antibodies, see above), in a preferred embodiment the first peptide n-mer is different from the second peptide n-mer. For similar reasons, preferably, the peptide $P_a$ is different from the peptide $P_b$, preferably wherein the peptide $P_a$ and the peptide $P_b$ are two different epitopes of the same antigen or two different epitope parts of the same epitope.

Especially for better targeting of polyclonal antibodies, it is advantageous when the peptide $P_a$ and the peptide $P_b$ comprise the same amino-acid sequence fragment, wherein the amino-acid sequence fragment has a length of at least 2 amino acids, preferably at least 3 amino acids, more preferably at least 4 amino acids, yet more preferably at least 5 amino acids, even more preferably at least 6 amino acids, yet even more preferably at least 7 amino acids, especially at least 8 amino acids or even at least 9 amino acids.

Further, for stronger reduction of the titre of the undesired antibodies relative to the amount of scaffold used, the compound comprises a plurality of said first peptide n-mer (e.g. up to 10 or 20 or 30) and/or a plurality of said second peptide n-mer (e.g. up to 10 or 20 or 30).

For stronger reduction of the titre of the undesired antibodies relative to the amount of scaffold used, the compound may also comprise at least

a third peptide n-mer of the general formula:

$$\mathbf{P}\ (\ -\ \mathbf{S}\ -\ \mathbf{P}\ )_{(n-1)}\ ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_c$, wherein $P_c$ is a peptide with a sequence length of 2-13 amino acids, preferably 3-11 amino acids, more preferably 4-9 amino acids, especially 5-8 amino acids,

more preferably wherein $P_c$ is circularized;

a fourth peptide n-mer of the general formula:

$$\mathbf{P}\ (\ -\ \mathbf{S}\ -\ \mathbf{P}\ )_{(n-1)}\ ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_d$, wherein $P_d$ is a peptide with a sequence length of 2-13 amino acids, preferably 3-11 amino acids, more preferably 4-9 amino acids, especially 5-8 amino acids,

more preferably wherein $P_d$ is circularized;

a fifth peptide n-mer of the general formula:

$$P (-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer, preferably wherein each occurrence of P is $P_e$, wherein $P_e$ is a peptide with a sequence length of 2-13 amino acids, preferably 3-11 amino acids, more preferably 4-9 amino acids, especially 5-8 amino acids, more preferably wherein $P_e$ is circularized;

a sixth peptide n-mer of the general formula:

$$P (-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer, preferably wherein each occurrence of P is $P_f$, wherein $P_f$ is a peptide with a sequence length of 2-13 amino acids, preferably 3-11 amino acids, more preferably 4-9 amino acids, especially 5-8 amino acids, more preferably wherein $P_f$ is circularized;

a seventh peptide n-mer of the general formula:

$$P (-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer, preferably wherein each occurrence of P is $P_g$, wherein $P_g$ is a peptide with a sequence length of 2-13 amino acids, preferably 3-11 amino acids, more preferably 4-9 amino acids, especially 5-8 amino acids, more preferably wherein $P_g$ is circularized;

a eigth peptide n-mer of the general formula:

$$P (-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_h$, wherein $P_h$ is a peptide with a sequence length of 2-13 amino acids, preferably 3-11 amino acids, more preferably 4-9 amino acids, especially 5-8 amino acids,

more preferably wherein $P_h$ is circularized;

a ninth peptide n-mer of the general formula:

$$P (-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_i$, wherein $P_i$ is a peptide with a sequence length of 2-13 amino acids, preferably 3-11 amino acids, more preferably 4-9 amino acids, especially 5-8 amino acids,

more preferably wherein $P_i$ is circularized;

a tenth peptide n-mer of the general formula:

$$P\ (\ -\ S\ -\ P\ )_{(n-1)}\ ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_j$, wherein $P_j$ is a peptide with a sequence length of 2-13 amino acids, preferably 3-11 amino acids, more preferably 4-9 amino acids, especially 5-8 amino acids,

more preferably wherein $P_j$ is circularized.

Peptides $P_c$-$P_j$ may have one or more of same features (e.g. sequence) as disclosed herein for peptides $P_a$ and $P_b$.

As also illustrated above, it is highly preferred when the compound of the present invention is non-immunogenic in a mammal, preferably in a human, in a non-human primate, in a sheep, in a pig, in a dog or in a rodent.

In the context of the present invention, a non-immunogenic compound preferably is a compound wherein the biopolymer scaffold (if it is a protein) and/or the peptides (of the peptide n-mers) have an IC50 higher than 100 nM, preferably higher than 500 nM, even more preferably higher than 1000 nM, especially higher than 2000 nM, against HLA-DRB1_0101 as predicted by the NetMHCII-2.3 algorithm. The NetMHCII-2.3 algorithm is described in detail in Jensen et al, which is incorporated herein by reference. The algorithm is publicly available under http://www.cbs.dtu.dk/services/NetMHCII-2.3/. Even more preferably, a non-immunogenic compound (or pharmaceutical composition) does not bind to any HLA and/or MHC molecule (e.g. in a mammal, preferably in a human, in a non-human primate, in a sheep, in a pig, in a dog or in a rodent; or of the individual to be treated) in vivo.

According to a further preference, the compound is for intracorporeal sequestration (or intracorporeal depletion) of at least one antibody in an individual, preferably in the bloodstream of the individual and/or for reduction of the titre of at least one antibody in the individual, preferably in the bloodstream of the individual.

In another preferred embodiment, the entire sequence, optionally with the exception of an N-terminal and/or C-terminal cysteine, of at least one occurrence of P, preferably of at least 10% of all occurrences of P, more preferably of at least 25% of all occurrences of P, yet more preferably of at least 50% of all occurrences of P, even more preferably of at least 75% of all occurrences of P, yet even more preferably of at least 90% of all occurrences of P or even of at least 95% of all occurrences of P, especially of all of the occurrences of P, is identical to a sequence fragment of a protein, wherein the protein is identified by one of the UniProt accession codes disclosed herein; optionally wherein the sequence fragment comprises at most four, preferably at most three, more preferably at most two, especially at most one amino acid substitutions (e.g. for the purposes mentioned above, such as creating mimotopes).

In another preferred embodiment, the entire sequence, optionally with the exception of an N-terminal and/or C-terminal cysteine, of peptide $P_a$ is identical to a sequence fragment of a protein, wherein the protein is identified by one of the UniProt accession codes disclosed herein; optionally wherein said sequence fragment comprises at most four, preferably at most three, more preferably at most two, especially at most one amino acid substitutions (e.g. for the purposes mentioned above, such as creating mimotopes).

In another preferred embodiment, the entire sequence, optionally with the exception of an N-terminal and/or C-terminal cysteine, of peptide $P_b$ is identical to a sequence fragment of a protein, wherein the protein is identified by one of the UniProt accession codes disclosed herein; optionally wherein said sequence fragment comprises at most four, preferably at most three, more preferably at most two, especially at most one amino acid substitutions (e.g. for the purposes mentioned above, such as creating mimotopes).

In another preferred embodiment, the entire sequence, optionally with the exception of an N-terminal and/or C-terminal cysteine, of peptide $P_a$ is identical to a sequence fragment of a protein and the entire sequence, optionally with the exception of an N-terminal and/or C-terminal cysteine, of peptide $P_b$ is identical to the same or another, preferably another, sequence fragment of the same protein, wherein the protein is identified by one of the UniProt accession codes listed herein; optionally wherein said sequence fragment and/or said another sequence fragment comprises at most four, preferably at most three, more preferably at most two, especially at most one amino acid substitutions (e.g. for the purposes mentioned above, such as creating mimotopes).

Myasthenia gravis is an autoimmune neuromuscular disorder mediated by autoantibodies that cause a broad spectrum of several clinical symptoms from mild muscle weakness to a life-threatening myasthenic crisis with breathing problems. Around 80% of myasthenic patients develop anti nicotinic acetylcholine receptor (AChR) antibodies that lead to complement-mediated damaging of the postsynaptic membrane (Howard 2018), direct AChR blocking or receptor endocytosis. These disease-causing autoantibodies are mainly directed to defined immunogenic regions AChR or MuSK (Ruff 2018). They represent a good example for functionally well characterized, disease-causing autoantibodies. Although general immunosuppressive or B-cell targeting strategies exist, a strategy is needed whereby only diseases-causing antibodies (rather than all, mostly protective antibodies) are rapidly inactivated or depleted (especially in a myasthenic crisis), since neither of the generally immunosuppressive treatments with corticoids, IVIG or by plasma exchange are satisfactory. So far, no convenient therapeutic intervention exists that can deplete or neutralize disease causing antibodies in myasthenia gravis rapidly and selectively.

Non-selective B-cell targeting or immunotherapeutic approaches are not yet an established therapeutic option for the treatment of myasthenia gravis. Alternatively, few intra- and extracorporeal selective antibody depletion or B-cell suppression strategies targeting disease-causing antibodies in myasthenia gravis were proposed using indirect or direct targeting approaches against disease causing antibodies (see e.g. Homma 2017 and Lazaridis 2017). In addition, an AChR-specific immunosuppressive therapy using an adjuvanted AChR vaccine was proposed (Luo 2015). However, there remains an urgent need for a comparatively effective and safe and rapidly acting selective antibody depletion therapy.

Accordingly, as mentioned in the summary, the present invention also relates to a compound (for use in the prevention or treatment of myasthenia gravis, especially in a myasthenic crisis), preferably for the sequestration (or depletion) of anti human muscle nicotinic acetylcholine receptor (AChR) antibodies, anti human muscle-specific receptor tyrosine kinase antibodies and/or anti human low-density lipoprotein receptor related protein 4 antibodies present in a human individual, the compound comprising a biopolymer scaffold and at least two peptides with a sequence length of 7-13 amino acids, wherein each of the peptides independently comprises a 7-13 amino-acid sequence fragment of the AChR subunit alpha sequence identified by UniProt accession code P02708 or of the muscle-specific receptor tyrosine kinase sequence identified by UniProt accession code 015146 or of the low-density lipoprotein receptor related protein 4 sequence identified by UniProt accession code O75096, wherein the peptides are covalently bound to the biopolymer scaffold, preferably via a linker, wherein the biopolymer scaffold is selected from the group consisting of human globulins and human albumin.

According to a particular preference, in particular for stronger reduction of the titre of the undesired antibodies relative to the amount of scaffold used, the at least two peptides comprise a peptide $P_1$ and a peptide $P_2$, wherein $P_1$ and $P_2$ comprise the same 7-13 amino-acid sequence fragment of AChR subunit alpha or of the muscle-specific receptor tyrosine kinase or of low-density lipoprotein receptor related protein 4, wherein $P_1$ and $P_2$ are present in form of a peptide dimer $\mathbf{P_1}$ - $\mathbf{S}$ - $\mathbf{P_2}$, wherein S is a non-peptide spacer, wherein the peptide dimer is covalently bound to the biopolymer scaffold, preferably via a linker.

Preferably, said 7-13 amino-acid sequence fragment of AChR subunit alpha is a fragment of the sequence consisting of amino acids 21-255 of the AChR subunit alpha sequence identified by UniProt accession code P02708.

In a further preferred embodiment, said 7-13 amino-acid sequence fragment of AChR subunit alpha is a fragment of the sequence LKWNPDDYGGVKKIHIPSEK (SEQ ID NO: 1), preferably of the sequence WNPDDYGGVK (SEQ ID NO: 2) or VKKIHIPSEK (SEQ ID NO: 3).

In a further preferred embodiment, the peptides have a sequence length of 8-13 amino acids, preferably 9-12 amino acids, more preferably 10-12 amino acids, especially wherein the peptides consist of the sequence VKKIHIPSEKG (SEQ ID NO: 4) optionally with an N-terminal and/or C-terminal cysteine residue.

According to a further preferred embodiment, the compound further comprises at least one peptide with a sequence length of 7-13 amino acids, wherein the at least one peptide comprises a 7-13 amino-acid sequence fragment of the muscle-specific receptor tyrosine kinase sequence identified by UniProt accession code 015146 or of the low-density lipoprotein receptor related protein 4 sequence identified by UniProt accession code O75096, wherein the at least one peptide is covalently bound to the biopolymer scaffold, preferably via a linker.

Furthermore, also for use in the prevention or treatment of myasthenia gravis (especially in a myasthenic crisis), in a preferred embodiment of inventive compound, at least one occurrence of P is $P_a$ and at least one occurrence of P is $P_b$, wherein $P_a$ is a peptide with a sequence length of 5-13, preferably 7-13, amino acids, wherein $P_b$ is a peptide with a sequence length of 5-13, preferably 7-13, amino acids, wherein the entire sequence, optionally with the exception of an N-terminal and/or C-terminal cysteine, of peptide $P_a$ is identical to a sequence fragment of a protein, wherein the protein is identified by UniProt accession code P02708, 015146 or O75096, optionally wherein said sequence fragment comprises at most four, preferably at most three, more preferably at most two, especially at most one amino acid substitutions, wherein the entire sequence, optionally with the exception of an N-terminal and/or C-terminal cysteine, of peptide $P_b$ is identical to a sequence fragment of a protein, wherein the protein is identified by UniProt accession code P02708, 015146 or O75096, optionally wherein said sequence fragment comprises at most four, preferably at most three, more preferably at most two, especially at most one amino acid substitutions.

In embodiments, in particular for $P_a$ and/or $P_b$, said sequence fragment of the protein is a fragment of the sequence consisting of amino acids 21-255 of the AChR subunit alpha sequence identified by UniProt accession code P02708. In further embodiments, in particular for Pa and/or Pb, said sequence fragment of the protein is a fragment of the sequence LKWNPDDYGGVKKIHIPSEK (SEQ ID NO: 1), preferably of the sequence WNPDDYGGVK (SEQ ID NO: 2) or VKKIHIPSEK (SEQ ID NO: 3). In particular, peptide Pa and/or peptide Pb consist of the sequence VKKIHIPSEKG (SEQ ID NO: 4) optionally with an N-terminal and/or C-terminal cysteine residue.

Further, for stronger reduction of the titre of the undesired antibodies related to myasthenia gravis, in a preferred embodiment, the first peptide n-mer is $P_a$ - $S$ - $P_b$ and the second peptide n-mer is $P_a$ - $S$ - $P_b$.

Pre-eclampsia is an exemplary disease of pregnancy that involves not only the placenta, but the entire organism. It occurs in 3-5% of all pregnancies predominantly in pregnant teens and women over 40 and it remains a leading cause of neonatal morbidity and mortality, typically later in pregnancy. An onset of hypertension in women that had no history of high blood pressure, elevated liver enzymes proteinuria, renal failure, low platelets (HELLP syndrome) and cerebral edema with seizures are hallmark of this condition. No specific cures are known, and the exact causes for preeclampsia appear to be complex. In general, therapeutic options are very limited.

Pathogenesis of preeclampsia involves abnormal placental implantation, placental hypoxia combined with the release of circulating factors, alterations of endothelial cell function and involvement of angiogenic factors and inflammatory cytokines. Moreover, the renin angiotensin aldosterone system (RAAS) plays an important role in preeclampsia which has been corroborated by the finding that autoantibodies against the angiotensin II type 1-receptor (AT1-AA) contribute to the underlying pathomechanism (Wallukat 1999).

The repertoire of preeclampsia-associated autoantibody specificities was recently extended e.g. to anti-alpha1-adrenoreceptors, prothrombin, anti-cardiolipin and more recently to GRP50 (Elliott 2016). Elliott and colleagues found an antigenic mimicry mechanism: Preeclampsia patients showed antibody titers against a peptide epitope within the Epstein-Barr virus nuclear antigen 1 (EBNA-1) that cross reacted with the placental GPR50 membrane protein that is expressed in placental tissue. Antibodies to the EBNA-1 antigen are also associated with several other autoimmune diseases such as Systemic Lupus Erythematosus, Multiple Sclerosis and myalgic encephalitis / chronic fatigue syndrome.

Accordingly, as mentioned in the summary, the present invention also relates to a compound, preferably for the sequestration (or depletion) of anti-Epstein-Barr virus nuclear antigen 1 (EBNA-1) antibodies, anti human melatonin-related receptor (GPR50) antibodies and/or anti human type-1 angiotensin II receptor (AT1AR) antibodies present in a human individual, the compound comprising a biopolymer scaffold and at least two peptides with a sequence length of 7-13 amino acids,

wherein each of the peptides independently comprises a 7-13 amino-acid sequence fragment of the EBNA1 sequence identified by UniProt accession code Q1HVF7 or P03211 or of the GPR50 sequence identified by UniProt accession code Q13585 or of the type-1 angiotensin II receptor (AT1AR) sequence identified by UniProt accession code P30556, wherein the peptides are covalently bound to the biopolymer scaffold,

wherein the biopolymer scaffold is selected from the group consisting of human globulins, preferably from the group consisting of human immunoglobulins and human haptoglobin, and human albumin.

The compound can selectively reduce the levels of undesired antibodies that crossreact with a viral antigen (such as EBNA-1) and an endogenous membrane receptor protein (such as GRP50).

According to a particular preference, in particular for stronger reduction of the titre of the undesired antibodies relative to the amount of scaffold used, the at least two peptides comprise a peptide $P_1$ and a peptide $P_2$, wherein $P_1$ and $P_2$ comprise the same 7-13 amino-acid sequence fragment of said EBNA1 sequence or said GPR50 sequence or said AT1AR sequence, wherein $P_1$ and $P_2$ are present in form of a peptide dimer $P_1$ - $S$ - $P_2$, wherein S is a non-peptide spacer, wherein the peptide dimer is covalently bound to the biopolymer scaffold, preferably via a linker.

Preferably, said 7-13 amino-acid sequence fragment is a fragment of the sequence RPQKRPSCIGCKGTH (SEQ ID NO: 5) or RPQKRPSCIGCKGAH (SEQ ID NO: 6), preferably of the sequence KRPSCIGCK (SEQ ID NO: 7).

In a further preferred embodiment, said 7-13 amino-acid sequence fragment is a fragment of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12).

In a further preferred embodiment, the peptides have a sequence length of 8-13 amino acids, preferably 9-12 amino acids, more preferably 10-12 amino acids, especially wherein at least one of the at least two, preferably each of the peptides consist of the sequence GRPQKRPSCIG (SEQ ID NO: 13) optionally with an N-terminal and/or C-terminal cysteine residue.

According to a further preferred embodiment, the compound further comprises at least one peptide with a sequence length of 7-13 amino acids, wherein the at least one peptide comprises a 7-13 amino-acid sequence fragment of the type-1 angiotensin II receptor (AT1AR) sequence identified by UniProt accession code P30556, preferably of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12); wherein the at least one peptide is covalently bound to the biopolymer scaffold, preferably via a linker.

Furthermore, also for use in the prevention or treatment of pre-eclampsia, in a preferred embodiment of inventive compound, at least one occurrence of P is $P_a$ and at least one occurrence of P is $P_b$,
wherein $P_a$ is a peptide with a sequence length of 5-13, preferably 7-13, amino acids,
wherein $P_b$ is a peptide with a sequence length of 5-13, preferably 7-13, amino acids,
wherein the entire sequence, optionally with the exception of an N-terminal and/or C-terminal cysteine, of peptide $P_a$ is identical to a sequence fragment of a protein, wherein the protein is identified by UniProt accession code Q1HVF7, P03211, Q13585 or P30556, optionally wherein said sequence fragment comprises at most four, preferably at most three, more preferably at most two, especially at most one amino acid substitutions,
wherein the entire sequence, optionally with the exception of an N-terminal and/or C-terminal cysteine, of peptide $P_b$ is identical to a sequence fragment of a protein, wherein the protein is identified by UniProt accession code Q1HVF7, P03211, Q13585 or P30556, optionally wherein said sequence fragment comprises at most four, preferably at most three, more preferably at most two, especially at most one amino acid substitutions.

In embodiments, in particular for $P_a$ and/or $P_b$, said sequence fragment of the protein is a fragment of the sequence RPQKRPSCIGCKGTH (SEQ ID NO: 5) or RPQKRPSCIGCKGAH (SEQ ID NO: 6), preferably of the sequence KRPSCIGCK (SEQ ID NO: 7). In further embodiments, in particular for $P_a$ and/or $P_b$, said sequence fragment of the protein is a fragment of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12). In particular, peptide $P_a$ and/or peptide $P_b$ consist of the sequence GRPQKRPSCIG (SEQ ID NO: 13) optionally with an N-terminal and/or C-terminal cysteine residue.

Further, for stronger reduction of the titre of the undesired antibodies related to pre-eclampsia, in a preferred embodiment, the first peptide n-mer is $P_a$ **- S -** $P_b$ and the second peptide n-mer is $P_a$ **-** **S** **-** $P_b$.
Especially in the context of depleting undesired anti-drug antibodies, in yet another preferred embodiment of the present invention, the entire sequence, optionally with the exception of an N-terminal and/or C-terminal cysteine, of at least one occurrence of P, preferably of at least 10% of all occurrences of P, more preferably of at least 25% of all occurrences of P, yet more preferably of at least 50% of all occurrences of P, even more preferably of at least 75% of all occurrences of P, yet even more preferably of at least 90% of all occurrences of P or even of at least 95% of all occurrences of P, especially of all of the occurrences of P, is identical to a sequence fragment of an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-1a, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab; optionally wherein said sequence fragment comprises at most four, preferably at most three, more preferably at most two, especially at most one amino acid substitutions.

Especially in the same context, in another preferred embodiment, the entire sequence, optionally with the exception of an N-terminal and/or C-terminal cysteine, of peptide $P_a$ and/or $P_b$ is identical to a sequence fragment of an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-la, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab; optionally wherein said sequence fragment comprises at most four, preferably at most three, more preferably at most two, especially at most one amino acid substitutions.

Especially in the same context of anti-drug antibodies, in another preferred embodiment, the entire sequence, the entire sequence, optionally with the exception of an N-terminal and/or C-terminal cysteine, of peptide $P_a$ is identical to a sequence fragment of an amino-acid sequence and the entire sequence, optionally with the exception of an N-terminal and/or C-terminal cysteine, of peptide $P_b$ is identical to the same or another, preferably another, sequence fragment of the same amino-acid sequence, wherein the amino-acid sequence is an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-1a, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab; optionally wherein said sequence fragment and/or said another sequence fragment comprises at most four, preferably at most three, more preferably at most two, especially at most one amino acid substitutions.

In an aspect, the present invention relates to a pharmaceutical composition comprising the inventive and at least one pharmaceutically acceptable excipient.

In embodiments, the composition is prepared for intraperitoneal, subcutaneous, intramuscular and/or intravenous administration.

In a preference, the molar ratio of peptide P or $P_a$ or $P_b$ to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.

In another aspect, the compound of the present invention is for use in therapy.

Preferably, the compound is for use in prevention or treatment of an autoimmune disease in an individual having the autoimmune disease or being at risk of developing the autoimmune disease. These autoimmune diseases include neuromyelitis optica, seropositive neuromyelitis optica spectrum disorders, autoimmune-encephalitis, multiple sclerosis, amyotrophic lateral sclerosis, systemic lupus erythematosus dementia, myasthenia gravis, in particular transient neonatal myasthenia gravis, dilatative Cardiomyopathy, pulmonary hypertension, Sjögren's Syndrome, celiac Disease, Graves Disease, Goodpasture Disease, preeclampsia, Behcet's Disease, systemic sclerosis, hypertension, type I diabetes, type II diabetes, systemic lupus erythematosus, anti N-methyl-D-aspartate receptor (NMDAR) encephalitis, antiphospholipid syndrome, membranous nephropathy, primary biliary cholangitis, amyotrophic lateral sclerosis, Chagas disease cardiomyopathy, immune thrombocytopenic purpura, pemphigus vulgaris, bullous pemphigoid, epidermolysis bullosa acquisita and bullous systemic lupus erythematosus.

The compound of the present invention is also useful for prevention or treatment of transplant rejection in an individual having a transplant or eligible for a transplantation.

In another embodiment, the compound is for use in prevention or treatment of adverse reactions based on anti-drug antibodies or anti-gene-delivery vector antibodies in an individual undergoing therapy with the drug or eligible for therapy with the drug, or in an individual undergoing gene therapy or eligible for gene therapy,

Preferably wherein the drug is a peptide or protein, especially selected from the group of enzymes, enzyme inhibitors, antibodies, antibody fragments, antibody mimetics, antibody-drug conjugates, hormones, growth factors, clotting factors and cytokines, preferably wherein the entire sequence, optionally with the exception of an N-terminal and/or C-terminal cysteine, of at least one occurrence of peptide P, or of peptide $P_a$ and/or of peptide $P_b$ is identical to a sequence fragment of an amino-acid sequence of the peptide or protein, optionally wherein said sequence fragment comprises at most four, preferably at most three, more preferably at most two, especially at most one amino acid substitutions. The drug may be e.g. any one of the drugs disclosed herein.

In embodiments, one or more antibodies are present in the individual which are specific for at least one occurrence of peptide P, or for peptide $P_a$ and/or peptide $P_b$, preferably wherein said antibodies are related to said disease.

It is highly preferred that the composition is non-immunogenic in the individual (e.g. it does not comprise an adjuvant).

The composition of the present invention may be administered at a dose of 1-1000 mg, preferably 2-500 mg, more preferably 3-250 mg, even more preferably 4-100 mg, especially 5-50 mg, compound per kg body weight of the individual. Such administration may be intraperitoneally, subcutaneously, intramuscularly or intravenously.

In an aspect, the present invention relates to a method of sequestering (or depleting) one or more antibodies present in an individual, comprising

obtaining a pharmaceutical composition as defined herein, wherein the composition is non-immunogenic in the individual and wherein the one or more antibodies present in the individual are specific for at least one occurrence of P, or for peptide $P_a$ and/or peptide $P_b$; and

administering the pharmaceutical composition to the individual.

In the context of the present invention, the individual (to be treated) may be a non-human animal, preferably a non-human primate, a sheep, a pig, a dog or a rodent, in particular a mouse.

Preferably, the biopolymer scaffold is autologous with respect to the individual, preferably wherein the biopolymer scaffold is an autologous protein (i.e. murine albumin is used when the individual is a mouse).

In embodiments, the individual is administered a heterologous protein, preferably a heterologous antibody such as a nanobody, and wherein the one or more antibodies present in the individual are specific for said heterologous protein, preferably wherein said administering of the heterologous protein is prior to, concurrent with and/or subsequent to said administering of the pharmaceutical composition.

The heterologous protein (in particular a human or humanized antibody) may for instance be for therapy (in particular immunotherapy) of a malignancy or a cancer. In embodiments, the individual may have the malignancy or the cancer and may e.g. be treated or eligible to be treated or designated to be treated with the heterologous protein such as the antibody.

In a preference, the individual is a non-human animal and the heterologous protein is human or humanized such as a human or humanized antibody (e.g. for preclinical testing of a human or humanized biological such as a monoclonal antibody).

In a further preference, the individual is administered a drug and wherein the one or more antibodies present in the individual are specific for said drug, preferably wherein said administering of the drug is prior to, concurrent with and/or subsequent to said administering of the pharmaceutical composition.

The drug may be any drug as disclosed herein.

In embodiments, the individual is healthy.

In another aspect, the present invention relates to a pharmaceutical composition, comprising the compound of the present inevntion and further comprising an active agent such as a protein or a peptide and optionally at least one pharmaceutically acceptable excipient, wherein the active agent comprises a peptide fragment with a sequence length of 2-13 amino acids, preferably 3-11 amino acids, more preferably 4-9 amino acids, especially 5-8 amino acids, and wherein the sequence of at least one occurrence of peptide P, or peptide $P_a$ and/or peptide $P_b$, of the compound is at least 70% identical, preferably at least 75% identical, more preferably at least 80% identical, yet more preferably at least 85% identical, even more preferably at least 90% identical, yet even more preferably at least 95% identical, especially completely identical to the sequence of said peptide fragment.

The active agent may be an enzyme, preferably a human enzyme, an antibody, preferably a human or humanized antibody, a hormone, a growth factor, a clotting factor, a cytokine or a gene delivery vector, in particular as disclosed herein.

This composition is preferably for use in inhibition of an immune reaction, preferably an antibody-mediated immune reaction, against the active agent.

This composition is furthermore preferably non-immunogenic in the individual.

In yet another aspect, the present invention relates to a method of inhibiting an immune reaction to a treatment with an active agent in an individual in need of treatment with the active agent, comprising obtaining a pharmaceutical composition as defined above; wherein the compound of the pharmaceutical composition is non-immunogenic in the individual, and administering the pharmaceutical composition to the individual.

In the context of the present invention, for improved bioavailability, it is preferred that the inventive compound has a solubility in water at 25°C of at least 0.1 $\mu$g/ml, preferably at least 1 $\mu$g/ml, more preferably at least 10 $\mu$g/ml, even more preferably at least 100 $\mu$g/ml, especially at least 1000 $\mu$g/ml.

The term "preventing" or "prevention" as used herein means to stop a disease state or condition from occurring in a patient or subject completely or almost completely or at least to a (preferably significant) extent, especially when the patient or subject or individual is predisposed to such a risk of contracting a disease state or condition.

The pharmaceutical composition of the present invention is preferably provided as a (typically aqueous) solution, (typically aqueous) suspension or (typically aqueous) emulsion. Excipients suitable for the pharmaceutical composition of the present invention are known to the person skilled in the art, upon having read the present specification, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives. Other suitable excipients include any compound that does not itself induce the production of antibodies in the patient (or individual) that are harmful for the patient (or individual). Examples are well tolerable proteins, polysaccharides, polylactic acids, polyglycolic acid, polymeric amino acids and amino acid copolymers. This pharmaceutical composition can (as a drug) be administered via appropriate procedures known to the skilled person (upon having read the present specification) to a patient or individual in need thereof (i.e. a patient or individual having or having the risk of developing the diseases or conditions mentioned herein). The preferred route of administration of said pharmaceutical composition is parenteral administration, in particular through intraperitoneal, subcutaneous, intramuscular and/or intravenous administration. For parenteral administration, the pharmaceutical composition of the present invention is preferably provided in injectable dosage unit form, e.g. as a solution (typically as an aqueous solution), suspension or emulsion, formulated in conjunction with the above-defined pharmaceutically acceptable excipients. The dosage and method of administration, however, depends on the individual patient or individual to be treated. Said pharmaceutical composition can be administered in any suitable dosage known from other biological dosage regimens or specifically evaluated and optimised for a given individual. For example, the active agent may be present in the pharmaceutical composition in an amount from 1 mg to 10 g, preferably 50 mg to 2 g, in particular 100 mg to 1 g. Usual dosages can also be determined on the basis of kg body weight of the patient, for example preferred dosages are in the range of 0.1 mg to 100 mg/kg body weight, especially 1 to 10 mg/kg body weight (per administration session). The administration may occur e.g. once daily, once every other day, once per week or once every two weeks. As the preferred mode of administration of the inventive pharmaceutical composition is parenteral administration, the pharmaceutical composition according to the present invention is preferably liquid or ready to be dissolved in liquid such sterile, de-ionised or distilled water or sterile isotonic phosphate-buffered saline (PBS). Preferably, 1000 $\mu$g (dry-weight) of such a composition comprises or consists of 0.1-990 $\mu$g, preferably 1-900$\mu$g, more preferably 10- 200$\mu$g compound, and option-ally 1-500 $\mu$g, preferably 1-100 $\mu$g, more preferably 5-15 $\mu$g (buffer) salts (preferably to yield an isotonic buffer in the final volume), and optionally 0.1-999.9 $\mu$g, preferably 100-999.9 $\mu$g, more preferably 200-999 $\mu$g other excipients. Preferably, 100 mg of such a dry composition is dissolved in sterile, de-ionised/distilled water or sterile isotonic phosphate-buffered saline (PBS) to yield a final volume of 0.1-100 ml, preferably 0.5-20 ml, more preferably 1-10 ml.

It is evident to the skilled person that active agents and drugs described herein can also be administered in salt-form (i.e. as a pharmaceutically acceptable salt of the active agent). Accordingly, any mention of an active agent herein shall also include any pharmaceutically acceptable salt forms thereof.

Methods for chemical synthesis of peptides used for the compound of the present invention are well-known in the art. Of course, it is also possible to produce the peptides using recombinant methods. The peptides can be produced in microorganisms such as bacteria, yeast or fungi, in eukaryotic cells such as mammalian or insect cells, or in a recombinant virus vector such as adenovirus, poxvirus, herpesvirus, Simliki forest virus, baculovirus, bacteriophage, sindbis virus or sendai virus. Suitable bacteria for producing the peptides include E. coli, B. subtilis or any other bacterium that is capable of expressing such peptides. Suitable yeast cells for expressing the peptides of the present invention include Saccharomyces cerevisiae, Schizosaccharomyces pombe, Candida, Pichiapastoris or any other yeast capable of expressing peptides. Corresponding means and methods are well known in the art. Also, methods for isolating and purifying recombinantly produced peptides are well known in the art and include e.g. gel filtration, affinity chromatography, ion exchange chromatography etc.

Beneficially, cysteine residues are added to the peptides at the N- and/or C-terminus to facilitate coupling to the biopolymer scaffold, especially.

To facilitate isolation of said peptides, fusion polypeptides may be made wherein the peptides are translationally fused (covalently linked) to a heterologous polypeptide which enables isolation by affinity chromatography. Typical heterologous polypeptides are His-Tag (e.g. His6; 6 histidine residues), GST-Tag (Glutathione-S-transferase) etc. The fusion polypeptide facilitates not only the purification of the peptides but can also prevent the degradation of the peptides during the purification steps. If it is desired to remove the heterologous polypeptide after purification, the fusion polypeptide may comprise a cleavage site at the junction between the peptide and the heterologous polypeptide. The cleavage site may consist of an amino acid sequence that is cleaved with an enzyme specific for the amino acid sequence at the site (e.g. proteases).

The coupling/conjugation chemistry used to link the peptides / peptide n-mers to the biopolymer scaffold (e.g. via heterobifunctional compounds such as GMBS and of course also others as described in "Bioconjugate Techniques", Greg T. Hermanson) or used to conjugate the spacer to the peptides in the context of the present invention can also be selected from reactions known to the skilled in the art. The biopolymer scaffold itself may be recombinantly produced or obtained from natural sources.

Herein, the term "specific for" - as in "molecule A specific for molecule B" - means that molecule A has a binding preference for molecule B compared to other molecules in an individual's body. Typically, this entails that molecule A (such as an antibody) has a dissociation constant (also called "affinity") in regard to molecule B (such as the antigen, specifically the binding epitope thereof) that is lower than (i.e. "stronger than") 1000 nM, preferably lower than 100 nM, more preferably lower than 50 nM, even more preferably lower than 10 nM, especially lower than 5 nM.

Herein, "UniProt" refers to the Universal Protein Resource. UniProt is a comprehensive resource for protein sequence and annotation data. UniProt is a collaboration between the European Bioinformatics Institute (EMBL-EBI), the SIB Swiss Institute of Bioinformatics and the Protein Information Resource (PIR). Across the three institutes more than 100 people are involved through different tasks such as database curation, software development and support. Website: http://www.uniprot.org/

Entries in the UniProt databases are identified by their accession codes (referred to herein e.g. as "UniProt accession code" or briefly as "UniProt" followed by the accession code), usually a code of six alphanumeric letters (e.g. "Q1HVF7"). If not specified otherwise, the accession codes used herein refer to entries in the Protein Knowledgebase (UniProtKB) of UniProt. If not stated otherwise, the UniProt database state for all entries referenced herein is of 13 February 2019 (UniProt/UniProtKB Release 2019_02).

In the context of the present application, sequence variants (designated as "natural variant" in UniProt) are expressly included when referring to a UniProt database entry.

"Percent (%) amino acid sequence identity" or "X% identical" (such as "70% identical") with respect to a reference polypeptide or protein sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2, Megalign (DNASTAR) or the "needle" pairwise sequence alignment application of the EMBOSS software package. Those skilled in the art can determine appropriate parameters for aligning sequences, including any algorithms needed to achieve maximal alignment over the full length of the sequences being compared. For purposes herein, however, % amino acid sequence identity values are calculated using the sequence alignment of the computer programme "needle" of the EMBOSS software package (publicly available from European Molecular Biology Laboratory; Rice et al., EMBOSS: the European Molecular Biology Open Software Suite, Trends Genet. 2000 Jun;16(6):276-7, PMID: 10827456).

The needle programme can be accessed under the web site http://www.ebi.ac.uk/Tools/psa/emboss_needle/ or downloaded for local installation as part of the EMBOSS package from http://emboss.sourceforge.net/. It runs on many widely-used UNIX operating systems, such as Linux.

To align two protein sequences, the needle programme is preferably run with the following parameters:

Commandline: needle -auto -stdout -asequence SEQUENCE_FILE_A -bsequence SEQUENCE_FILE_B -datafile EBLOSUM62-gapopen 10.0 -gapextend 0.5 -endopen 10.0 -endextend 0.5-aformat3 pair -sprotein1 -sprotein2 (Align_format: pair Report_file: stdout)

The % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program needle in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. In cases where "the sequence of A is more than N% identical to the entire sequence of B", Y is the entire sequence length of B (i.e. the entire number of amino acid residues in B). Unless specifically stated otherwise, all % amino acid sequence identity values used herein are obtained as described in the immediately preceding paragraph using the needle computer program.

The present invention further relates to the following embodiments:

Embodiment 1. A compound comprising

- a biopolymer scaffold and at least
- a first peptide n-mer of the general formula:

$$P \ ( \ - \ S \ - \ P \ )_{(n-1)}$$

and

- a second peptide n-mer of the general formula:

$$P \ ( \ - \ S \ - \ P \ )_{(n-1)} \ ;$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer, wherein, independently for each of the peptide n-mers, n is an integer of at least 1, preferably of at least 2, more preferably of at least 3, especially of at least 4, wherein each of the peptide n-mers is bound to the biopolymer scaffold, preferably via a linker each.

Embodiment 2. The compound of embodiment 1, wherein at least one occurrence of P is a circularized peptide, preferably wherein at least 10% of all occurrences of P are circularized peptides, more preferably wherein at least 25% of all occurrences of P are circularized peptides, yet more preferably wherein at least 50% of all occurrences of P are circularized peptides, even more preferably wherein at least 75% of all occurrences of P are circularized peptides, yet even more preferably wherein at least 90% of all occurrences of P are circularized peptides or even wherein at least 95% of all occurrences of P are circularized peptides, especially wherein all of the occurrences of P are circularized peptides.

Embodiment 3. The compound of embodiment 1 or 2, wherein, independently for each of the peptide n-mers, n is at least 2, more preferably at least 3, especially at least 4.

Embodiment 4. The compound of any one of embodiments 1 to 3, wherein, independently for each of the peptide n-mers, n is less than 10, preferably less than 9, more preferably less than 8, even more preferably less than 7, yet even more preferably less than 6, especially less than 5.

Embodiment 5. The compound of any one of embodiments 1 to 4, wherein, for each of the peptide n-mers, n is 2.

Embodiment 6. The compound of any one of embodiments 1 to 5, wherein at least one occurrence of P is $P_a$ and/or at least one occurrence of P is $P_b$,

wherein $P_a$ is a peptide with a sequence length of 2-13 amino acids, preferably 3-11 amino acids, more preferably 4-9 amino acids, especially 5-8 amino acids,

wherein $P_b$ is a peptide with a sequence length of 2-13 amino acids, preferably 3-11 amino acids, more preferably 4-9 amino acids, especially 5-8 amino acids.

Embodiment 7. The compound of any one of embodiments 1 to 6, wherein, independently for each occurrence, P is $P_a$ or $P_b$.

Embodiment 8. The compound of any one of embodiments 1 to 7, wherein, in the first peptide n-mer, each occurrence of P is $P_a$ and, in the second peptide n-mer, each occurrence of P is $P_b$.

Embodiment 9. The compound of any one of embodiments 1 to 8, wherein

the first peptide n-mer is **$P_a$ - S - $P_a$** and the second peptide n-mer is **$P_a$ - S - $P_a$**; or

the first peptide n-mer is **$P_a$ - S - $P_a$** and the second peptide n-mer is **$P_b$ - S - $P_b$**;

the first peptide n-mer is $P_b$ - $S$ - $P_b$ and the second peptide n-mer is $P_b$ - $S$ - $P_b$;

the first peptide n-mer is $P_a$ - $S$ - $P_b$ and the second peptide n-mer is $P_a$ - $S$ - $P_b$;

the first peptide n-mer is $P_a$ - $S$ - $P_b$ and the second peptide n-mer is $P_a$ - $S$ - $P_a$; or

the first peptide n-mer is $P_a$ - $S$ - $P_b$ and the second peptide n-mer is $P_b$ - $S$ - $P_b$.

Embodiment 10. A compound comprising

- a biopolymer scaffold and at least

- a first peptide n-mer which is a peptide dimer of the formula $P_a$ - $S$ - $P_a$ or $P_a$ - $S$ - $P_b$,

wherein $P_a$ is a peptide with a sequence length of 2-13 amino acids, preferably 3-11 amino acids, more preferably 4-9 amino acids, especially 5-8 amino acids, $P_b$ is a peptide with a sequence length of 2-13 amino acids, preferably 3-11 amino acids, more preferably 4-9 amino acids, especially 5-8 amino acids, and S is a non-peptide spacer, wherein the first peptide n-mer is bound to the biopolymer scaffold, preferably via a linker.

Embodiment 11. The compound of embodiment 10, further comprising a second peptide n-mer which is a peptide dimer of the formula $P_b$ - $S$ - $P_b$ or $P_a$ - $S$ - $P_b$, wherein the second peptide n-mer is bound to the biopolymer scaffold, preferably via a linker.

Embodiment 12. The compound of any one of embodiments 1 to 9 and 11, wherein the first peptide n-mer is different from the second peptide n-mer.

Embodiment 13. The compound of any one of embodiments 6 to 12, wherein the peptide $P_a$ is different from the peptide $P_b$, preferably wherein the peptide $P_a$ and the peptide $P_b$ are two different epitopes of the same antigen or two different epitope parts of the same epitope.

Embodiment 14. The compound of any one of embodiments 6 to 13, wherein the peptide $P_a$ and the peptide $P_b$ comprise the same amino-acid sequence fragment, wherein the amino-acid sequence fragment has a length of at least 2 amino acids, preferably at least 3 amino acids, more preferably at least 4 amino acids, yet more preferably at least 5 amino acids, even more preferably at least 6 amino acids, yet even more preferably at least 7 amino acids, especially at least 8 amino acids or even at least 9 amino acids.

Embodiment 15. The compound of any one of embodiments 6 to 14, wherein $P_a$ and/or $P_b$ is circularized.

Embodiment 16. The compound of any one of embodiments 1 to 15, wherein the compound comprises a plurality of said first peptide n-mer and/or a plurality of said second peptide n-mer.

Embodiment 17. The compound of any one of embodiments 1 to 16, wherein the biopolymer scaffold is a protein, preferably a mammalian protein such as a human protein, a non-human primate protein, a sheep protein, a pig protein, a dog protein or a rodent protein.

Embodiment 18. The compound of embodiment 17, wherein the biopolymer scaffold is a globulin.

Embodiment 19. The compound of embodiment 18, wherein the biopolymer scaffold is selected from the group consisting of immunoglobulins, alpha1-globulins, alpha2-globulins and beta-globulins.

Embodiment 20. The compound of embodiment 19, wherein the biopolymer scaffold is selected from the group consisting of immunoglobulin G, haptoglobin and transferrin.

Embodiment 21. The compound of embodiment 20, wherein the biopolymer scaffold is haptoglobin.

Embodiment 22. The compound of embodiment 17, wherein the biopolymer scaffold is an albumin.

Embodiment 23. The compound of any one of embodiments 1 to 22, wherein the compound is non-immunogenic in a mammal, preferably in a human, in a non-human primate, in a sheep, in a pig, in a dog or in a rodent.

Embodiment 24. The compound of any one of embodiments 1 to 23, wherein the compound is for intracorporeal sequestration (or intracorporeal depletion) of at least one antibody in an individual, preferably in the bloodstream of the individual and/or for reduction of the titre of at least one antibody in the individual, preferably in the bloodstream of the individual.

Embodiment 25. The compound of any one embodiments 1 to 24, wherein the compound further comprises at least

a third peptide n-mer of the general formula:

$$P(-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_c$, wherein $P_c$ is a peptide with a sequence length of 2-13 amino acids, preferably 3-11 amino acids, more preferably 4-9 amino acids, especially 5-8 amino acids,

more preferably wherein $P_c$ is circularized;

a fourth peptide n-mer of the general formula:

$$P(-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_d$, wherein $P_d$ is a peptide with a sequence length of 2-13 amino acids, preferably 3-11 amino acids, more preferably 4-9 amino acids, especially 5-8 amino acids,

more preferably wherein $P_d$ is circularized;

a fifth peptide n-mer of the general formula:

$$P(-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_e$, wherein $P_e$ is a peptide with a sequence length of 2-13 amino acids, preferably 3-11 amino acids, more preferably 4-9 amino acids, especially 5-8 amino acids,

more preferably wherein $P_e$ is circularized;

a sixth peptide n-mer of the general formula:

$$P(-S-P)_{(n-1)},$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_f$, wherein $P_f$ is a peptide with a sequence length of 2-13 amino acids, preferably 3-11 amino acids, more preferably 4-9 amino acids, especially 5-8 amino acids,

more preferably wherein $P_f$ is circularized;

a seventh peptide n-mer of the general formula:

$$P (- S - P)_{(n-1)} ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,
preferably wherein each occurrence of P is $P_g$, wherein $P_g$ is a peptide with a sequence length of 2-13 amino acids, preferably 3-11 amino acids, more preferably 4-9 amino acids, especially 5-8 amino acids, more preferably wherein $P_g$ is circularized;

a eigth peptide n-mer of the general formula:

$$P (- S - P)_{(n-1)} ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,
preferably wherein each occurrence of P is $P_h$, wherein $P_h$ is a peptide with a sequence length of 2-13 amino acids, preferably 3-11 amino acids, more preferably 4-9 amino acids, especially 5-8 amino acids, more preferably wherein $P_h$ is circularized;

a ninth peptide n-mer of the general formula:

$$P (- S - P)_{(n-1)} ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,
preferably wherein each occurrence of P is $P_i$, wherein $P_i$ is a peptide with a sequence length of 2-13 amino acids, preferably 3-11 amino acids, more preferably 4-9 amino acids, especially 5-8 amino acids, more preferably wherein $P_i$ is circularized;

a tenth peptide n-mer of the general formula:

$$P (- S - P)_{(n-1)} ,$$

wherein, independently for each occurrence, P is a peptide with a sequence length of 2-13 amino acids, preferably 3-11 amino acids, more preferably 4-9 amino acids, especially 5-8 amino acids, and S is a non-peptide spacer,

preferably wherein each occurrence of P is $P_j$, wherein $P_j$ is a peptide with a sequence length of 2-13 amino acids, preferably 3-11 amino acids, more preferably 4-9 amino acids, especially 5-8 amino acids,

more preferably wherein $P_j$ is circularized.

Embodiment 26. The compound of any one of embodiments 1 to 25, wherein the entire sequence, optionally with the exception of an N-terminal and/or C-terminal cysteine, of at least one occurrence of P, preferably of at least 10% of all occurrences of P, more preferably of at least 25% of all occurrences of P, yet more preferably of at least 50% of all occurrences of P, even more preferably of at least 75% of all occurrences of P, yet even more preferably of at least 90% of all occurrences of P or even of at least 95% of all occurrences of P, especially of all of the occurrences of P, is identical to a sequence fragment of a protein, wherein the protein is identified by one of the following UniProt accession codes:

P01023, A8K2U0, P49588, Q5JTZ9, O95477, Q8IZY2, P08183, P33527, 015438, Q96IU4, P00519, P42684, Q9BYF1, P22303, Q99798, P68133, P60709, P63261, P12814, O43707, P61158, Q13705, P37023, O75077, Q9UKQ2, Q76LX8, Q6ZMM2, P35611, P07327, P00325, P35348, P25100, P08588, P07550, P25098, P35626, P30566, P43652, P02771, Q5U5Z8, Q15109, P35573, Q9UL18, Q9UKV8, O00468, P01019, P30556, Q09666, P02765, 043918, Q9Y6K8, Q02952, P14550, P15121, O95154, P02768, P00352, P49189, Q9UM73, P09923, P05187, P03971, P49418, P03950, Q9BY76, Q15327, P15144, P04083, P50995, P07355, Q3ZCQ2, P12429, P09525, P08758, P08133, O76027, Q13367, P27695, Q9BZZ5, P02647, P04114, P02749, P05067, P29972, P55087, Q8N726, P05089, Q9UNA1, P52566, Q99819, Q15052, P07306, P04424, P08243, Q9BXN1, P15336, P13637, P05026, P98194, P20648, P51164, P06576, P48047, P54252, Q8WXX7, P01185, P25311, Q9H6S1, P61769, Q13072, 075531, Q99728, P10415, P41182, P11274, 014503, Q93088, O00499, O15392, P35226, P12643, P18075, Q8N8U9, Q13873, P17213, Q9NP55, Q96DR5, Q8TDL5, P15056, Q7Z569, P38398, P51587, Q58F21, Q8IWQ3, Q8NE79, Q9Y224, Q13901, P02745, P01024, P00915, P00918, P07451, O00555, Q00975, Q9NY47, Q9Y698, Q8TC20, Q05682, P27482, P27797, P27824, P04632, P52907, P42574, Q14790, P31415, P41180, P20810, 015446, P04040, Q9NTU7, Q5M9N0, Q3V6T2, P10147, P13501, P20248, P14635, P24385, Q8ND76, P51681, P49368, P48643, P50990, Q9NZQ7, P28906, P16671, P04234, P15529, P08174, P13987, P01732, P21926, P30305, P12830, P55291, P22223, P55283, P06493, P42771, P51861, Q01850, Q9H211, P13688, P06731, Q9UNI1, P49450, P07199, Q03188, Q02224, P49454, Q9H3R5, Q92674, Q6IPU0, Q7L2Z9, A8MT69, Q5JTW2, P00751, P08603, Q03591, P36980, Q02985, Q9P2M7, O95992, Q14839, P10645, P36222, Q15782, Q9UKJ5, Q9Y259, P11229, P08172, P20309, P08173, P08912, P02708, Q9UGM1, P11230, Q8NCH0, Q99828, O75339, Q14011, Q07065, P12277, Q96MX0, P06732, A8K7I4, O95832, O75508, P30622, Q96KN2, Q12860, Q02246, Q8IWV2, O94779, Q9UQ52, P78357, Q9UHC6, Q7Z7A1, P38432, Q5TAT6, Q9UMD9, P02452, Q01955, P29400, Q14031, P12111, Q02388, Q9Y215, P49747, Q14019, P00450, P16870, Q8TCG5, P17927, Q9NS37, Q9UJA2, P02741, P02511, P53674, O95825, O75390, Q9Y600, P04141, P09919, PODML2, Q14406, Q6UVK1, Q01459, Q9GZU7, P16410, P35222, P53634, P07339, P08311, Q14247, O60494, Q14999, Q86UP6, P61073, P05108, P05093, P04798, P05177, P08686, P11509, P20813, P33261, P11712, P10635, P05181, P08684, Q8N907, P09172, P43146, P07585, P20711, Q16832, Q9NR30, 000571, Q86XP3, Q9NY93, O75398, P35659, P17661, Q96SL1, O94907, P10515, P09622, P36957, P24855, Q8NFT8, O00429, Q8N608, P27487, P42658, Q14195, Q9BPU6, P21728, P14416, Q08554, Q02487, Q14574, Q02413, Q14126, P32926, Q86SJ6, P15924, Q03001, Q9NRD8, Q05923, O75923, O95905, Q9NTX5, Q16610, O43854, P25101, Q15075, P68104, 000418, O95967, P01133, P00533, P20042, P38919, Q04637, P08246, Q12926, Q14576, P26378, P15502, P19622, P06733, P09104, P22413, O43768, P11171, P16422, P07099, P34913, P01588, P11678, P58107, P04626, Q96RT1, Q8IUD2, Q14264, P10768, P03372, Q9Y603, Q92817, Q9Y3B2, Q01780, Q13868, Q9NQT5, Q9NPD3, Q9NQT4, Q5RKV6, Q15024, Q96B26, Q06265, P15311, P00488, P08709, P00451, P00740, P15090, Q14320, P48023, P49327, Q8TES7, P22087, P35555, Q75N90, P09467, P12319, 075015, O75636, Q7L513, P02675, P11362, P62942, Q9UIM3, P20930, Q14315, O75955, Q14254, 043155, P35916, P02751, Q04609, P01225, Q12841, O95954, P02794, P02792, P09958, P35637, P51114, Q9UM11, P35575, 095166, P60520, Q9UBS5, O75899, Q99259, Q05329, Q13065, P22466, Q14376, P04406, P41250, P01350, P15976, P50440, P02774, P01275, Q8N6F7, P23434, P55107, P50395, P56159, Q9UJY5, P01241, P01286, Q9UBU3, P09681, O14908, P29033, Q9NS71, Q6ZMI3, P23415, P15104, Q6IB77, P49915, Q13823, P01148, P30968, Q92805, Q08379, Q08378, Q13439, A6NI86, A8MQT2, Q14789, P07359, P55259, P40197, Q9HCN6, P14770, Q9NQX3, P06744, Q13098, P24298, P18283, P42261, P42262, P42263, P48058, O43424, P39086, Q13002, Q16478, Q05586, Q12879, Q13224, Q4V328, Q13255, P41594, P28799, P07492, P08263, P21266, P78417, P09211, Q00403, P35269, P25092, P08236, P02724, P07305, P16104, O75367, P84243, P12081, Q96D42, P68871, Q13547, Q92769, 015379, P56524, Q9UQL6, P19113, Q9UBI9, P51858, Q00341, Q9NRV9, 000291, 075146, P54198, P16402, P58876, P62805, P19367, P09429, P26583, P04035, Q01581, P54868, P05114, P05204, Q14541, P09651, P22626, Q99729, Q14103, P52597, P31943, P31942, P61978, P14866, Q8WVV9, Q9NSC5, Q99714, Q7Z5P4, P14060, P08238, P14625, P0DMV8, P0DMV9, P34932, P11021, P11142, P04792, Q12988, P10809, Q92598, P08908, Q13639, Q9Y4L1, P10997, Q05084, Q9UMF0, O75874, Q5TF58, Q16666, Q9BYX4, P01563, P01574, P01579, Q9NWB7, P05019, P08069, P01344, Q9NZI8, Q9Y6M1, O00425, P11717, P18065, P17936, P01876, P01877, P01854, P01857, P01859, P01860, P01861, A6NGN9, Q8N6C5, P22301, Q13651, Q08334, Q14005, Q16552, Q96PD4, Q14116, P01583, P01584, P14778, P60568, Q9GZX6, P08700, P05112, P05231, P40189, Q96LU5, Q9NV31, P29218, O14732, P12268, Q9NQS7, P01308, Q96T92, P06213, P46940, Q14653, Q13568, P35568, P17301, P08514, P23229, P20701, P11215, P05107, P05106, P16144, Q14643, Q9Y6Y0, O60674, P17275, Q15046, P16389, P22459, Q9UK17, Q9NZI2, Q9NS61, P78508, P48050, P51787, O43525, Q8N5I3, Q6PI47, P35968, Q9Y4F3, Q96Q89, P43626, P43628, Q5JT82, Q53G59, Q8IXQ5, Q9UKR3, P03952, P26715, P26717, Q13241, P13645, P02533, P19012, P08779, Q04695, P05783, P08727, P12035, Q8N1N4, P05787, Q9NSB2, O15230, P11047, P13473, Q14739, P31025, P13796, P07195, P01130, Q9Y2U8, P09382, P05162, P17931, Q08380, Q3ZCW2, O95970, Q5TDP6, P22888, P49917, P07098, P02545, P20700, Q03252, P61968, P29536, P08519, Q07954, P98164, O75096, Q8TF66, Q32MZ4, Q8ND56, Q9Y4Z0, P02788, Q17RY6, P20645, Q8NHW3, P20916, P43358, O15479, O60732, Q9H0U3, P46821, P11137, Q16584, O43318, P45984, Q16644, P21941, O00339, P56270, P02144, Q9UIS9, P11226, P02686, Q01726, P32245, Q8IVS2, Q99705, Q969V1, Q8TDD5, Q8NE86, P40925, Q00987, O00255, P50579, P46013, Q16655, P03956, P45452, P08253, P09237, P14780, Q13201, Q13875, Q16653, Q13724, Q14149, Q9UBU8, O00566, Q99547, P40238, P05164, Q00013, Q9NZW5, P25189, P22897, Q9Y605, P82909, P43246, P52701, Q13421, P26038, Q9UJ68, P26927, Q13043, Q04912, Q9NZJ7, Q86UE4, P15941, Q8WXI7, O15146, Q9UIF7, P10242, P01106, Q99417, P12524, Q8N699, P12882, P35580, P35749, Q9UKX3, Q7Z406, Q9Y2K3, Q9UKX2, P11055, Q9Y623, P13533, P12883, A7E2Y1, P13535, P35579, B0I1T2, P54296, Q14CX7, E9PAV3, Q13765, Q8WY41, Q96I59, Q9UBB6, Q9UHB4, Q00604, P28331, P20929, P07196, P07197, Q8NG66, Q8TD19, O60524, O94856, P01138, Q8N4C6, P30414, P59047, Q8N427, Q13253, Q15155, P29475, P51513, Q9UNW9, P55786, O60500, P06748, P01160, P17342, P01303, Q9Y5X4, Q8IXM6, Q9ULB1, Q9HDB5, Q9Y4C0, Q9NXX6, P04629, Q16620, Q16288, Q02818, P80303, Q14980, P49790, Q8TEM1, O15504, Q14990, Q5BJF6, Q9ULJ1, Q6UX06, P78380, P41143, P35372, Q9P0S3, Q92791, Q9UQ80, Q13310, Q9UM07, Q7Z2X7, Q5JRK9, Q96GU1, Q13177, Q99497, P09874, P40424, Q15154, P12004, P29120, Q8WUM4, O95263, O76083, P16234, P09619, O00330, P30101, Q8N165, O00151, Q5T2W1, P16284, P02776, P10720, P35080, P18669, P00558, O95394, P35232, Q99623, Q9BVI0, Q92576, O43175, P11309, O75364, Q9Y446, P04054, Q13018, P16885, Q15149, Q9H7P9, P40967, P29590, Q01453, Q9NR77, P54277, P16233, P54317, Q8ND90, Q9UL42, P00491, Q9H9Y6, O14802, Q99575, P16435, Q15063, Q01851, Q12837, Q15181, P62937, O60437, P35813, P01298, Q9HAZ2, P32119, Q13162, P30041, P13727, Q92954, P17612, P17252, P01236, P04553, P04554, O60678, P04070, Q9UNN8, P54821, Q99811, P07477, P24158, Q9BXM0, O43653, O75475, P20618, P40306, P49721, P28074, P28062, P28065, P61289, Q6PGN9, P26599, Q8WV60, P01270, P06454, Q06124, Q9Y2R2, P08575, Q12913, Q16849, Q92932, Q86Y79, Q9UHX1, P20472, Q9BRP8, P51153, Q9UI14, Q15276, P63244, Q92878, Q06609, P04049, Q15311, Q9UKM9, Q14498, P38159, P10745, Q06330, P53805, O95199, Q9P258, P35243, P46063, P05451, Q8IX06, P57771, P08100, P12271, O60930, O00584, Q9ULK6, Q99942, Q9UBF6, P13489, O75116, Q01973, P15927, Q9Y2J0, Q9UNE2, Q02878, P05388, P05386, P05387, Q9BUL9, P78346, P78345, P62277, P60866, O75676, O43159, Q15404, O00442, Q92541, Q9NQC3, Q9Y265, Q9Y230, P48443, P21817, Q92736, P31151, P04271, P0DJI8, P0DJI9, P10523, P49591, O43290, Q99590, Q8WTV0, Q14108, P13521, P05408, Q14524, Q9BWW7, P34741, Q86SQ7, Q9UDX4, Q13228, P16109, P04279, Q9HC62, P49908, Q9HD40, P01009, P05543, P30740, P29508, P48594, P35237, P05121, P07093, P05155, Q9BYW2, Q7Z333, Q8N474, Q9BWM7, Q99961, O15266, O60902, Q9NYZ4, Q9Y336, Q9H0K1, Q14190, Q13239, Q14493, Q9H0C2, P12235, P05141, Q9H2B4, O43511, P11168, Q8IWU4, O00400, P08195, Q8IWA5, P48751, Q9Y6R1, Q9BRV3, Q92911, P37840, O76070, P08621, P09012, P14678, P09234, P62314, P62316, P62318, P62304, P62306, P62308, P63162, O14512, P00441, P04179, Q9BQB4, O00570, P56693, P35716, O15370, O60248, Q9UN79, O95416, Q9H6I2, P35713, P48431, Q9Y651, P41225, O94993, Q06945, P35711, P35712, Q9BT81, P57073, P48436, P08047, P23497, Q13342, Q9H930, Q15506, Q8N0X2, P00995, P16150, O43791, P10451, Q8TCT8, Q8TCT7, Q8TCT6, Q13813, Q13501, P10124, P61011, O76094, Q05066, P05455, O43805, P61278, Q13586, Q9P246, P31948, P49842, P16949, Q7Z7C7, Q13033, O75558, P61266, Q13190, Q8IWZ8, Q9Y2Z0, Q8IWU6, P63165, P61956, P17600, P08247, P21579, P37837, Q15633, Q13148, P26639, Q9NYW0, P20226, O60806, P24557, P17987, O60522, O14746, P02787, P05549, Q92734, P10646, P02786, P01266, P01137, P21980, Q08188, P49221, P07204, P40225, P10827, P10828, Q9UPZ6, P31483, P29401, Q9Y490, O60602, Q8TDI7, P17152, P42167, P42166, P01375, O00300, P43489, P19237, P48788, P19429, P13805, P45379, P45378, P09430, Q8NDV7, P11387, Q969P6, P11388, Q13472, O95985, P04637, Q9H3D4, O15350, P60174, P09493, P07202, P12270, P56180, O43280, Q92519, Q96RU7, P19474, O15164, Q9UPN9, Q6AZZ1, P10155, P48995, Q13507, Q7Z4N2, Q7Z2W7, Q9HBA0, Q9BZW7, P01222, P16473, Q9H2G4, Q14166, Q8WZ42, P02766, P07437, O00294, Q15672, Q9P2K2, Q86VQ3, Q6A555, P14679, Q9BZF9, Q13404, Q14139, O95155, P11441, Q9UMX0, P17480, P09936, P15374, Q9Y3C8, P19224, P16662, P07911, Q8TCY9, Q9Y6N9, Q13107, P63027, Q15836, P18206, P55072, P21796, P08670, P04275, O75083, Q14191, P98170, Q13426, P13010, P12956, P67809, Q9Y2T7, O43829, Q13105, Q15915, O95409, Q8N9L1, Q9UDV7, Q9Y3S2, Q9UL40, Q14966, Q9H0M5, Q9Y5V0, Q96C28, Q9H5H4, A9RAI0, B5SUY7, O41855, O56137, O56139, P03135, P04133, P04882, P08362, P10269, P12538, P69353, Q5Y9B2, Q5Y9B4, Q65311, Q6JC40, Q6VGT5, Q8JQF8, Q8JQG0, Q98654, Q9WBP8, Q9YIJ1, Q1HVF7, P03211, Q13585, P04233, O15523, O14602, Q30201, P01891, P01892, P04439, P05534, P10314, P10316, P13746, P16188, P16189, P16190, P18462, P30443, P30447, P30450, P30453, P30455, P30456, P30457, P30459, P30512, Q09160, P01889, P03989, P10319, P18463, P18464, P18465, P30460, P30461, P30462, P30464, P30466, P30475, P30479, P30480, P30481, P30483, P30484, P30485, P30486, P30487, P30488, P30490, P30491, P30492, P30493, P30495, P30498, P30685, Q04826, Q29718, Q29836, Q29940, Q31610, Q31612, Q95365, P04222, P10321, P30499, P30501, P30504, P30505, P30508, P30510, Q07000, Q29865, Q29960, Q29963, Q95604, Q9TNN7, P28067, P28068, P06340, P13765, P20036, P04440, P01909, P01906, P01920, P05538, P01903, P01911, P01912, P04229, P13760, P13761, P20039, Q29974, Q30134, Q30167, Q5Y7A7, Q95IE3, Q9GIY3, Q9TQE0, P79483, P13762, Q30154, P13747, P30511, P17693, Q9BY66, Q29983, Q29980, P22090, Q03519, 014607, P08048; optionally wherein the sequence fragment comprises at most four, preferably at most three, more preferably at most two, especially at most one amino acid substitutions.

Embodiment 27. The compound of any one of embodiments 1 to 26, wherein the entire sequence, optionally with the exception of an N-terminal and/or C-terminal cysteine, of peptide P$_a$ is identical to a sequence fragment of a protein, wherein the protein is identified by one of the UniProt accession codes listed in embodiment 26; optionally wherein said sequence fragment comprises at most four, preferably at most three, more preferably at most two, especially at most one amino acid substitutions.

Embodiment 28. The compound of any one of embodiments 1 to 27, wherein the entire sequence, optionally with the exception of an N-terminal and/or C-terminal cysteine, of peptide P$_b$ is identical to a sequence fragment of a protein, wherein the protein is identified by one of the UniProt accession codes listed in embodiment 26; optionally wherein said sequence fragment comprises at most four, preferably at most three, more preferably at most two, especially at most one amino acid substitutions.

Embodiment 29. The compound of any one of embodiments 1 to 28, wherein the entire sequence, optionally with the exception of an N-terminal and/or C-terminal cysteine, of peptide P$_a$ is identical to a sequence fragment of a protein and the entire sequence, optionally with the exception of an N-terminal and/or C-terminal cysteine, of peptide P$_b$ is identical to the same or another, preferably another, sequence fragment of the same protein, wherein the protein is identified by one of the UniProt accession codes listed in embodiment 26; optionally wherein said sequence fragment and/or said another sequence fragment comprises at most four, preferably at most three, more preferably at most two, especially at most one amino acid substitutions.

Embodiment 30. The compound of any one of embodiments 1 to 29, wherein the entire sequence, optionally with the exception of an N-terminal and/or C-terminal cysteine, of at least one occurrence of P, preferably of at least 10% of all occurrences of P, more preferably of at least 25% of all occurrences of P, yet more preferably of at least 50% of all occurrences of P, even more preferably of at least 75% of all occurrences of P, yet even more preferably of at least 90% of all occurrences of P or even of at least 95% of all occurrences of P, especially of all of the occurrences of P, is identical to a sequence fragment of an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-1a, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab; optionally wherein said sequence fragment comprises at most four, preferably at most three, more preferably at most two, especially at most one amino acid substitutions.

Embodiment 31. The compound of any one of embodiments 1 to 30, wherein the entire sequence, optionally with the exception of an N-terminal and/or C-terminal cysteine, of peptide $P_a$ is identical to a sequence fragment of an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-la, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab; optionally wherein said sequence fragment comprises at most four, preferably at most three, more preferably at most two, especially at most one amino acid substitutions.

Embodiment 32. The compound of any one of embodiments 1 to 31, wherein the entire sequence, optionally with the exception of an N-terminal and/or C-terminal cysteine, of peptide $P_b$ is identical to a sequence fragment of an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-la, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab;
optionally wherein said sequence fragment comprises at most four, preferably at most three, more preferably at most two, especially at most one amino acid substitutions.

Embodiment 33. The compound of any one of embodiments 1 to 32, wherein the entire sequence, optionally with the exception of an N-terminal and/or C-terminal cysteine, of peptide $P_a$ is identical to a sequence fragment of an amino-acid sequence and the entire sequence, optionally with the exception of an N-terminal and/or C-terminal cysteine, of peptide $P_b$ is identical to the same or another, preferably another, sequence fragment of the same amino-acid sequence, wherein the amino-acid sequence is an amino-acid sequence of Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-1a, Interferon alfa, interferon alfa-2b, interferon alfacon-1, interferon gamma-lb, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab; optionally wherein said sequence fragment and/or said another sequence fragment comprises at most four, preferably at most three, more preferably at most two, especially at most one amino acid substitutions.

Embodiment 34. The compound of any one of embodiments 1 to 33, wherein each of the peptide n-mers is covalently bound to the biopolymer scaffold, preferably via a linker each.

Embodiment 35. The compound of any one of embodiments 1 to 34, wherein at least one of said linkers is selected from disulphide bridges and PEG molecules.

Embodiment 36. The compound of any one of embodiments 1 to 35, wherein at least one of the spacers S is selected from PEG molecules or glycans.

Embodiment 37. The compound of any one of embodiments 1 to 36, wherein at least one occurrence of P is $P_a$ and at least one occurrence of P is $P_b$,

wherein $P_a$ is a peptide with a sequence length of 5-13, preferably 7-13, amino acids,

wherein $P_b$ is a peptide with a sequence length of 5-13, preferably 7-13, amino acids,

wherein the entire sequence, optionally with the exception of an N-terminal and/or C-terminal cysteine, of peptide $P_a$ is identical to a sequence fragment of a protein, wherein the protein is identified by UniProt accession code P02708, 015146 or O75096, optionally wherein said sequence fragment comprises at most four, preferably at most three, more preferably at most two, especially at most one amino acid substitutions,

wherein the entire sequence, optionally with the exception of an N-terminal and/or C-terminal cysteine, of peptide $P_b$ is identical to a sequence fragment of a protein, wherein the protein is identified by UniProt accession code P02708, 015146 or O75096, optionally wherein said sequence fragment comprises at most four, preferably at most three, more preferably at most two, especially at most one amino acid substitutions.

Embodiment 38. The compound of any one of embodiments 14 to 37, wherein, in particular for $P_a$ and/or $P_b$, said sequence fragment of the protein is a fragment of the sequence consisting of amino acids 21-255 of the AChR subunit alpha sequence identified by UniProt accession code P02708.

Embodiment 39. The compound of any one of embodiments 14 to 38, wherein, in particular for $P_a$ and/or $P_b$, said sequence fragment of the protein is a fragment of the sequence LKWNPDDYGGVKKIHIPSEK (SEQ ID NO: 1), preferably of the sequence WNPDDYGGVK (SEQ ID NO: 2) or VKKIHIPSEK (SEQ ID NO: 3).

Embodiment 40. The compound of any one of embodiments 6 to 39, wherein peptide $P_a$ and/or peptide $P_b$ consist of the sequence VKKIHIPSEKG (SEQ ID NO: 4) optionally with an N-terminal and/or C-terminal cysteine residue.

Embodiment 41. The compound of any one of embodiments 6 to 40, wherein the first peptide n-mer is **$P_a$ - S - $P_b$** and the second peptide n-mer is **$P_a$ - S - $P_b$**.

Embodiment 42. The compound of any one of embodiments 6 to 40, wherein the peptide $P_a$ and the peptide $P_b$ comprise the same amino-acid sequence fragment, wherein the amino-acid sequence fragment has a length of at least 5 amino acids, even more preferably at least 6 amino acids, yet even more preferably at least 7 amino acids, especially at least 8 amino acids or even at least 9 amino acids.

Embodiment 43. The compound of any one of embodiments 1 to 36, wherein at least one occurrence of P is $P_a$ and at least one occurrence of P is $P_b$,

wherein $P_a$ is a peptide with a sequence length of 5-13, preferably 7-13, amino acids,

wherein $P_b$ is a peptide with a sequence length of 5-13, preferably 7-13, amino acids,

wherein the entire sequence, optionally with the exception of an N-terminal and/or C-terminal cysteine, of peptide $P_a$ is identical to a sequence fragment of a protein, wherein the protein is identified by UniProt accession code Q1HVF7, P03211, Q13585 or P30556, optionally wherein said sequence fragment comprises at most four, preferably at most three, more preferably at most two, especially at most one amino acid substitutions,

wherein the entire sequence, optionally with the exception of an N-terminal and/or C-terminal cysteine, of peptide $P_b$ is identical to a sequence fragment of a protein, wherein the protein is identified by UniProt accession code Q1HVF7, P03211, Q13585 or P30556, optionally wherein said sequence fragment comprises at most four, preferably at most three, more preferably at most two, especially at most one amino acid substitutions.

Embodiment 44. The compound of any one of embodiments 14 to 36 and 43, wherein, in particular for $P_a$ and/or $P_b$, said sequence fragment of the protein is a fragment of the sequence RPQKRPSCIGCKGTH (SEQ ID NO: 5) or RPQKRPSCIGCKGAH (SEQ ID NO: 6), preferably of the sequence KRPSCIGCK (SEQ ID NO: 7).

Embodiment 45. The compound of any one of embodiments 14 to 36 and 43 to 44, wherein, in particular for $P_a$ and/or $P_b$, said sequence fragment of the protein is a fragment of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12).

Embodiment 46. The compound of any one of embodiments 6 to 36 and 43 to 45, wherein peptide $P_a$ and/or peptide $P_b$ consist of the sequence GRPQKRPSCIG (SEQ ID NO: 13) optionally with an N-terminal and/or C-terminal cysteine residue.

Embodiment 47. The compound of embodiments 6 to 36 and 43 to 46, wherein the first peptide n-mer is $P_a$ - S - $P_b$ and the second peptide n-mer is $P_a$ - S - $P_b$.

Embodiment 48. The compound of embodiments 6 to 36 and 43 to 47, wherein the peptide $P_a$ and the peptide $P_b$ comprise the same amino-acid sequence fragment, wherein the amino-acid sequence fragment has a length of at least 5 amino acids, even more preferably at least 6 amino acids, yet even more preferably at least 7 amino acids, especially at least 8 amino acids or even at least 9 amino acids.

Embodiment 49. A compound, preferably for the sequestration (or depletion) of anti human muscle nicotinic acetylcholine receptor (AChR) antibodies, anti human muscle-specific receptor tyrosine kinase antibodies and/or anti human low-density lipoprotein receptor related protein 4 antibodies present in a human individual, the compound comprising a biopolymer scaffold and at least two peptides with a sequence length of 7-13 amino acids, wherein each of the peptides independently comprises a 7-13 amino-acid sequence fragment of the AChR subunit alpha sequence identified by UniProt accession code P02708 or of the muscle-specific receptor tyrosine kinase sequence identified by UniProt accession code 015146 or of the low-density lipoprotein receptor related protein 4 sequence identified by UniProt accession code O75096, wherein the peptides are covalently bound to the biopolymer scaffold, wherein the biopolymer scaffold is selected from the group consisting of human globulins and human albumin.

Embodiment 50. The compound of embodiment 49, wherein the at least two peptides comprise a peptide $P_1$ and a peptide $P_2$, wherein $P_1$ and $P_2$ comprise the same 7-13 amino-acid sequence fragment of AChR subunit alpha, wherein $P_1$ and $P_2$ are present in form of a peptide dimer
$P_1$ - S - $P_2$, wherein S is a non-peptide spacer, wherein the peptide dimer is covalently bound to the biopolymer scaffold, preferably via a linker.

Embodiment 51. The compound of 49 or 50, wherein said 7-13 amino-acid sequence fragment of AChR subunit alpha is a fragment of the sequence consisting of amino acids 21-255 of the AChR subunit alpha sequence identified by UniProt accession code P02708.

Embodiment 52. The compound of any one of embodiments 49 to 51, wherein said 7-13 amino-acid sequence fragment of AChR subunit alpha is a fragment of the sequence LKWNPDDYGGVKKIHIPSEK (SEQ ID NO: 1), preferably of the sequence WNPDDYGGVK (SEQ ID NO: 2) or VKKIHIPSEK (SEQ ID NO: 3).

Embodiment 53. The compound of any one of embodiments 49 to 52, wherein the peptides have a sequence length of 8-13 amino acids, preferably 9-12 amino acids, more preferably 10-12 amino acids, especially wherein the peptides consist of the sequence VKKIHIPSEKG (SEQ ID NO: 4) optionally with an N-terminal and/or C-terminal cysteine residue.

Embodiment 54. The compound of any one of embodiments 1 to 53, wherein the compound further comprises at least one peptide with a sequence length of 7-13 amino acids, wherein the at least one peptide comprises a 7-13 amino-acid sequence fragment of the muscle-specific receptor tyrosine kinase sequence identified by UniProt accession code 015146 or of the low-density lipoprotein receptor related protein 4 sequence identified by UniProt accession code O75096, wherein the at least one peptide is covalently bound to the biopolymer scaffold, preferably via a linker.

Embodiment 55. A compound, preferably for the sequestration (or depletion) of anti-Epstein-Barr virus nuclear antigen 1 (EBNA-1) antibodies, anti human melatonin-related receptor (GPR50) antibodies and/or anti human type-1 angiotensin II receptor (AT1AR) antibodies present in a human individual, the compound comprising a biopolymer scaffold and at least two peptides with a sequence length of 7-13 amino acids,

> wherein each of the peptides independently comprises a 7-13 amino-acid sequence fragment of the EBNA1 sequence identified by UniProt accession code Q1HVF7 or P03211 or of the GPR50 sequence identified by UniProt accession code Q13585 or of the type-1 angiotensin II receptor (AT1AR) sequence identified by UniProt accession code P30556, wherein the peptides are covalently bound to the biopolymer scaffold,

> wherein the biopolymer scaffold is selected from the group consisting of human globulins, preferably from the group consisting of human immunoglobulins and human haptoglobin, and human albumin.

Embodiment 56. The compound of embodiment 55, wherein the at least two peptides comprise a peptide $P_1$ and a peptide $P_2$, wherein $P_1$ and $P_2$ comprise the same 7-13 amino-acid sequence fragment of said EBNA1 sequence or said GPR50 sequence, wherein $P_1$ and $P_2$ are present in form of a peptide dimer $P_1$ - $S$ - $P_2$, wherein S is a non-peptide spacer, wherein the peptide dimer is covalently bound to the biopolymer scaffold, preferably via a linker.

Embodiment 57. The compound of embodiment 55 or 56, wherein said 7-13 amino-acid sequence fragment is a fragment of the sequence RPQKRPSCIGCKGTH (SEQ ID NO: 5) or RPQKRPSCIGCKGAH (SEQ ID NO: 6), preferably of the sequence KRPSCIGCK (SEQ ID NO: 7); and/or wherein the peptides have a sequence length of 8-13 amino acids, preferably 9-12 amino acids, more preferably 10-12 amino acids, especially wherein at least one of the at least two, preferably each of the peptides consist of the sequence GRPQKRPSCIG (SEQ ID NO: 13) optionally with an N-terminal and/or C-terminal cysteine residue.

Embodiment 58. The compound of any one of embodiments 55 to 57, wherein said 7-13 amino-acid sequence fragment is a fragment of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12).

Embodiment 59. The compound of any one of embodiments 1 to 58, wherein the compound further comprises at least one peptide with a sequence length of 7-13 amino acids, wherein the at least one peptide comprises a 7-13 amino-acid sequence fragment of the type-1 angiotensin II receptor (AT1AR) sequence identified by UniProt accession code P30556, preferably of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12); wherein the at least one peptide is covalently bound to the biopolymer scaffold, preferably via a linker.

Embodiment 60. The compound of any one of embodiments 1 to 59, wherein each of the peptides is covalently bound to the scaffold via a linker.

Embodiment 61. The compound of any one embodiments 1 to 60, wherein the biopolymer scaffold is selected from human immunoglobulins and human haptoglobins.

Embodiment 62. The compound of embodiment any one of embodiments 1 to 61, wherein the biopolymer scaffold is human haptoglobin. Embodiment 63. The compound of any one of embodiments 49 to 62, wherein at least one of the at least two peptides is circularized.

Embodiment 64. The compound of any one of embodiments 1 to 63, wherein the compound is non-immunogenic in humans.

Embodiment 65. A pharmaceutical composition comprising the compound of any one of embodiments 1 to 64 and at least one pharmaceutically acceptable excipient.

Embodiment 66. The pharmaceutical composition of embodiment 65, wherein the composition is prepared for intraperitoneal, subcutaneous, intramuscular and/or intravenous administration.

Embodiment 67. The pharmaceutical composition of any one of embodiments 1 to 66, wherein the molar ratio of peptide P to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.

Embodiment 68. The pharmaceutical composition of any one of embodiments 6 to 67, wherein the molar ratio of peptide $P_a$ to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.

Embodiment 69. The pharmaceutical composition of any one of embodiments 6 to 68, wherein the molar ratio of peptide $P_b$ to biopolymer scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.

Embodiment 70. The pharmaceutical composition of any one of embodiments 65 to 69 for use in therapy.

Embodiment 71. The pharmaceutical composition for use according to embodiment 70, for use in prevention or treatment of an autoimmune disease in an individual having the autoimmune disease or being at risk of developing the autoimmune disease.

Embodiment 72. The pharmaceutical composition for use according to embodiment 71, wherein the autoimmune disease is selected from the group consisting of neuromyelitis optica, seropositive neuromyelitis optica spectrum disorders, autoimmune-encephalitis, multiple sclerosis, amyotrophic lateral sclerosis, systemic lupus erythematosus dementia, myasthenia gravis, in particular transient neonatal myasthenia gravis, dilatative Cardiomyopathy, pulmonary hypertension, Sjögren's Syndrome, celiac Disease, Graves Disease, Goodpasture Disease, preeclampsia, Behcet's Disease, systemic sclerosis, hypertension, type I diabetes, type II diabetes, systemic lupus erythematosus, anti N-methyl-D-aspartate receptor (NMDAR) encephalitis, antiphospholipid syndrome, membranous nephropathy, primary biliary cholangitis, amyotrophic lateral sclerosis, Chagas disease cardiomyopathy, immune thrombocytopenic purpura, pemphigus vulgaris, bullous pemphigoid, epidermolysis bullosa acquisita and bullous systemic lupus erythematosus.

Embodiment 73. The pharmaceutical composition for use according to embodiment 70, for use in prevention or treatment of transplant rejection in an individual having a transplant or eligible for a transplantation.

Embodiment 74. The pharmaceutical composition for use according to embodiment 70, for use in prevention or treatment of adverse reactions based on anti-drug antibodies or anti-gene-delivery vector antibodies in an individual undergoing therapy with the drug or eligible for therapy with the drug, or in an individual undergoing gene therapy or eligible for gene therapy, preferably wherein the drug is a peptide or protein, especially selected from the group of enzymes, enzyme inhibitors, antibodies, antibody fragments, antibody mimetics, antibody-drug conjugates, hormones, growth factors, clotting factors and cytokines, preferably wherein the entire sequence, optionally with the exception of an N-terminal and/or C-terminal cysteine, of at least one occurrence of peptide P, or of peptide $P_a$ and/or of peptide $P_b$ is identical to a sequence fragment of an amino-acid sequence of the peptide or protein, optionally wherein said sequence fragment comprises at most four, preferably at most three, more preferably at most two, especially at most one amino acid substitutions.

Embodiment 75. The pharmaceutical composition for use according to embodiment 74, wherein the drug is Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-la, Interferon alfa, interferon alfa-2b, interferon alfacon-1, interferon gamma-1b, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab, preferably wherein the entire sequence, optionally with the exception of an N-terminal and/or C-terminal cysteine, of at least one occurrence of peptide P, or of peptide $P_a$ and/or of peptide $P_b$ is identical to a sequence fragment of an amino-acid sequence of the drug, optionally wherein said sequence fragment comprises at most four, preferably at most three, more preferably at most two, especially at most one amino acid substitutions.

Embodiment 76. The pharmaceutical composition for use according to any one of embodiments 70 to 75, wherein one or more antibodies are present in the individual which are specific for at least one occurrence of peptide P, or for peptide $P_a$ and/or peptide $P_b$, preferably wherein said antibodies are related to said disease.

Embodiment 77. The pharmaceutical composition for use according to any one of embodiments 70 to 76, wherein the composition is non-immunogenic in the individual.

Embodiment 78. The pharmaceutical composition for use according to any one of embodiments 70 to 77, wherein the composition is administered at a dose of 1-1000 mg, preferably 2-500 mg, more preferably 3-250 mg, even more preferably 4-100 mg, especially 5-50 mg, compound per kg body weight of the individual.

Embodiment 79. The pharmaceutical composition for use according to any one of embodiments 70 to 78, wherein the composition is administered intraperitoneally, subcutaneously, intramuscularly or intravenously.

Embodiment 80. A method of sequestering (or depleting) one or more antibodies present in an individual, comprising

obtaining a pharmaceutical composition as defined in any one of embodiments 65 to 69, wherein the composition is non-immunogenic in the individual and wherein the one or more antibodies present in the individual are specific for at least one occurrence of P, or for peptide $P_a$ and/or peptide $P_b$; and

administering the pharmaceutical composition to the individual.

Embodiment 81. The method of embodiment 80, wherein the individual is a non-human animal, preferably a non-human primate, a sheep, a pig, a dog or a rodent, in particular a mouse.

Embodiment 82. The method of embodiments 80 or 81, wherein the biopolymer scaffold is autologous with respect to the individual, preferably wherein the biopolymer scaffold is an autologous protein.

Embodiment 83. The method of any one of embodiments 80 to 82, wherein the individual is administered a heterologous protein, preferably a heterologous antibody such as a nanobody, and wherein the one or more antibodies present in the individual are specific for said heterologous protein, preferably wherein said administering of the heterologous protein is prior to, concurrent with and/or subsequent to said administering of the pharmaceutical composition.

Embodiment 84. The method of any one of embodiments 80 to 83, wherein the individual is a non-human animal and the heterologous protein is human or humanized.

Embodiment 85. The method of any one of embodiments 80 to 82, wherein the individual is administered a drug and wherein the one or more antibodies present in the individual are specific for said drug, preferably wherein said administering of the drug is prior to, concurrent with and/or subsequent to said administering of the pharmaceutical composition.

Embodiment 86. The method of embodiment 85, wherein the drug is Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-la, Interferon alfa, interferon alfa-2b, interferon alfacon-1, interferon gamma-1b, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab.

Embodiment 87. The method of any one of embodiments 80 to 86, wherein the individual is healthy.

Embodiment 88. The method of any one of embodiments 80 to 87, wherein the composition is administered intraperitoneally, subcutaneously, intramuscularly or intravenously.

Embodiment 89. A pharmaceutical composition, comprising the compound of any one of embodiments 1 to 64 and further comprising an active agent such as a protein or a peptide and optionally at least one pharmaceutically acceptable excipient, wherein the active agent comprises a peptide fragment with a sequence length of 2-13 amino acids, preferably 3-11 amino acids, more preferably 4-9 amino acids, especially 5-8 amino acids, and
wherein the sequence of at least one occurrence of peptide P, or peptide $P_a$ and/or peptide $P_b$, of the compound is at least 70% identical, preferably at least 75% identical, more preferably at least 80% identical, yet more preferably at least 85% identical, even more preferably at least 90% identical, yet even more preferably at least 95% identical, especially completely identical to the sequence of said peptide fragment.

Embodiment 90. The pharmaceutical composition of embodiment 89, wherein the active agent is an enzyme, preferably a human enzyme, an antibody, preferably a human or humanized antibody, a hormone, a growth factor, a clotting factor, a cytokine or a gene delivery vector.

Embodiment 91. The pharmaceutical composition of embodiment 89 or 90, wherein the active agent is Alpha-1-proteinase inhibitor, Alglucerase, Taliglucerase alfa, Pegademase, Agalsidase beta, Alglucosidase alfa, Laronidase, Idursulfase, Elosulfase alfa, Galsulfase, Sebelipase alfa, Cerliponase alfa, Sebelipase alfa, Asfotase Alfa, Elapegademase, Olipudase alpha, Velmanase alpha, N(4)-(beta-N-acetylglucosaminyl)-L-asparaginase, Rasburicase, Pegloticase, Human Antithrombin III, Plasma protease C1 inhibitor, Turoctocog alfa, Drotrecogin alfa, Emicizumab, Coagulation factor VIIa Recombinant Human, Antihemophilic factor human recombinant, Von Willebrand Factor Human, Susoctocog alfa, Antihemophilic factor human recombinant, Antihemophilic factor, human recombinant, Oprelvekin, Aldesleukin, Rilonacept, Anakinra, Denileukin diftitox, Erythropoietin, Interferon beta-1a, Interferon alfa, interferon alfa-2b, interferon alfacon-1, interferon gamma-1b, interferon alfa-2b recombinant, growth hormone (UniProt P01241), insulin (UniProt P01308), IGF1 (UniProt P05019), PTH (UniProt P01270), Thyrotropin alfa, Choriogonadotropin alfa, Follitropin, Lutropin alfa, Somatotropin, Albiglutide, Metreleptin, Corifollitropin alfa, Filgrastim, FGF2 (UniProt P09038), NGF (UniProt P01138), GDNF (UniProt P39905), BDNF (UniProt P23560), Mecasermin, Palifermin, GCSF (UniProt P09919), IGF2 (UniProt P01344), Becaplermin, Palifermin, Tasonermin, Aflibercept, Rilonacept, Romiplostim, Tagraxofusp, Efmoroctocog alfa, Eftrenonacog alfa, Rilonacept, Belatacept, Atacicept, Albutrepenonacog alfa , Dulaglutide , Etanercept, Asfotase Alfa, Natalizumab, Rituximab, Adalimumab, Ipilimumab, Trastuzumab, Bevacizumab, Evolocumab, Ixekizumab, Omalizumab, Teprotumumab, Idarucizumab, Cetuximab, Oportuzumab monatox, Ibritumomab tiuxetan, Abciximab, Rituximab, Ofatumumab, Erenumab, Emicizumab or Atezolizumab.

Embodiment 92. The pharmaceutical composition of any one of embodiments 89 to 91, wherein the composition is prepared for intravenous administration.

Embodiment 93. The pharmaceutical composition of any one of embodiments 89 to 92, wherein the composition is an aqueous solution.

Embodiment 94. The pharmaceutical composition of any one of embodiments 89 to 93 for use in inhibition of an immune reaction, preferably an antibody-mediated immune reaction, against the active agent.

Embodiment 95. The pharmaceutical composition for use according to embodiment 94, wherein the composition is non-immunogenic in the individual.

Embodiment 96. A method of inhibiting an immune reaction to a treatment with an active agent in an individual in need of treatment with the active agent, comprising

obtaining a pharmaceutical composition as defined in any one of embodiments 89 to 95; wherein the compound of the pharmaceutical composition is non-immunogenic in the individual, and

administering the pharmaceutical composition to the individual.

Embodiment 97. The method of embodiment 96, wherein the individual is human.

Embodiment 98. The method of embodiment 96 or 97, wherein the biopolymer scaffold is autologous with respect to the individual, preferably wherein the biopolymer scaffold is an autologous protein.

Embodiment 99. The method of any one of embodiments 96 to 98, wherein the composition is administered intraperitoneally, subcutaneously, intramuscularly or intravenously.

The present invention is further illustrated by the following figures and examples, without being restricted thereto.
In the context of the following figures and examples the compound of the present invention is also referred to as "Selective Antibody Depletion Compound" (SADC).

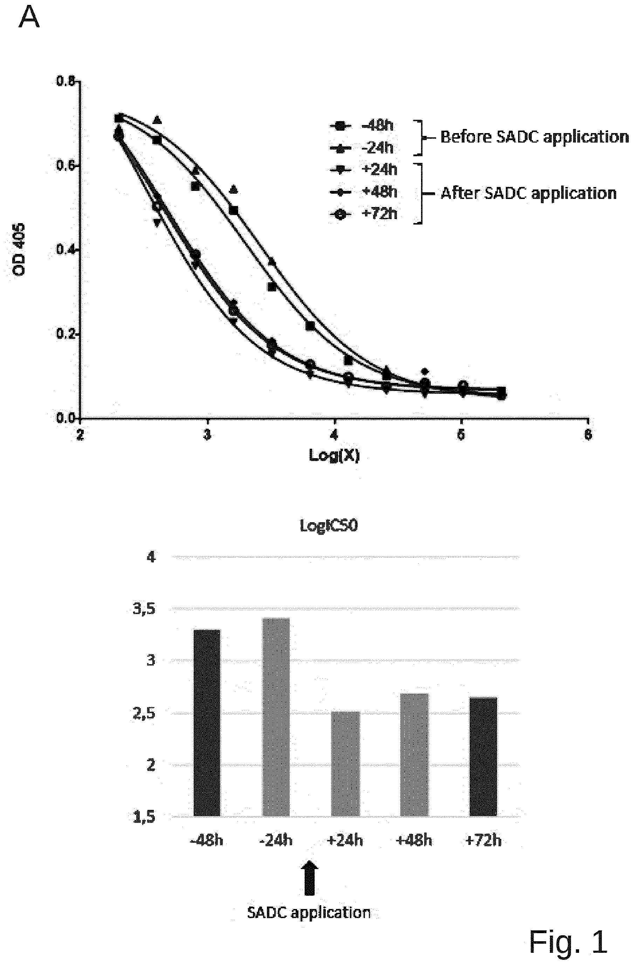

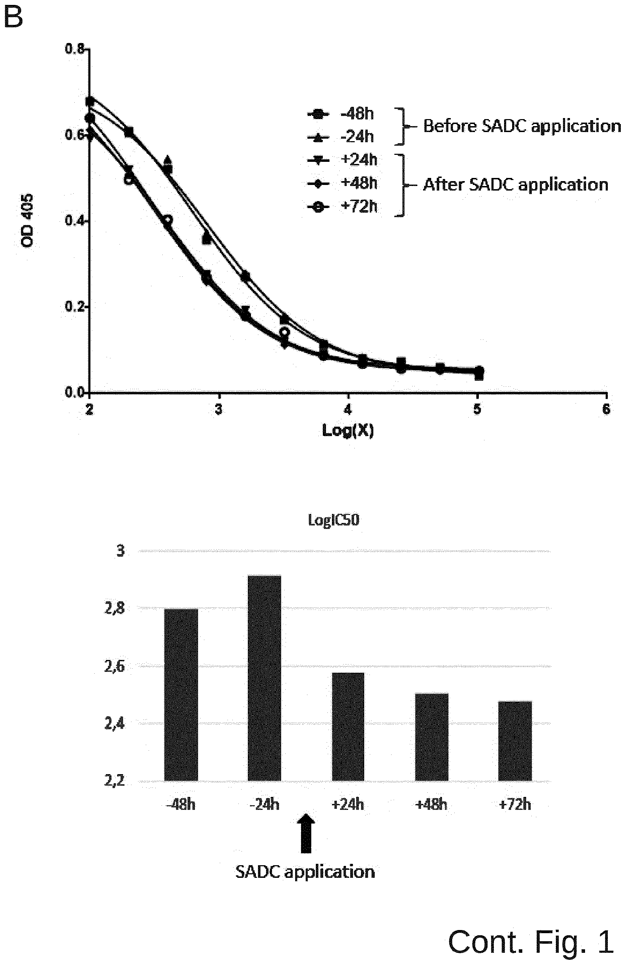

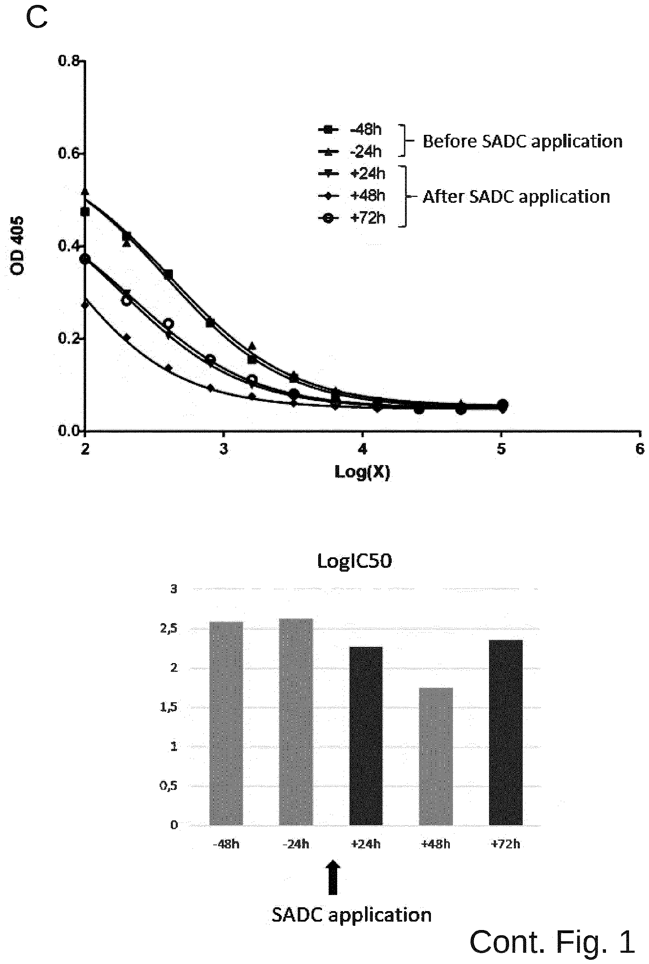

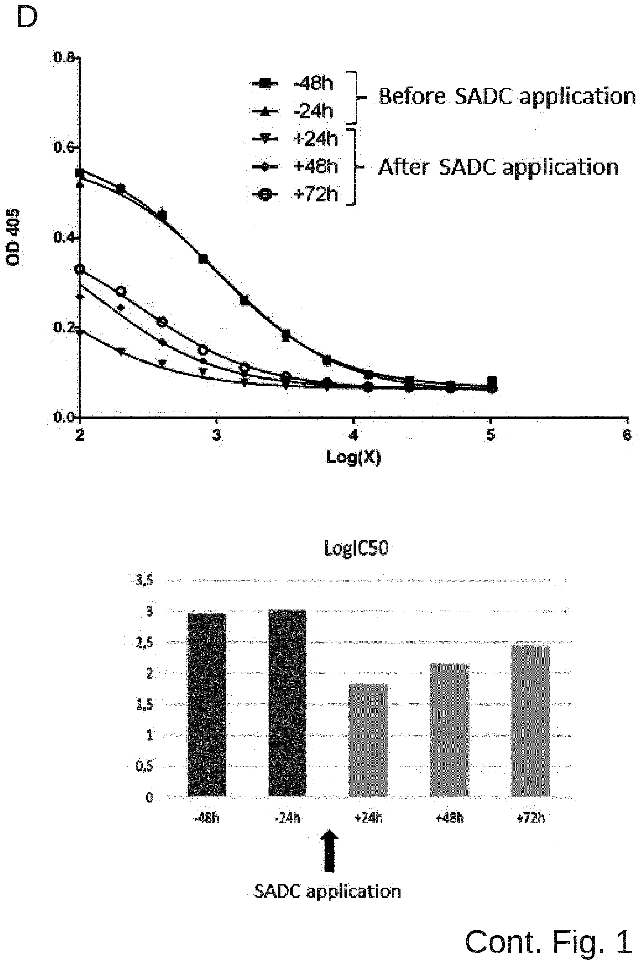

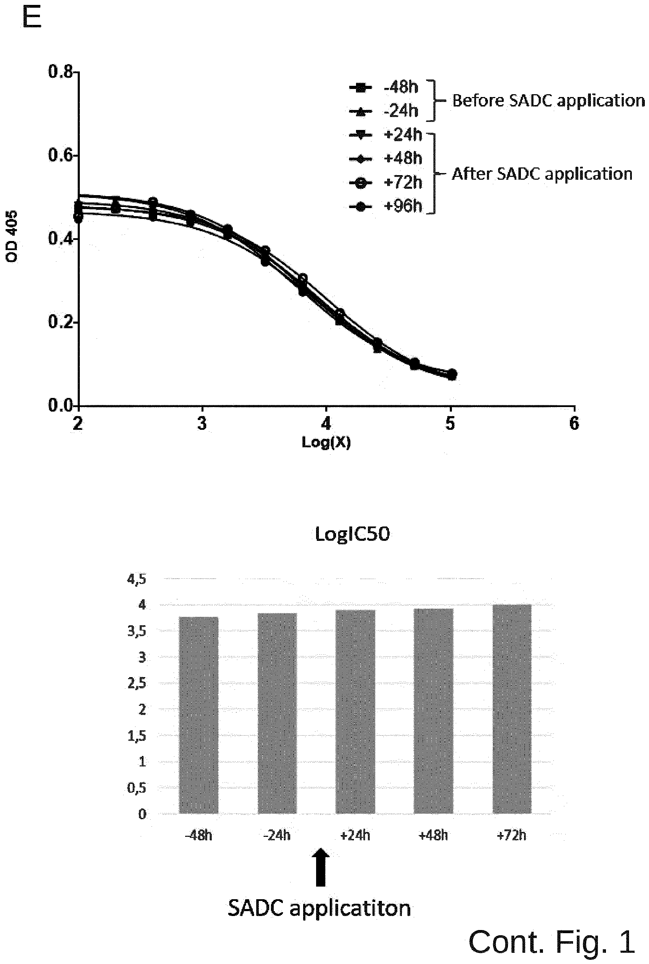

**Fig. 1: The compound of the present invention successfully reduces the titre of undesired antibodies.** Each compound of the invention was applied at time point 0 by i.p. injection into Balb/c mice pre-immunized by peptide immunization against a defined antigen. Each top panel shows anti-peptide titers (0.5x dilution steps; X-axis shows log(X) dilutions) against OD values (y-axis) according to a standard ELISA detecting the corresponding antibody. Each bottom panel shows titers LogIC50 (y-axis) before injection of each compound of the invention (i.e. titers at -48h and -24h) and after application of each compound of the invention (i.e. titers +24h, +48h and +72h after injection; indicated on the x-axis). (**A**) Compound with albumin as the biopolymer scaffold that binds to antibodies directed against EBNA1 (associated with pre-eclampsia). The mice were pre-immunized with a peptide vaccine carrying the EBNA-1 model epitope. (**B**) Compound with albumin as the biopolymer scaffold that binds to antibodies directed against a peptide derived from the human AChR protein MIR (associated with myasthenia gravis). The mice were pre-immunized with a peptide vaccine carrying the AChR MIR model epitope. (**C**) Compound with immunoglobulin as the biopolymer scaffold that binds to antibodies directed against EBNA1 (associated with pre-eclampsia). The mice were pre-immunized with a peptide vaccine carrying the EBNA-1 model epitope. (**D**) Compound with haptoglobin as the biopolymer scaffold that binds to antibodies directed against EBNA1 (associated with pre-eclampsia). The mice were pre-immunized with a peptide vaccine carrying the EBNA-1 model epitope. (**E**) Demonstration of selectivity using the same immunoglobulin-based compound of the invention binding to antibodies directed against EBNA1 that was used in the experiment shown in panel C. The mice were pre-immunized with an unrelated amino acid sequence. No titre reduction occurred, demonstrating selectivity of the compound.

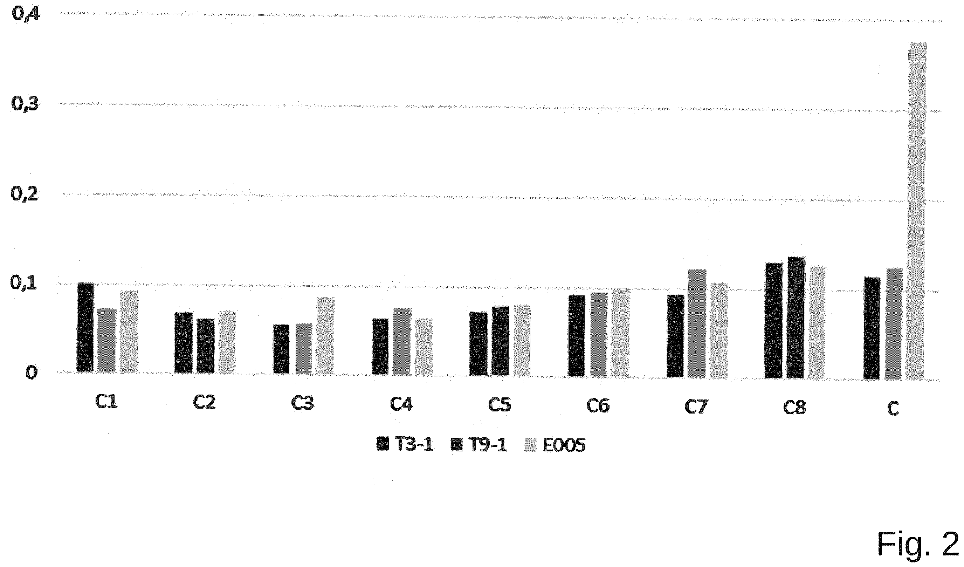

**Fig. 2: The compound of the invention is non-immunogenic and does not induce antibody formation after repeated injection into mice.** Animals C1-C4 as well as animals C5-C8 were treated i.p. with two different compounds of the invention. Control animal C was vaccinated with a KLH-peptide derived from the human AChR protein MIR. Using BSA-conjugated peptide probes T3-1, T9-1 and E005 (grey bars, as indicated in the graph), respectively, for antibody titer detection by standard ELISA at a dilution of 1:100, it could be demonstrated that antibody induction was absent in animals treated with a compound of the invention, when compared to the vaccine-treated control animal C (y-axis, OD450 nm).

## EXAMPLES

**Example 1: The compound of the present invention effectively reduces the titre of undesired antibodies.**

*Animal models:* In order to provide *in vivo* models with measurable titers of prototypic undesired antibodies in human indications, BALB/c mice were immunized using standard experimental vaccination with KLH-conjugated peptide vaccines derived from established human autoantigens or anti-drug antibodies. After titer evaluation by standard peptide ELISA, immunized animals were treated with the corresponding test SADCs to demonstrate selective antibody lowering by SADC treatment. All experiments were performed in compliance with the guidelines by the corresponding animal ethics authorities.

*Immunization of mice with model antigens:* Female BALB/c mice (aged 8-10 weeks) were supplied by Janvier (France), maintained under a 12h light/12h dark cycle and given free access to food and water. Immunizations were performed by s.c. application of KLH carrier-conjugated peptide vaccines injected 3 times in biweekly intervals. KLH conjugates were generated with peptide T3-2 (SEQ ID NO. 14: CGRPQKRPSCIGCKG), which represents an example for molecular mimicry between a viral antigen (EBNA-1) and an endogenous human receptor antigen, namely the placental GPR50 protein, that was shown to be relevant to preeclampsia (Elliott et al.). In order to confirm the generality of this approach, a larger antigenic peptide derived from the autoimmune condition myasthenia gravis was used for immunization of mice with a human autoepitope. In analogy to peptide T3-2, animals were immunized with peptide T1-1 (SEQ ID NO. 15: LKWNPDDYGGVKKIHIPSEKGC), derived from the MIR (main immunogenic region) of the human AChR protein which plays a fundamental role in pathogenesis of the disease (Luo et al.). The T1-1 peptide was used for immunizing mice with a surrogate partial model epitope of the human AChR autoantigen. The peptide T8-1 (SEQ ID NO. 16: DHTLYTPYHTHPG) was used to immunize control mice to provide a control titer for proof of selectivity of the system. For vaccine conjugate preparation, KLH carrier (Sigma) was activated with sulfo-GMBS (Cat. Nr. 22324 Thermo), according to the manufacturer's instructions, followed by addition of either N- or C-terminally cysteinylated peptides T3-2 and T1-1 and final addition of Alhydrogel® before injection into the flank of the animals. The doses for vaccines T3-2 and T1-1 were 15$\mu$g of conjugate in a volume of 100ul per injection containing Alhydrogel® (InvivoGen VAC-Alu-250) at a final concentration of 1% per dose.

*Generation of prototypic SADCs:* For testing selective antibody lowering activity by SADCs of T3-2 and T1-1 immunized mice, SADCs were prepared with mouse serum albumin (MSA) or mouse immunoglobulin (mouse-Ig) as biopolymer scaffold in order to provide an autologous biopolymer scaffold, that will not induce any immune reaction in mice, or non-autologuous human haptoglobin as biopolymer scaffold (that did not induce an allogenic reaction after one-time injection within 72 hours). N-terminally cysteinylated SADC peptide E049 (SEQ ID NO. 13: GRPQKRPSCIG) and/or C-terminally cysteinylated SADC peptide E006 (SEQ ID NO. 4: VKKIHIPSEKG) were linked to the scaffold using sulfo-GMBS (Cat. Nr. 22324 Thermo)-activated MSA (Sigma; Cat. Nr. A3559) or -mouse-Ig (Sigma, 15381) or -human haptoglobin (Sigma H0138) according to the instructions of the manufacturer, thereby providing MSA-, Ig- and haptoglobin-based SADCs with the corresponding cysteinylated peptides, that were covalently attached to the lysines of the corresponding biopolymer scaffold. Beside conjugation of the cysteinylated peptides to the lysines via a bifunctional amine-to-sulfhydryl crosslinker, a portion of the added cysteinylated SADC peptides directly reacted with sulfhydryl groups of cysteins of the albumin scaffold protein, which can be detected by treating the conjugates with DTT followed by subsequent detection of free peptides using mass spectrometry or any other analytical method that detects free peptide. Finally, these SADC conjugates were dialysed against water using Pur-A-Lyzer™ (Sigma) and subsequently lyophilized. The lyophilized material was resuspended in PBS before injection into animals.

*In vivo functional testing of* SADCs: Prototypic SADCs, SADC-E049 and SADC-E006 were injected intraperitoneally (i.p.; as a surrogate for an intended intravenous application in humans and larger animals) into the mice that had previously been immunized with peptide vaccine T3-2 (carrying the EBNA-1 model epitope) and peptide vaccine T1-1 (carrying the AChR MIR model epitope). The applied dose was 30$\mu$g SADC conjugate in a volume of 50$\mu$l PBS. Blood takes were performed by submandibular vein puncture, before (-48h, -24h) and after (+24h,+48h,+72h, etc.) i.p. SADC injections, respectively, using capillary micro-hematocrit tubes. Using ELISA analysis (see below), it was found that both prototypic SADCs were able to clearly reduce the titers over a period of at least 72 hrs in the present animal model. It could therefore be concluded that SADCs can be used to effectively reduce titers *in vivo.*

*Titer analysis:* Peptide ELISAs were performed according to standard procedures using 96-well plates (Nunc Medisorp plates; Thermofisher, Cat Nr 467320) coated for 1h at RT with BSA-coupled peptides (30nM, dissolved in PBS) and incubated with the appropriate buffers while shaking (blocking buffer, 1% BSA, 1x PBS; washing buffer, 1xPBS / 0,1% Tween; dilution buffer, 1xPBS / 0.1% BSA /0,1% Tween). After serum incubation (dilutions starting at 1:50 in PBS; typically in 1:3 or 1:2 titration steps), bound antibodies were detected using Horseradish Peroxidase-conjugated goat anti-mouse IgG (Fc) from Jackson immunoresearch (115-035-008). After stopping the reaction, plates were measured at 450nm for 20min using TMB. EC50 were calculated from readout values using curve fitting with a 4-parameter logistic regression model (GraphPad Prism) according to the procedures recommended by the manufacturer. Constraining parameters for ceiling and floor values were set accordingly, providing curve fitting quality levels of $R^2$ >0.98.

Figure 1A shows an in vivo proof of concept in a mouse model for in vivo selective plasma-lowering activity of a prototypic albumin-based SADC candidate that binds to antibodies directed against EBNA1, as a model for autoantibodies and mimicry in preeclampsia (Elliott et al.). For these mouse experiments, mouse albumin was used, in order to avoid any reactivity against a protein from a foreign species. Antibody titers were induced in 6 months old Balb/c mice by standard peptide vaccination. The bottom panel demonstrates that titers LogIC50 (y-axis) before SADC injection (i.e. titers at -48h and -24h) were higher than titers LogIC50 after SADC application (i.e. titers +24h, +48h and +72h after injection; indicated on the x-axis).

A similar example is shown in Figure 1B, using an alternative example of a peptidic antibody binding moiety for a different disease indication. Antibody lowering activity of an albumin-based SADC in a mouse model that was pre-immunized with a different peptide derived from the human AChR protein MIR region (Luo et al.) in order to mimic the situation in myasthenia gravis. The induced antibody titers against the AChR-MIR region were used as surrogate for anti-AChR-MIR autoantibodies known to play a causative role in myasthenia gravis (reviewed by Vincent et al.). A clear titer reduction was seen after SADC application.

Figures 1C and 1D demonstrate the functionality of SADC variants comprising alternative biopolymer scaffolds. Specifically, Figure 1C shows that an immunoglobulin scaffold can be successfully used whereas Figure 1D demonstrates the use of a haptoglobin-scaffold for constructing an SADC. Both examples show an *in vivo* proof of concept for selective antibody lowering by an SADC, carrying covalently bound example peptide E049.

The haptoglobin-based SADC was generated using human Haptoglobin as a surrogate although the autologuous scaffold protein would be preferred. In order to avoid formation of antihuman-haptoglobin antibodies, only one single SADC injection per mouse of the non-autoluouos scaffold haptoglobin was used for the present experimental conditions. As expected, under the present experimental conditions (i.e. one-time application), no antibody reactivity was observed against the present surrogate haptoglobin homologue.

Figure 1E demonstrates the selectivity of the SADC system. The immunoglobulin-based SADC carrying the peptide E049 (i.e. the same as in Figure 1C) cannot reduce the Ig-titer that was induced by a peptide vaccine with an unrelated, irrelevant aminoacid sequence, designated peptide T8-1 (SEQ ID NO. 16: DHTLYTPYHTHPG). The example shows an *in vivo* proof of concept for the selectivity of the system. The top panel shows anti-peptide T8-1 titers (0,5x dilution steps starting from 1:50 to 1:102400; X-axis shows log(X) dilutions) against OD values (y-axis) according to a standard ELISA. T8-1-titers are unaffected by administration of SADC-Ig-E049 after application. The bottom panel demonstrates that the initial titers LogIC50 (y-axis) *before* SADC injection (i.e. titers at -48h and -24h) are unaffected by administration of SADC-Ig-E049 (arrow) when compared to the titers LogIC50 *after* SADC application (i.e. titers +24h, +48h and +72h; as indicated on the x-axis), thereby demonstrating the selectivity of the system.

**Example 2: Immunogenicity of SADCs.**

In order to exclude immunogenicity of SADCs, prototypic candidate SADCs were tested for their propensity to induce antibodies upon repeated injection. Peptides T3-1 and T9-1 were used for this test. T3-1 is a 10-amino acid peptide derived from a reference epitope of the Angiotensin receptor, against which agonistic autoantibodies are formed in a pre-eclampsia animal model (Zhou et al.); T9-1 is a 12-amino acid peptide derived from a reference anti-drug antibody epitope of human IFN gamma (Lin et al.). These control SADC conjugates were injected 8 x every two weeks i.p. into naive, non-immunized female BALB/c mice starting at an age of 8-10 weeks.

Animals C1-C4 were treated i.p. (as described in example 1) with SADC T3-1. Animals C5-C8 were treated i.p. with an SADC carrying the peptide T9-1. As a reference signal for ELISA analysis, plasma from a control animal that was vaccinated 3 times with KLH-peptide T1-1 (derived from the AChR-MIR, explained in Example 1) was used. Using BSA-conjugated peptide probes T3-1, T9-1 and E005 (SEQ ID NO. 17: GGVKKIHIPSEK), respectively, for antibody titer detection by standard ELISA at a dilution of 1:100, it could be demonstrated that antibody induction was absent in SADC-treated animals, when compared to the vaccine-treated control animal C (see Figure 2). The plasmas were obtained by submandibular blood collection, 1 week after the 3rd vaccine injection (control animal C) and after the last of 8 consecutive SADC injections in 2-weeks intervals (animals C1-C8), respectively. Thus it was demonstrated that SADCs are non-immunogenic and do not induce antibody formation after repeated injection into mice.

Non-patent references

Carter, John Mark, and Larry Loomis-Price. "B cell epitope mapping using synthetic peptides." Current protocols in immunology 60.1 (2004): 9-4.

Elliott, Serra E., et al. "A pre-eclampsia-associated Epstein-Barr virus antibody cross-reacts with placental GPR50." Clinical Immunology 168 (2016): 64-71.

Erlandsson, Ann, et al. "In vivo clearing of idiotypic antibodies with antiidiotypic antibodies and their derivatives." Molecular immunology 43.6 (2006): 599-606.

Garces, Jorge Carlos, et al. "Antibody-mediated rejection: a review." The Ochsner Journal 17.1 (2017): 46.

Gfeller, David, et al. "Current tools for predicting cancer-specific T cell immunity." Oncoimmunology 5.7 (2016): e1177691.

Jansson, Liselotte, et al. "Immunotherapy With Apitopes Blocks the Immune Response to TSH Receptor in HLA-DR Transgenic Mice." Endocrinology 159.9 (2018): 3446-3457.

Jensen, Kamilla Kjærgaard, et al. "Improved methods for predicting peptide binding affinity to MHC class II molecules." Immunology 154.3 (2018): 394-406.

Jurtz, Vanessa, et al. "NetMHCpan-4.0: improved peptide-MHC class I interaction predictions integrating eluted ligand and peptide binding affinity data." The Journal of Immunology 199.9 (2017): 3360-3368.

Koşaloğlu-Yalçın, Zeynep, et al. "Predicting T cell recognition of MHC class I restricted neoepitopes." Oncoimmunology 7.11 (2018): e1492508.

Hansen, Lajla Bruntse, Soren Buus, and Claus Schafer-Nielsen. "Identification and mapping of linear antibody epitopes in human serum albumin using high-density peptide arrays." PLoS One 8.7 (2013): e68902.

Homma, Masayuki, et al. "A Novel Fusion Protein, AChR-Fc, Ameliorates Myasthenia Gravis by Neutralizing Antiacetylcholine Receptor Antibodies and Suppressing Acetylcholine Receptor-Reactive B Cells." Neurotherapeutics 14.1 (2017): 191-198.

Howard Jr, James F. "Myasthenia gravis: the role of complement at the neuromuscular junction." Annals of the New York Academy of Sciences 1412.1 (2018): 113-128.

Howarth, M., & Brune, K. D. (2018). New routes and opportunities for modular construction of particulate vaccines: stick, click and glue. Frontiers in immunology, 9, 1432.

Lazaridis, Konstantinos, et al. "Specific removal of autoantibodies by extracorporeal immunoadsorption ameliorates experimental autoimmune myasthenia gravis." Journal of neuroimmunology 312 (2017): 24-30.

Lim, Sung In, and Inchan Kwon. "Bioconjugation of therapeutic proteins and enzymes using the expanded set of genetically encoded amino acids." Critical reviews in biotechnology 36.5 (2016): 803-815.

Lin, Chia-Hao, et al. "Identification of a major epitope by anti-interferon-γ autoantibodies in patients with mycobacterial disease." Nature medicine 22.9 (2016): 994.

Lorentz, Kristen M., et al. "Engineered binding to erythrocytes induces immunological tolerance to E. coli asparaginase." Science advances 1.6 (2015): e1500112.

Luo, Jie, et al. "Main immunogenic region structure promotes binding of conformation-dependent myasthenia gravis autoantibodies, nicotinic acetylcholine receptor conformation maturation, and agonist sensitivity." Journal of Neuroscience 29.44 (2009): 13898-13908.

Luo, Jie, and Jon Lindstrom. "AChR-specific immunosuppressive therapy of myasthenia gravis." Biochemical pharmacology 97.4 (2015): 609-619.

Mazor, Ronit, et al. "Tolerogenic nanoparticles restore the antitumor activity of recombinant immunotoxins by mitigating immunogenicity." Proceedings of the National Academy of Sciences 115.4 (2018) : E733-E742.

Meister, Daniel, S. Maryamdokht Taimoory, and John F. Trant. "Unnatural amino acids improve affinity and modulate immunogenicity: Developing peptides to treat MHC type II autoimmune disorders." Peptide Science 111.1 (2019): e24058.

Mingozzi, Federico, et al. "Overcoming preexisting humoral immunity to AAV using capsid decoys." Science translational medicine 5.194 (2013): 194ra92-194ra92.

Mingozzi, Federico, and Katherine A. High. "Overcoming the host immune response to adeno-associated virus gene delivery vectors: the race between clearance, tolerance, neutralization, and escape." Annual review of virology 4 (2017): 511-534.

Moussa, Ehab M., et al. "Immunogenicity of therapeutic protein aggregates." Journal of pharmaceutical sciences 105.2 (2016): 417-430.

Muller, Manuel M. "Post-translational modifications of protein backbones: unique functions, mechanisms, and challenges." Biochemistry 57.2 (2017): 177-185.

Siang Ong, Yong, et al. "Recent advances in synthesis and identification of cyclic peptides for bioapplications." Current topics in medicinal chemistry 17.20 (2017): 2302-2318.

Peters, Bjoern, et al. "A community resource benchmarking predictions of peptide binding to MHC-I molecules." PLoS computational biology 2.6 (2006): e65.

Pishesha, Novalia, et al. "Engineered erythrocytes covalently linked to antigenic peptides can protect against autoimmune disease." Proceedings of the National Academy of Sciences (2017): 201701746.

Ruff, Robert L., and Robert P. Lisak. "Nature and action of antibodies in myasthenia gravis." Neurologic clinics 36.2 (2018): 275-291.

Rummler, Silke, et al. "Current techniques for AB0-incompatible living donor liver transplantation." World journal of transplantation 6.3 (2016): 548.

Runcie, Karie, et al. "Bi-specific and tri-specific antibodies-the next big thing in solid tumor therapeutics." Molecular Medicine 24.1 (2018): 50.

Ryan, Brent J., Ahuva Nissim, and Paul G. Winyard. "Oxidative post-translational modifications and their involvement in the pathogenesis of autoimmune diseases." Redox biology 2 (2014): 715-724.

Sørensen, Karen Kristine, et al. "Liver sinusoidal endothelial cells." Comprehensive Physiology 5.4 (2011): 1751-1774.

Spiess, Christoph, Qianting Zhai, and Paul J. Carter. "Alternative molecular formats and therapeutic applications for bispecific antibodies." Molecular immunology 67.2 (2015): 95-106.

Taddeo, Adriano, et al. "Selection and depletion of plasma cells based on the specificity of the secreted antibody." European journal of immunology 45.1 (2015): 317-319.

Teschner, Sven, et al. "ABO-incompatible kidney transplantation using regenerative selective immunoglobulin adsorption." Journal of clinical apheresis 27.2 (2012): 51-60.

Tetala, Kishore KR, et al. "Selective depletion of neuropathy-related antibodies from human serum by monolithic affinity columns containing ganglioside mimics." Journal of medicinal chemistry 54.10 (2011): 3500-3505.

Vincent, Angela, et al. "Serological and experimental studies in different forms of myasthenia gravis." Annals of the New York Academy of Sciences 1413.1 (2018): 143-153.

Wallukat, Gerd, et al. "Patients with preeclampsia develop agonistic autoantibodies against the angiotensin AT 1 receptor." The Journal of clinical investigation 103.7 (1999): 945-952.

Zhou, Cissy C., et al. "Angiotensin receptor agonistic autoantibodies induce pre-eclampsia in pregnant mice." Nature medicine 14.8 (2008): 855.

**Claims**

1. A compound for the sequestration of anti-Epstein-Barr virus nuclear antigen 1 (EBNA-1) antibodies and/or anti human melatonin-related receptor (GPR50) antibodies present in a human individual, the compound comprising a biopolymer scaffold and at least two peptides with a sequence length of 7-13 amino acids,
   wherein each of the peptides independently comprises a 7-13 amino-acid sequence fragment of the EBNA1 sequence identified by UniProt accession code Q1HVF7 or P03211 or of the GPR50 sequence identified by UniProt accession code Q13585, wherein the peptides are covalently bound to the biopolymer scaffold,
   wherein the biopolymer scaffold is selected from the group consisting of human globulins, preferably from the group consisting of human immunoglobulins and human haptoglobin, and human albumin.

2. The compound of claim 1, wherein the biopolymer scaffold is human haptoglobin.

3. The compound of claim 1 or 2, wherein the at least two peptides comprise a peptide $P_1$ and a peptide $P_2$, wherein $P_1$ and $P_2$ comprise the same 7-13 amino-acid sequence fragment of said EBNA1 sequence or said GPR50 sequence, wherein $P_1$ and $P_2$ are present in form of a peptide dimer **$P_1$ - S - $P_2$**, wherein S is a non-peptide spacer, wherein the peptide dimer is covalently bound to the biopolymer scaffold, preferably via a linker.

4. The compound of any one of claims 1 to 3, wherein at least one of the at least two peptides is circularized.

5. The compound of any one of claims 1 to 4, wherein the compound is non-immunogenic in humans.

6. The compound of any one of claims 1 to 5, wherein said 7-13 amino-acid sequence fragment is a fragment of the sequence RPQKRPSCIGCKGTH (SEQ ID NO: 5) or RPQKRPSCIGCKGAH (SEQ ID NO: 6), preferably of the sequence KRPSCIGCK (SEQ ID NO: 7).

7. The compound of any one of claims 1 to 6, wherein the peptides have a sequence length of 8-13 amino acids, preferably 9-12 amino acids, more preferably 10-12 amino acids, especially wherein at least one of the at least two, preferably each of the peptides consist of the sequence GRPQKRPSCIG (SEQ ID NO: 13) optionally with an N-terminal and/or C-terminal cysteine residue.

8. The compound of any one of claims 1 to 7, wherein the compound further comprises at least one peptide with a sequence length of 7-13 amino acids, wherein the at least one peptide comprises a 7-13 amino-acid sequence fragment of the type-1 angiotensin II receptor (AT1AR) sequence identified by UniProt accession code P30556, preferably of any one of the sequences MILNSSTEDGIKRIQDDCPKAGRHNYI (SEQ ID NO: 8), TAMEYRWPFGNYLCK (SEQ ID NO: 9), AIIHRNVFFIENTNITVCAFHYESQNSTLP (SEQ ID NO: 10), DVLIQLGIIRDCR (SEQ ID NO: 11), more preferably of the sequence AFHYESQ (SEQ ID NO: 12); wherein the at least one peptide is covalently bound to the biopolymer scaffold, preferably via a linker.

9. A pharmaceutical composition comprising the compound of any one of claims 1 to 8 and at least one pharmaceutically acceptable excipient.

10. The pharmaceutical composition of claim 9, wherein the composition is prepared for intraperitoneal, subcutaneous, intramuscular and/or intravenous administration.

11. The pharmaceutical composition of any one of claims 9 to 10, wherein the molar ratio of the peptides to scaffold in the composition is from 2:1 to 100:1, preferably from 3:1 to 90:1, more preferably from 4:1 to 80:1, even more preferably from 5:1 to 70:1, yet even more preferably from 6:1 to 60:1, especially from 7:1 to 50:1 or even from 8:10 to 40:1.

12. The pharmaceutical composition of any one of claims 9 to 11, for use in prevention or treatment of pre-eclampsia in a human individual.

13. The pharmaceutical composition for use according to claim 12, wherein the composition is administered at a dose of 1-1000 mg, preferably 2-500 mg, more preferably 3-250 mg, even more preferably 4-100 mg, especially 5-50 mg, compound per kg body weight of the individual.

14. The pharmaceutical composition for use according to claim 12 or 13, wherein the composition is administered intraperitoneally, subcutaneously, intramuscularly or intravenously.

15. A method of ameliorating or treating pre-eclampsia in a human individual in need thereof, comprising
obtaining a pharmaceutical composition as defined in any one of claims 9 to 11; and
administering an effective amount of the pharmaceutical composition to the individual.

Fig. 1

B

LogIC50

SADC application

Cont. Fig. 1

C

Cont. Fig. 1

Cont. Fig. 1

E

LogIC50

SADC applicatiton

Cont. Fig. 1

Fig. 2

Europäisches
Patentamt

European
Patent Office

Office européen
des brevets

# EUROPEAN SEARCH REPORT

Application Number

EP 19 16 4785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | ELLIOTT SERRA E ET AL: "A pre-eclampsia-associated Epstein-Barr virus antibody cross-reacts with placental GPR50", CLINICAL IMMUNOLOGY, ACADEMIC PRESS, US, vol. 168, 12 May 2016 (2016-05-12), pages 64-71, XP029631414, ISSN: 1521-6616, DOI: 10.1016/J.CLIM.2016.05.002 * the whole document * | 1-15 | INV. C07K17/06 A61K39/00 A61K47/54 C07K14/05 |
| Y | US 6 022 544 A (DINTZIS HOWARD M [US] ET AL) 8 February 2000 (2000-02-08) * col. 11, line 11 - col. 17, line 34, col. 35-37, Ex. 5.A.b * | 1-15 | |
| Y | JUMPEI MORIMOTO ET AL: "Dextran as a Generally Applicable Multivalent Scaffold for Improving Immunoglobulin-Binding Affinities of Peptide and Peptidomimetic Ligands", BIOCONJUGATE CHEMISTRY, vol. 25, no. 8, 20 August 2014 (2014-08-20), pages 1479-1491, XP055636727, US ISSN: 1043-1802, DOI: 10.1021/bc500226j * the whole document * | 3 | |
| Y | WO 2015/181393 A1 (JAKOBSSON PER-JOHAN [SE]; GÖRANSSON ULF [SE] ET AL.) 3 December 2015 (2015-12-03) * p. 2, line 39 - p. 7, line 11, claims 1-2 * | 1-15 | |
| Y,D | WO 98/30586 A2 (OKLAHOMA MED RES FOUND [US]) 16 July 1998 (1998-07-16) * p. 18, line 11 - p. 25, line 24, Table 6, SEQ ID NO: 16 * | 1-15 | |

TECHNICAL FIELDS SEARCHED (IPC)

C07K
A61K

The present search report has been drawn up for all claims

| Place of search | Date of completion of the search | Examiner |
|---|---|---|
| Munich | 29 October 2019 | R. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 16 4785

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2019

| Patent document cited in search report | | Publication date | Patent family member(s) | | Publication date |
|---|---|---|---|---|---|
| US 6022544 | A | 08-02-2000 | US | 6022544 A | 08-02-2000 |
| | | | US | 6340460 B1 | 22-01-2002 |
| | | | US | 6375951 B1 | 23-04-2002 |
| | | | US | 2002187156 A1 | 12-12-2002 |
| WO 2015181393 | A1 | 03-12-2015 | NONE | | |
| WO 9830586 | A2 | 16-07-1998 | AU | 6018598 A | 03-08-1998 |
| | | | CA | 2277910 A1 | 16-07-1998 |
| | | | EP | 1007552 A2 | 14-06-2000 |
| | | | JP | 2001511120 A | 07-08-2001 |
| | | | US | 7888458 B1 | 15-02-2011 |
| | | | US | 2002164355 A1 | 07-11-2002 |
| | | | US | 2004086522 A1 | 06-05-2004 |
| | | | WO | 9830586 A2 | 16-07-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9213558 A1
- WO 2015136027 A1
- WO 2017046172 A1
- US 20040258683 A1
- US 5637454 A
- US 20070026396 A1
- WO 1992014150 A1
- WO 1998030586 A2

- WO 2018102668 A1
- WO 2016020377 A1
- WO 2012000889 A1
- WO 0033887 A2
- WO 2017220569 A1
- WO 2017087589 A2
- US 82100547 B
- EP 1697421 A2

**Non-patent literature cited in the description**

- **RICE et al.** EMBOSS: the European Molecular Biology Open Software Suite. *Trends Genet,* June 2000, vol. 16 (6), 276-7
- **CARTER, JOHN MARK ; LARRY LOOMIS-PRICE.** B cell epitope mapping using synthetic peptides. *Current protocols in immunology,* 2004, vol. 60.1, 9-4
- **ELLIOTT, SERRA E. et al.** A pre-eclampsia-associated Epstein-Barr virus antibody cross-reacts with placental GPR50. *Clinical Immunology,* 2016, vol. 168, 64-71
- **ERLANDSSON, ANN et al.** In vivo clearing of idiotypic antibodies with antiidiotypic antibodies and their derivatives. *Molecular immunology,* 2006, vol. 43.6, 599-606
- **GARCES, JORGE CARLOS et al.** Antibody-mediated rejection: a review. *The Ochsner Journal,* 2017, vol. 17.1, 46
- **GFELLER, DAVID et al.** Current tools for predicting cancer-specific T cell immunity. *Oncoimmunology,* 2016, vol. 5.7, e1177691
- **JANSSON, LISELOTTE et al.** Immunotherapy With Apitopes Blocks the Immune Response to TSH Receptor in HLA-DR Transgenic Mice. *Endocrinology,* 2018, vol. 159.9, 3446-3457
- **JENSEN, KAMILLA KJÆRGAARD et al.** Improved methods for predicting peptide binding affinity to MHC class II molecules. *Immunology,* 2018, vol. 154.3, 394-406
- **JURTZ, VANESSA et al.** NetMHCpan-4.0: improved peptide-MHC class I interaction predictions integrating eluted ligand and peptide binding affinity data. *The Journal of Immunology,* 2017, vol. 199.9, 3360-3368

- **HANSEN, LAJLA BRUNTSE ; SOREN BUUS ; CLAUS SCHAFER-NIELSEN.** Identification and mapping of linear antibody epitopes in human serum albumin using high-density peptide arrays. *PLoS One,* 2013, vol. 8.7, e68902
- **HOMMA, MASAYUKI et al.** A Novel Fusion Protein, AChR-Fc, Ameliorates Myasthenia Gravis by Neutralizing Antiacetylcholine Receptor Antibodies and Suppressing Acetylcholine Receptor-Reactive B Cells. *Neurotherapeutics,* 2017, vol. 14.1, 191-198
- **HOWARD JR ; JAMES F.** Myasthenia gravis: the role of complement at the neuromuscular junction. *Annals of the New York Academy of Sciences,* 2018, vol. 1412.1, 113-128
- **HOWARTH, M. ; BRUNE, K. D.** New routes and opportunities for modular construction of particulate vaccines: stick, click and glue. *Frontiers in immunology,* 2018, vol. 9, 1432
- **LAZARIDIS, KONSTANTINOS et al.** Specific removal of autoantibodies by extracorporeal immunoadsorption ameliorates experimental autoimmune myasthenia gravis. *Journal of neuroimmunology,* 2017, vol. 312, 24-30
- **LIM, SUNG IN ; INCHAN KWON.** Bioconjugation of therapeutic proteins and enzymes using the expanded set of genetically encoded amino acids. *Critical reviews in biotechnology,* 2016, vol. 36.5, 803-815
- **LIN, CHIA-HAO et al.** Identification of a major epitope by anti-interferon-γ autoantibodies in patients with mycobacterial disease. *Nature medicine,* 2016, vol. 22.9, 994
- **LORENTZ, KRISTEN M. et al.** Engineered binding to erythrocytes induces immunological tolerance to E. coli asparaginase. *Science advances,* 2015, vol. 1.6, e1500112

- **LUO, JIE et al.** Main immunogenic region structure promotes binding of conformation-dependent myasthenia gravis autoantibodies, nicotinic acetylcholine receptor conformation maturation, and agonist sensitivity. *Journal of Neuroscience,* 2009, vol. 29.44, 13898-13908
- **LUO, JIE ; JON LINDSTROM.** AChR-specific immunosuppressive therapy of myasthenia gravis. *Biochemical pharmacology,* 2015, vol. 97.4, 609-619
- **MAZOR, RONIT et al.** Tolerogenic nanoparticles restore the antitumor activity of recombinant immunotoxins by mitigating immunogenicity. *Proceedings of the National Academy of Sciences,* 2018, vol. 115.4, E733-E742
- **MEISTER, DANIEL ; S. MARYAMDOKHT TAIMOORY ; JOHN F. TRANT.** Unnatural amino acids improve affinity and modulate immunogenicity: Developing peptides to treat MHC type II autoimmune disorders. *Peptide Science,* 2019, vol. 111.1, e24058
- **MINGOZZI, FEDERICO et al.** Overcoming preexisting humoral immunity to AAV using capsid decoys. *Science translational medicine,* 2013, vol. 5.194, 194ra92-194ra92
- **MINGOZZI, FEDERICO ; KATHERINE A. HIGH.** Overcoming the host immune response to adeno-associated virus gene delivery vectors: the race between clearance, tolerance, neutralization, and escape. *Annual review of virology,* 2017, vol. 4, 511-534
- **MOUSSA, EHAB M. et al.** Immunogenicity of therapeutic protein aggregates. *Journal of pharmaceutical sciences,* 2016, vol. 105.2, 417-430
- **MULLER, MANUEL M.** Post-translational modifications of protein backbones: unique functions, mechanisms, and challenges. *Biochemistry,* 2017, vol. 57.2, 177-185
- **SIANG ONG, YONG et al.** Recent advances in synthesis and identification of cyclic peptides for bioapplications. *Current topics in medicinal chemistry,* 2017, vol. 17.20, 2302-2318
- **PETERS, BJOERN et al.** A community resource benchmarking predictions of peptide binding to MHC-I molecules. *PLoS computational biology,* 2006, vol. 2.6, e65
- **PISHESHA, NOVALIA et al.** Engineered erythrocytes covalently linked to antigenic peptides can protect against autoimmune disease. *Proceedings of the National Academy of Sciences,* 2017, 201701746
- **RUFF, ROBERT L. ; ROBERT P. LISAK.** Nature and action of antibodies in myasthenia gravis. *Neurologic clinics,* 2018, vol. 36.2, 275-291
- **RUMMLER, SILKE et al.** Current techniques for AB0-incompatible living donor liver transplantation. *World journal of transplantation,* 2016, vol. 6.3, 548
- **RUNCIE, KARIE et al.** Bi-specific and tri-specific antibodies-the next big thing in solid tumor therapeutics. *Molecular Medicine,* 2018, vol. 24.1, 50
- **RYAN, BRENT J. ; AHUVA NISSIM ; PAUL G. WINYARD.** Oxidative post-translational modifications and their involvement in the pathogenesis of autoimmune diseases. *Redox biology,* 2014, vol. 2, 715-724
- **SØRENSEN, KAREN KRISTINE et al.** Liver sinusoidal endothelial cells. *Comprehensive Physiology,* 2011, vol. 5.4, 1751-1774
- **SPIESS, CHRISTOPH ; QIANTING ZHAI ; PAUL J. CARTER.** Alternative molecular formats and therapeutic applications for bispecific antibodies. *Molecular immunology,* 2015, vol. 67.2, 95-106
- **TADDEO, ADRIANO et al.** Selection and depletion of plasma cells based on the specificity of the secreted antibody. *European journal of immunology,* 2015, vol. 45.1, 317-319
- **TESCHNER, SVEN et al.** ABO-incompatible kidney transplantation using regenerative selective immunoglobulin adsorption. *Journal of clinical apheresis,* 2012, vol. 27.2, 51-60
- **TETALA, KISHORE KR et al.** Selective depletion of neuropathy-related antibodies from human serum by monolithic affinity columns containing ganglioside mimics. *Journal of medicinal chemistry,* 2011, vol. 54.10, 3500-3505
- **VINCENT, ANGELA et al.** Serological and experimental studies in different forms of myasthenia gravis. *Annals of the New York Academy of Sciences,* 2018, vol. 1413.1, 143-153
- **WALLUKAT, GERD et al.** Patients with preeclampsia develop agonistic autoantibodies against the angiotensin AT 1 receptor. *The Journal of clinical investigation,* 1999, vol. 103.7, 945-952
- **ZHOU, CISSY C. et al.** Angiotensin receptor agonistic autoantibodies induce pre-eclampsia in pregnant mice. *Nature medicine,* 2008, vol. 14.8, 855

SEQUENCE LISTING

<110> Ablevia biotech GmbH

<120> Compound for the prevention or treatment of pre-eclampsia

<130> R 74456/EP

<160> 17

<170> BiSSAP 1.3

<210> 1
<211> 20
<212> PRT
<213> Artificial Sequence

<220>
<223> peptide

<400> 1
Leu Lys Trp Asn Pro Asp Asp Tyr Gly Gly Val Lys Lys Ile His Ile
1               5                   10                  15
Pro Ser Glu Lys
            20

<210> 2
<211> 10
<212> PRT
<213> Artificial Sequence

<220>
<223> peptide

<400> 2
Trp Asn Pro Asp Asp Tyr Gly Gly Val Lys
1               5                   10

<210> 3
<211> 10
<212> PRT
<213> Artificial Sequence

<220>
<223> peptide

<400> 3
Val Lys Lys Ile His Ile Pro Ser Glu Lys
1               5                   10

<210> 4
<211> 11
<212> PRT
<213> Artificial Sequence

<220>
<223> peptide

<400> 4
Val Lys Lys Ile His Ile Pro Ser Glu Lys Gly
1               5                   10

<210> 5

46

```
<211> 15
<212> PRT
<213> Artificial Sequence

<220>
<223> peptide

<400> 5
Arg Pro Gln Lys Arg Pro Ser Cys Ile Gly Cys Lys Gly Thr His
1               5                   10                  15

<210> 6
<211> 15
<212> PRT
<213> Artificial Sequence

<220>
<223> peptide

<400> 6
Arg Pro Gln Lys Arg Pro Ser Cys Ile Gly Cys Lys Gly Ala His
1               5                   10                  15

<210> 7
<211> 9
<212> PRT
<213> Artificial Sequence

<220>
<223> peptide

<400> 7
Lys Arg Pro Ser Cys Ile Gly Cys Lys
1               5

<210> 8
<211> 27
<212> PRT
<213> Artificial Sequence

<220>
<223> peptide

<400> 8
Met Ile Leu Asn Ser Ser Thr Glu Asp Gly Ile Lys Arg Ile Gln Asp
1               5                   10                  15
Asp Cys Pro Lys Ala Gly Arg His Asn Tyr Ile
            20                  25

<210> 9
<211> 15
<212> PRT
<213> Artificial Sequence

<220>
<223> peptide

<400> 9
Thr Ala Met Glu Tyr Arg Trp Pro Phe Gly Asn Tyr Leu Cys Lys
1               5                   10                  15

<210> 10
<211> 30
```

```
<212> PRT
<213> Artificial Sequence

<220>
<223> peptide

<400> 10
Ala Ile Ile His Arg Asn Val Phe Phe Ile Glu Asn Thr Asn Ile Thr
1               5                   10                  15
Val Cys Ala Phe His Tyr Glu Ser Gln Asn Ser Thr Leu Pro
            20                  25                  30

<210> 11
<211> 13
<212> PRT
<213> Artificial Sequence

<220>
<223> peptide

<400> 11
Asp Val Leu Ile Gln Leu Gly Ile Ile Arg Asp Cys Arg
1               5                   10

<210> 12
<211> 7
<212> PRT
<213> Artificial Sequence

<220>
<223> peptide

<400> 12
Ala Phe His Tyr Glu Ser Gln
1               5

<210> 13
<211> 11
<212> PRT
<213> Artificial Sequence

<220>
<223> peptide

<400> 13
Gly Arg Pro Gln Lys Arg Pro Ser Cys Ile Gly
1               5                   10

<210> 14
<211> 15
<212> PRT
<213> Artificial Sequence

<220>
<223> peptide

<400> 14
Cys Gly Arg Pro Gln Lys Arg Pro Ser Cys Ile Gly Cys Lys Gly
1               5                   10                  15

<210> 15
<211> 22
<212> PRT
```

```
<213> Artificial Sequence

<220>
<223> peptide

<400> 15
Leu Lys Trp Asn Pro Asp Asp Tyr Gly Gly Val Lys Lys Ile His Ile
1               5                   10                  15
Pro Ser Glu Lys Gly Cys
                20


<210> 16
<211> 13
<212> PRT
<213> Artificial Sequence

<220>
<223> peptide

<400> 16
Asp His Thr Leu Tyr Thr Pro Tyr His Thr His Pro Gly
1               5                   10


<210> 17
<211> 12
<212> PRT
<213> Artificial Sequence

<220>
<223> peptide

<400> 17
Gly Gly Val Lys Lys Ile His Ile Pro Ser Glu Lys
1               5                   10
```